(19) Europäisches Patentamt / European Patent Office / Office européen des brevets

(11) **EP 4 721 728 A1**

(12) **EUROPEAN PATENT APPLICATION**
published in accordance with Art. 153(4) EPC

(43) Date of publication:
08.04.2026 Bulletin 2026/15

(21) Application number: 24814538.5

(22) Date of filing: 30.05.2024

(51) International Patent Classification (IPC):
A61K 9/127 (2025.01)   A61K 9/19 (2006.01)
A61K 9/51 (2006.01)   A61K 31/7088 (2006.01)
A61K 39/12 (2006.01)   A61K 47/26 (2006.01)
A61K 47/28 (2006.01)   A61K 47/44 (2017.01)
A61K 47/69 (2017.01)   C12N 15/87 (2006.01)
A61P 31/16 (2006.01)

(52) Cooperative Patent Classification (CPC):
A61K 9/127; A61K 9/19; A61K 9/51;
A61K 31/7088; A61K 39/12; A61K 47/26;
A61K 47/28; A61K 47/44; A61K 47/69;
A61P 31/16; C12N 15/87

(86) International application number:
PCT/CN2024/096347

(87) International publication number:
WO 2024/245341 (05.12.2024 Gazette 2024/49)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 30.05.2023 CN 202310626158
10.11.2023 CN 202311497985

(71) Applicant: CSPC Zhongqi Pharmaceutical
Technology
(Shijiazhuang) Co., Ltd.
Shijiazhuang, Hebei 050035 (CN)

(72) Inventors:
• LI, Chunlei
  Shijiazhuang, Hebei 050035 (CN)
• LI, Yanhui
  Shijiazhuang, Hebei 050035 (CN)
• WANG, Yajuan
  Shijiazhuang, Hebei 050035 (CN)
• LI, Yuanyuan
  Shijiazhuang, Hebei 050035 (CN)

• LI, Yongfeng
  Shijiazhuang, Hebei 050035 (CN)
• WANG, Shixia
  Shijiazhuang, Hebei 050035 (CN)
• SHU, Yun
  Shijiazhuang, Hebei 050035 (CN)
• NI, Beibei
  Shijiazhuang, Hebei 050035 (CN)
• ZHANG, Xiaoyan
  Shijiazhuang, Hebei 050035 (CN)
• DAN, Mo
  Shijiazhuang, Hebei 050035 (CN)
• GENG, Cong
  Shijiazhuang, Hebei 050035 (CN)

(74) Representative: Barrow, Nicholas Martin et al
Venner Shipley LLP
406 Cambridge Science Park
Milton Road
Cambridge CB4 0WW (GB)

Remarks:
The complete document including Reference
Table(s) and the Sequence Listing(s) can be
downloaded from the EPO website

(54) **NUCLEIC ACID LIPID NANOPARTICLE WITH LOW NO-LOAD RATE AND PREPARATION METHOD THEREFOR**

(57) A lipid nanoparticle composition for encapsulating a nucleic acid and a preparation method therefor. In the composition, the proportion of empty lipid nanoparticles containing no nucleic acid to the total number of lipid nanoparticles, i.e., the no-load rate, is no more than 10%. According to the preparation method, the ethanol content in the system is reduced to an acceptable level during particle fusion, so that the particles are fully fused.

EP 4 721 728 A1

Figure 9

## Description

### Cross-reference to related applications

This application claims the priority to Chinese Patent Application No. 202310626158.9 filed on May 30, 2023 and the priority to Chinese Patent Application No. 202311497985.9 filed on November 10, 2023. The entire contents of the above two patent applications are hereby incorporated herein by reference.

### Technical field of the invention

The present application belongs to the field of biomedical technology. Specifically, the present application relates to nucleic acid lipid nanoparticle with low no-load rate and preparation method therefor.

### Background art of the invention

Half a century ago, Friedmann and Roblin proposed that dysfunction caused by genetic diseases could be treated by introducing functional genes. Conventional drug targets are usually proteins, while nucleic acid drugs produce therapeutic effects by regulating gene expression. This method of introducing exogenous nucleic acids into cells to eliminate defective genes is a very effective treatment method that can achieve highly specific and lasting therapeutic effects. In the past 30 years, the study on nucleic acid drugs have also made breakthrough progress. Some in vitro and in vivo nucleic acid drugs for the prevention or treatment of genetic diseases such as infections, cancers, and musclar and retinal dystrophy have been approved or are in late stage clinical development.

Due to the poor stability, nucleic acid molecules are easily degraded by nucleases in vivo; at the same time, since nucleic acid molecules carry a large amount of negative charges, they cannot penetrate the cell membrane and enter the cytoplasm by themselves to exert their efficacy. Therefore, specific delivery technologies are required for nucleic acid drugs to be delivered into the cell to take effects. Current commonly used nucleic acid delivery technologies include lipid nanoparticles (LNPs), electroporation, protamine, cationic nanoemulsions, cationic lipid complexes, lipid polymer complexes, polymer nanoparticles, inorganic nanoparticles, etc. Electroporation generates pores in the cell membrane through high-voltage pulses, allowing nucleic acids to be directly delivered into human cells. Most of the other methods aim to protect nucleic acids from degradation or to promote the fusion of nucleic acids with cell membranes to improve delivery efficiency. For example, protamine is a natural cationic protein that can complex with negatively charged nucleic acids to form nano-scale nucleic acid particles, thereby protecting nucleic acids from degradation by nucleases in serum; however, as the binding of protamine with nucleic acids is too tight, the protein expression efficiency of nucleic acid drugs using this technology in cells is affected.

Nucleic acid drugs, especially mRNA vaccines, played an important role in epidemic prevention during the outbreak and widely spread of the novel coronavirus and received widespread attention. Both the marketed mRNA vaccines and earlier RNA drug Onpattro® use LNP for delivery. LNP is the most advanced non-viral gene delivery system in clinical practice. Compared with other types of nucleic acid drug delivery systems, LNP has many advantages, such as high encapsulation efficiency of nucleic acids, effective cell transfection, strong tissue penetration, and low cytotoxicity and immunogenicity, which make LNP an excellent nucleic acid delivery system.

LNPs are usually formed by self-assembly after rapid mixing of lipids in an organic phase with nucleic acids in an aqueous phase, followed by removal of the organic solvent and buffer solution replacement by dialysis or ultrafiltration. The typical preparation process and particle formation mechanism of LNPs are as follows: (1) Mixing process: The lipids are dissolved in an organic solvent, and the nucleic acids are dissolved in an acidic (e.g., pH 4.0) buffer solution. When the two phases are mixed, due to the significantly lower pH value of the system compared to the pKa of the ionizable lipids, the ionizable cationic lipids are protonated and positively charged, and generate electrostatic attraction with the negatively charged nucleic acids. At the same time, due to the poor water solubility of the lipids, hydrophobic interactions are generated, driving the lipids to encapsulate nucleic acids to form lipid nanoparticles by self-assembly. Cryo-transmission electron microscopy (Cryo-TEM) shows that at least two forms of LNPs exist simultaneously under this condition: vesicle-like particles containing a bilayer structure, and electron-dense structure particles; (2) Dialysis or ultrafiltration process: During the dialysis or ultrafiltration process, as the replacement of the mixed solution with a buffer solution having a physiological pH value (e.g., pH 7.4), the ionizable cationic lipids are deprotonated, losing most of their positive charges, and the electrostatic repulsion between particles is reduced; the ionizable cationic lipids migrate inward to form a hydrophobic amorphous core, the bilayer structure of the particle is destroyed, and the particles fuse to compensate for the decrease in the ratio of surface lipids to core lipids, rendering the size of LNP increase (Kulkarni JA, et al. On the Formation and Morphology of Lipid Nanoparticles Containing Ionizable Cationic Lipids and siRNA. ACS Nano. 2018 May 22; 12(5): 4787-4795). Different literatures have reported different morphologies of LNPs after dialysis, such as spherical electron-dense structures or non-spherical electron-dense structures containing vesicles of different sizes and percentages, indicating that the physical and chemical properties of the final product LNPs after fusion are different due to the influence of formulation and process factors, and changes in the physical and chemical properties (composition and structure) of LNPs have been shown to affect their stability and safety.

The instability of LNP and the need for storage under ultra-low temperature restrict the storage, transportation and circulation of nucleic acid drugs, especially mRNA vaccines, thus affecting their cost and accessibility. The two mRNA vaccines for the novel coronavirus, BNT162b2 and mRNA-1273, which have been launched on the market, need to be stored at -80°C~-60°C and -25°C~-15°C respectively, with a period of validity of only 6 months. Long term storage of LNP may bring about the following: aggregation and sedimentation of particles; oxidation and hydrolysis of mRNA; irreversible changes in phase after freezing and thawing, etc. Neutron scattering was used to characterize the thermal stability of the Pfizer-BioNtech novel coronavirus mRNA vaccine. The changes in the molecular vibrational spectra during the freezing and thawing process showed that, upon originally warming from the low-temperature frozen state, the vaccine exhibits two-step melting, indicative of a two-phase morphology; and after refreezing and warming, the vaccine exhibits one-step melting, indicative of a one-phase morphology. That is, after one freeze-thaw, the two-phase morphology of the Pfizer-BioNtech novel coronavirus mRNA vaccine has undergone an irreversible change, forming a one-phase morphology with greatly increased mobility and softness at a molecular level, thereby having an irreversible adverse effect on the long-term storage stability of the vaccine (Mamontov E, et al. Melting and ReFreezing Leads to Irreversible Changes in the Morphology and Molecular-Level Dynamics of Pfizer-BioNTech COVID-19 Vaccine. Medicina (Kaunas). 2021 Dec 9; 57(12): 1343).

With the widespread vaccination of novel coronavirus mRNA vaccines during this pandemic, the safety of nucleic acid drugs has also received widespread attention. A large-scale study of tens of millions of people quantified for the first time the risk of adverse cardiac events (myocarditis, pericarditis, and arrhythmia) associated with novel coronavirus vaccination. The study showed that mRNA vaccination and adenovirus vaccination were associated with an increased risk of myocarditis in adults; among men under 40 years old, the number of excessive myocarditis events per million people after the second dose of the mRNA vaccine mRNA-1273 was higher than that after SARS-CoV-2 infection, and significantly higher than that of the mRNA vaccine BNT162b2 and adenovirus vaccine ChAdOx1. The differences in the incidence of cardiac-related adverse events between different mRNA vaccines may be related to the differences in in-vivo behavior caused by the composition and structure of mRNA LNPs (Patone M, et al. Risk of Myocarditis After Sequential Doses of COVID-19 Vaccine and SARS-CoV-2 Infection by Age and Sex. Circulation. 2022 Sep 6; 146(10): 743-754).

Compared with vaccines made by traditional technology such as inactivated vaccines and subunit vaccines, mRNA vaccines can promote very strong humoral immunity and cellular immune responses. Studies have shown that empty LNPs without nucleic acids themselves can stimulate specific pathways of the immune system and achieve specific activation of the immune system. This is the advantage of LNP as a vaccine delivery vector; however, at the same time, the inflammatory nature of LNPs can also cause typical inflammatory side effects such as pain, swelling, and fever. With the development of vaccine-related technologies, it is necessary to seek a balance between the advantages of LNP immune responses and adverse inflammatory reactions (Ndeupen S, et al. The mRNA-LNP platform's lipid nanoparticle component used in preclinical vaccine studies is highly inflammatory. iScience. 2021 Dec 17; 24 (12): 103479). The no-load rate in LNP is an important feature that needs to be paid attention to when it is used as a nucleic acid delivery vector. Studies have shown that LNPs prepared by traditional processes may have 40%-80% of LNPs with no-load (Li S, et al. Payload distribution and capacity of mRNA lipidnanoparticles. Nat Commun. 2022 Sep 23; 13(1): 5561). No-load LNPs cannot deliver nucleic acids into cells, but instead increase the risk of inflammatory adverse reactions and affect the safety of nucleic acid drugs.

In summary, it remains desirable to provide lipid nanoparticles for delivering therapeutic or prophylactic nucleic acid drugs with improved safety, stability and efficacy.

**Summary of the invention**

An object of the present invention is to provide a lipid nanoparticle composition with low no-load rate for delivering a therapeutic or prophylactic nucleic acid drug.

Another object of the present invention is to provide a method for the preparation of a lipid nanoparticle composition with low no-load rate for delivering a therapeutic or prophylactic nucleic acid drug.

A technical problem to be solved by the present invention is to provide a lipid nanoparticle composition with low no-load rate for encapsulating a nucleic acid.

Another technical problem to be solved by the present invention is to provide a lipid nanoparticle composition with low no-load rate and improved thermal stability for encapsulating a nucleic acid.

Another technical problem to be solved by the present invention is to provide a lipid nanoparticle composition with low no-load rate, improved thermal stability and improved safety, for encapsulating a nucleic acid.

Another technical problem to be solved by the present invention is to provide a method for preparing a nucleic acid-containing lipid nanoparticle composition having one or more of the above advantages.

In order to solve the above-mentioned technical problems, the inventors of the present application studied the preparation process of nucleic acid-containing lipid nanoparticles and found that by increasing the degree of fusion of the particles, nucleic acid-containing lipid nanoparticle with low no-load rate can be obtained. The obtained nucleic acid-containing lipid nanoparticles have improved safety, stability and efficacy, thereby meeting the needs of the prior art.

In the first aspect of the present invention, a lipid nanoparticle composition for encapsulating a nucleic acid is provided, characterized in that the no-load rate of the lipid nanoparticle is not more than 10%, wherein the no-load rate refers to the ratio of the number of empty lipid nanoparticles without nucleic acids to the total number of lipid nanoparticles in the composition. In some embodiments, the no-load rate of the lipid nanoparticles is not more than 9%, preferably not more than 8.5%, more preferably not more than 6%, further preferably not more than 3%, or any value within the above ranges, for example, 2%, 3%, 4%, 5%, 6%, 7%, 7.1%, 7.6%, 7.7%, 7.9%, 8%, 8.3% or 9%.

In some embodiments, the average particle size of the lipid nanoparticles is 50 nm~150 nm, preferably 70 nm~120 nm, more preferably 90 nm~110 nm, or any value within the above ranges, for example, 96 nm, 97 nm, 98 nm, 99 nm, 100 nm, 101 nm, 102 nm, 103 nm, 104 nm, 105 nm, 106 nm, 107 nm, 108 nm, 109 nm, 110 nm, 111 nm, 112 nm, 113 nm, 114 nm, 115 nm, 116 nm, 117 nm, 118 nm, 119 nm or 120 nm.

In some embodiments, the encapsulation efficiency of the lipid nanoparticles is greater than 80%, preferably greater than 85%, more preferably greater than 90%.

In some embodiments, the lipid nanoparticle composition encapsulating a nucleic acid is provided, wherein the lipid nanoparticle comprises:

(1) a nucleic acid; and
(2) a lipid component, which comprises an ionizable cationic lipid, a structured lipid, a helper lipid and a surfactant.

In some embodiments, the nucleic acid is selected from the group consisting of mRNA, small interfering RNA (siRNA), DNA, plasmid, antisense oligonucleotide, ribozyme, etc. The nucleic acid encodes target therapeutic products, and is complexed with one or more lipids, encapsulated in one or more lipids, or associated with one or more lipids, thereby forming lipid nanoparticles.

In some embodiments, the nucleic acid is selected from an mRNA comprising a nucleotide sequence having at least 80%, at least 85%, at least 90%, at least 95%, at least 98%, at least 99% or 100% identity to any one of the nucleotide sequence of SEQ ID NOs: 1-6, or an mRNA encoding the coronavirus antigen which comprises an amino acid sequence having at least 80%, at least 85%, at least 90%, at least 95%, at least 98%, at least 99% or 100% identity to the amino acid sequence of SEQ ID NO: 7, or an mRNA comprsing a nucleotide sequence having at least 80%, at least 85%, at least 90%, at least 95%, at least 98%, at least 99% or 100% identity to any one of the nucleotide sequence of SEQ ID NOs: 8-26. Preferably, the mRNA comprises the nucleotide sequence of SEQ ID NO: 1. More preferably, the mRNA is the nucleotide sequence of SEQ ID NO: 1.

In some embodiments, the lipid component includes an ionizable cationic lipid, a structured lipid, a helper lipid and a surfactant, and the molar content of the ionizable cationic lipid, structured lipid, helper lipid and surfactant is 100% by mole (mol%). In some embodiments, the lipid component includes 20-60mol% of the ionizable cationic lipid, 25-55mol% of the structured lipid, 2-25mol% of the helper lipid and 0.5-15mol% of the surfactant.

In some embodiments, the ionizable cationic lipid is selected from the group consisting of SM-102 (CAS No.: 2089251-47-6), ALC-0315 (CAS No.: 2036272-55-4), Dlin-MC3-DMA (CAS No.: 1224606-06-7), DODMA (CAS No.: 104162-47-2), C12-200 (CAS No.: 1220890-25-4) and DlinDMA (CAS No.: 871258-12-7).

In some embodiments, the structured lipid is selected from cholesterol or cholesterol derivatives.

In some embodiments, the helper lipid is selected from the group consisting of distearoylphosphatidylcholine (DSPC), dioleoylphosphatidylethanolamine (DOPE), dioleoylphosphatidylcholine (DOPC), dioleoylphosphatidylglycerol (DOPG), and dioleoylphosphatidylserine (DOPS).

In some embodiments, the surfactant is selected from the group consisting of mPEG-DMG-2K (CAS No.: 160743-62-4), ALC-0159 (CAS No.: 1849616-42-7), PEGylated distearoylphosphatidylethanolamine (PEG-DSPE), methoxy PEG ditetradecylpropylamine (DTDA-PEG2000) and vitamin E polyethylene glycol succinate (TPGS).

In some embodiments, the lipid component of the lipid nanoparticle composition comprises 20-50 mol%, preferably 30-50 mol% or 40-50 mol% of the ionizable cationic lipid. For example, the lipid component may comprise 41 mol%, 42 mol%, 43 mol%, 44 mol%, 45 mol%, 46 mol%, 47 mol%, 48 mol%, 49 mol% or 50 mol% of the ionizable cationic lipid (such as SM-102 or Dlin-MC3-DMA). In some embodiments, the lipid component comprises 50 mol% of SM-102.

In other embodiments, the lipid component of the lipid nanoparticle composition comprises 50-60 mol% of the ionizable cationic lipid. For example, the lipid component may comprise 51 mol%, 52 mol%, 53 mol%, 54 mol%, 55 mol%, 56 mol%, 57 mol%, 58 mol%, 59 mol% or 60 mol% of the ionizable cationic lipid (such as SM-102 or Dlin-MC3-DMA).

In some embodiments, the lipid component of the lipid nanoparticle composition comprises 2-25 mol%, preferably 2-20 mol%, more preferably 2-15 mol% of the helper lipid. For example, the lipid component may comprise 2 mol%, 3 mol%, 4 mol%, 5 mol%, 6 mol%, 7 mol%, 8 mol%, 9 mol%, 10 mol%, 11 mol%, 12 mol%, 13 mol%, 14 mol% or 15 mol% of the helper lipid (such as DSPC). In some embodiments, the lipid component comprises 2-15 mol% of DSPC. In some embodiments, the lipid component comprises 10 mol% of DSPC.

In some embodiments, the lipid component of the lipid nanoparticle composition comprises 25-55 mol%, preferably 30-40 mol% of the structured lipid. For example, the lipid component may comprise 30 mol%, 31 mol%, 32 mol%, 33 mol%, 34 mol%, 35 mol%, 36 mol%, 37 mol%, 38 mol%, 39 mol% or 40 mol% of the structured lipid (such as cholesterol). In some embodiments, the lipid component comprises 38.5 mol% of the structured lipid. In some embodiments, the lipid component comprises 38.5 mol% of cholesterol.

In some embodiments, the lipid component of the lipid nanoparticle composition comprises 0.5-15 mol%, preferably 0.5-10 mol% or 0.5-5 mol%, more preferably 1-2 mol% of the surfactant. For example, the lipid component may comprise 1 mol%, 1.5 mol% or 2 mol% of the surfactant (such as mPEG-DMG-2K). In some embodiments, the lipid component comprises 1.5 mol% of mPEG-DMG-2K.

In some embodiments, the lipid component of the lipid nanoparticle composition comprises 50 mol% of the ionizable cationic lipid, 10 mol% of the helper lipid, 38.5 mol% of the structured lipid, and 1.5 mol% of the surfactant.

In some embodiments, the lipid component of the lipid nanoparticle composition comprises 50 mol% of SM-102, 10 mol% of DSPC, 38.5 mol% of cholesterol, and 1.5 mol% of mPEG-DMG-2K.

In some embodiments, the lipid nanoparticles have an N:P ratio of from about 2:1 to about 30:1, preferably from about 2:1 to about 15:1, more preferably from about 2:1 to about 10:1, and more preferably from about 3:1 to about 6:1. In some embodiments, the lipid nanoparticles have an N:P ratio of about 6:1. In some embodiments, the lipid nanoparticles have an N:P ratio of about 3:1.

In some embodiments, the weight ratio of the ionizable cationic lipid component to the nucleic acid in the lipid nanoparticle composition is from about 5: 1 to about 100: 1, preferably from about 5: 1 to about 50: 1, preferably from about 5: 1 to about 30: 1, more preferably from about 10: 1 to about 20: 1. In some embodiments, the weight ratio of the ionizable cationic lipid component to the nucleic acid in the lipid nanoparticle composition is about 20: 1. In some embodiments, the weight ratio of the ionizable cationic lipid component to the nucleic acid in the lipid nanoparticle composition is about 10: 1.

In some embodiments, the lipid nanoparticle composition further comprises a final product buffer.

In some embodiments, the final product buffer comprises a buffering agent and/or a cryoprotectant.

In some embodiments, the buffering agent can be selected from the group consisting of acetates, formates, carbonates, phosphates, borates, succinates, gluconates, lactates, citric acid, glycine, leucine, barbituric acid, phthalic acid, tromethamine (Tris), triethylamine, ammonium salts and combinations thereof. The content/concentration of the buffering agent is determined according to the acidity/alkalinity of the specific type of buffering agent, so that the pH value of the buffer solution is 7-8.

In some embodiments, the cryoprotectant can be selected from substances such as sugars, polyols, polymers, surfactants, amino acids and salts, wherein the sugars can be selected from lactose, sucrose, trehalose, galactose and the like.

In some embodiments, the amount of the cryoprotectant is from 1 to 50% w/w of the composition, such as from 2 to 50% w/w, or from 4 to 45% w/w, or from 6 to 12% w/w, preferably from 6 to 10% w/w, most preferably from 7 to 9% w/w.

In some embodiments, the final product buffer comprises tromethamine, sodium acetate and sucrose, and has a pH value of 7-8.

In some embodiments, the content of tromethamine is selected from 10-30 mmol/L, preferably 15-25 mmol/L, preferably 15-20 mmol/L, for example, 15 mmol/L, 15.5 mmol/L, 16 mmol/L, 16.5 mmol/L, 17 mmol/L, 17.5 mmol/L, 18 mmol/L, 18.5 mmol/L, 19 mmol/L, 19.5 mmol/L, 20 mmol/L, 20.5 mmol/L, 21 mmol/L, 21.5 mmol/L, 22 mmol/L, 22.5 mmol/L, 23 mmol/L, 23.5 mmol/L, 24 mmol/L, 24.5 mmol/L, 25 mmol/L, and most preferably 20 mmol/L.

In some embodiments, the content of sodium acetate is selected from 0-20 mmol/L, preferably 5-11 mmol/L, for example, 5 mmol/L, 5.5 mmol/L, 6 mmol/L, 6.5 mmol/L, 7 mmol/L, 7.5 mmol/L, 8 mmol/L, 8.5 mmol/L, 9 mmol/L, 9.5 mmol/L, 10 mmol/L, 10.5 mmol/L, 10.6 mmol/L, 10.7 mmol/L, 10.8 mmol/L, 10.9 mmol/L, 11 mmol/L, 11.5 mmol/L, 12 mmol/L, 12.5 mmol/L, 13 mmol/L, and most preferably 10.7 mmol/L.

In some embodiments, the content of sucrose is selected from 5-15%, preferably 7.5-10%, more preferably 7.5-9%, for example, 7.6%, 7.7%, 7.8%, 7.9%, 8.0%, 8.1%, 8.2%, 8.3%, 8.4%, 8.5%, 9%, 9.5%, 10%, and most preferably 8.7%.

In some embodiments, the composition comprises lipid nanoparticles encapsulating a nucleic acid and a final product buffer.

In some embodiments, the composition comprises lipid nanoparticles encapsulating a nucleic acid, a final product buffer, and a cryoprotectant.

In some embodiments, the composition comprises lipid nanoparticles encapsulating a nucleic acid, tromethamine, sodium acetate, and sucrose.

In some embodiments, the composition comprises lipid nanoparticles encapsulating a nucleic acid, 20 mmol/L of tromethamine, 10.7 mmol/L of sodium acetate and 8.7% of sucrose, and has a pH of 7.0-8.0, wherein the lipid nanoparticles comprise a nucleic acid and a lipid component, the nucleic acid is mRNA with a concentration of 100 $\mu$g/ml, and the lipid component comprises 50 mol% of SM-102, 10 mol% of DSPC, 38.5 mol% of cholesterol and 1.5 mol% of mPEG-DMG-2K, and the no-load rate of the composition is not more than 10%.

In the second aspect of the present invention, a method for preparing the lipid nanoparticle composition encapsulating a nucleic acid as recited above in the first aspect is provided, which comprises: (1) preparing precursor lipid nanoparticles to obtain a buffer system containing the precursor lipid nanoparticles, and (2) replacing the buffer system containing the precursor lipid nanoparticles with a neutral buffer system to obtain a final product lipid nanoparticle composition, wherein, by replacing the buffer system, the content of the organic solvent in the buffer system containing the precursor lipid nanoparticles is reduced to less than or equal to 5% (w/w) when the pH value of the system is lower than the pKa of the lipid nanoparticles by more than 0.5 units.

The precursor lipid nanoparticles can be prepared by methods known in the art for preparing lipid nanoparticles.

In some embodiments, the precursor lipid nanoparticles can be prepared as follows: dissolving a lipid component (an ionizable cationic lipid, a helper lipid, a structured lipid and a surfactant) in an organic solvent to form a lipid phase; dissolving a nucleic acid in an acidic buffer solution to form an aqueous phase; and mixing the lipid phase and the aqueous phase to allow the lipid to encapsulate the nucleic acid to form the precursor lipid nanoparticles.

In some embodiments, the precursor lipid nanoparticles can also be prepared as follows: dissolving a lipid component (an ionizable cationic lipid, a helper lipid, a structured lipid and a surfactant) in an organic solvent to form a lipid phase, mixing the lipid phase and an acidic buffer solution to form empty lipid nanoparticles; dissolving a nucleic acid in an acidic buffer solution to form an aqueous phase; and mixing the empty lipid nanoparticles with the aqueous phase containing the nucleic acid to allow the lipid to encapsulate the nucleic acid to form the precursor lipid nanoparticles.

In the above embodiments, the organic solvent is selected from C1-C4 low-carbon alcohols, preferably ethanol.

In the above embodiments, the total concentration of the lipid component (ionizable cationic lipid, structured lipid, helper lipid and surfactant) in the lipid phase is 10-15 mg/ml.

In the above embodiments, the concentration of the nucleic acid in the aqueous phase is 0.01-1 mg/ml, preferably 0.05-0.5 mg/ml, and more preferably 0.1-0.2 mg/ml.

In the above embodiments, the acidic buffer solution comprises a buffering agent selected from the group consisting of acetates, formates, carbonates, phosphates, borates, succinates, gluconates, lactatse, citric acid, glycine, leucine, barbituric acid, phthalic acid, tromethamine (Tris), triethylamine, ammonium salts, and combinations thereof. The content/concentration of the bufferring agent is determined based on the acidity/alkalinity of the specific type of buffering agent, such that the pH value of the acidic buffer solution is less than the pKa of the lipid nanoparticles. In some embodiments, the acidic buffer solution further comprises an osmotic pressure regulator, which is selected from sodium chloride, potassium chloride and the like.

In some embodiments, the acidic buffer solution comprises citric acid and sodium chloride. Preferably, the acidic buffer solution comprises 10-20 mM of citric acid and 120-140 mM of sodium chloride. In some embodiments, the acidic buffer solution comprises 10 mM of citric acid and 130 mM of sodium chloride, with a pH of 3.9-4.1, for example, a pH of 4.0. In some embodiments, the acidic buffer solution comprises 20 mM of citric acid and 130 mM of sodium chloride, with a pH of 3.9-4.1, for example, a pH of 4.0. Preferably, in some embodiments, the acidic buffer solution comprises 10 mM of citric acid and 130 mM of sodium chloride, with a pH of 4.0; or comprises 20 mM of citric acid and 130 mM of sodium chloride, with a pH of 4.0.

In the above embodiments, the volume ratio of the lipid phase and the aqueous phase for mixing is 1:2-1:9, preferably 1:2-1:5, preferably 1:3.

In the above embodiments, the mixing is performed by a microfluidic mixer, which is selected from a staggered herringbone mixer (SHM), a T-junction mixer, or a microfluidic hydrodynamic focusing (MHF) mixer.

In the above embodiments, the particle size of the resulting precursor lipid nanoparticles is 20-150 nm, preferably 30-100 nm, more preferably 40-90 nm, and even more preferably 50-80 nm.

In the above embodiments, the particle dispersion index PDI of the resulting precursor lipid nanoparticles is 0.01-0.3, preferably 0.01-0.2, and more preferably 0.01-0.15.

In the above embodiments, the encapsulation efficiency of the resulting precursor lipid nanoparticles is 50-100%, preferably 80%-100%, and more preferably 90%-100%.

After obtaining the precursor lipid nanoparticles, the buffer system containing the precursor lipid nanoparticles is replaced with a neutral buffer system to obtain a final product lipid nanoparticle composition.

In some embodiments, the buffer system replacement comprises a two-stage buffer system replacement process.

In some embodiments, the two-stage buffer system replacement process comprises replacement with an acidic buffer solution or a neutral buffer solution in the first stage and replacement with a neutral buffer solution in the second stage. In this process, in the first stage, the pH value of the system is maintained below the pKa of the lipid nanoparticles, and the content of the organic solvent in the system is reduced to a certain level (for example, less than or equal to 5%) by replacement with acidic buffer solution or neutral buffer solution, so as to ensure that there is low residual organic solvent in the system when the particles fuse in the second stage. In the second stage, when the pH value of the system increases to near the pKa of the lipid nanoparticles due to replacement with the neutral buffer solution, the particles begin to fuse, and the particle fusion is more complete at a low content of the organic solvent. When the pH value of the system is higher than the pKa of the lipid nanoparticles, the particle fusion is completed, forming thermodynamically stable particles (i.e., final product lipid nanoparticles).

In the above embodiments, the acidic buffer solution comprises a buffering agent selected from the group consisting of acetates, formates, carbonates, phosphates, borates, succinates, gluconates, lactates, citric acid, glycine, leucine, barbituric acid, phthalic acid, tromethamine (Tris), triethylamine, ammonium salts, and combinations thereof. The content/concentration of the buffering agent is determined based on the acidity/alkalinity of the specific type of buffering agent, so that the pH value of the acidic buffer solution is less than the pKa of the lipid nanoparticles. In some embodiments, the pKa of the lipid nanoparticles is 6.0-7.0, and the pH value of the acidic buffer solution is less than the pKa of the lipid nanoparticles, for example, the pH is 3.0-5.0, preferably 4.0-5.0.

In some embodiments, the acidic buffer solution further comprises an osmotic pressure regulator, which is selected from sodium chloride, potassium chloride and the like.

In some embodiments, the acidic buffer solution comprises citric acid and sodium chloride. Preferably, the acidic buffer solution comprises 10-20 mM of citric acid and 120-140 mM of sodium chloride. In some embodiments, the acidic buffer solution comprises 10 mM of citric acid and 130 mM of sodium chloride, with a pH of 3.9-4.1, for example, a pH of 4.0. Preferably, in some embodiments, the acidic buffer solution comprises 10 mM of citric acid and 130 mM of sodium chloride, with a pH of 4.0.

In the above embodiments, the neutral buffer solution comprises a buffering agent selected from the group consisting of acetates, formates, carbonates, phosphates, borates, succinates, gluconates, lactates, citric acid, glycine, leucine, barbituric acid, phthalic acid, tromethamine (Tris), triethylamine, ammonium salts, and combinations thereof. The content/concentration of the buffering agent is determined based on the acidity/alkalinity of the specific type of buffering agent, so that the pH value of the neutral buffer solution is greater than the pKa of the lipid nanoparticles. In some embodiments, the pKa of the lipid nanoparticles is 6.0-7.0, and the pH value of the neutral buffer solution is greater than the pKa of the lipid nanoparticles, for example, the pH is 7.0-8.0.

In some embodiments, the neutral buffer solution further comprises a cryoprotectant, which can be selected from substances such as sugars, polyols, polymers, surfactants, amino acids, and salts, wherein the sugars can be selected from lactose, sucrose, trehalose, galactose, and the like.

In some embodiments, the amount of the cryoprotectant is from 1% w/w to 50% w/w of the buffer solution, such as from 2% w/w to 50% w/w, or from 4% w/w to 45% w/w, or from 6% w/w to 12% w/w, preferably from 6% w/w to 10% w/w, most preferably from 7% w/w to 9% w/w.

In some embodiments, the neutral buffer solution comprises tromethamine, sodium acetate and sucrose, and has a pH value of 7-8.

In some embodiments, the content of tromethamine is selected from 10-30 mmol/L, preferably 15-25 mmol/L, preferably 15-20 mmol/L, for example, 15 mmol/L, 15.5 mmol/L, 16 mmol/L, 16.5 mmol/L, 17 mmol/L, 17.5 mmol/L, 18 mmol/L, 18.5 mmol/L, 19 mmol/L, 19.5 mmol/L, 20 mmol/L, 20.5 mmol/L, 21 mmol/L, 21.5 mmol/L, 22 mmol/L, 22.5 mmol/L, 23 mmol/L, 23.5 mmol/L, 24 mmol/L, 24.5 mmol/L, 25 mmol/L, and most preferably 20 mmol/L.

In some embodiments, the content of sodium acetate is selected from 0-20 mmol/L, preferably 5-11 mmol/L, for example, 5 mmol/L, 5.5 mmol/L, 6 mmol/L, 6.5 mmol/L, 7 mmol/L, 7.5 mmol/L, 8 mmol/L, 8.5 mmol/L, 9 mmol/L, 9.5 mmol/L, 10 mmol/L, 10.5 mmol/L, 10.6 mmol/L, 10.7 mmol/L, 10.8 mmol/L, 10.9 mmol/L, 11 mmol/L, 11.5 mmol/L, 12 mmol/L, 12.5 mmol/L, 13 mmol/L, and most preferably 10.7 mmol/L.

In some embodiments, the content of sucrose is selected from 5%-15%, preferably 7.5%-10%, more preferably 7.5%-9%, for example, 7.6%, 7.7%, 7.8%, 7.9%, 8.0%, 8.1%, 8.2%, 8.3%, 8.4%, 8.5%, 8.6%, 8.7%, 8.8%, 8.9%, 9%, 9.5%, 10%, and most preferably 8.7%.

In some embodiments, the neutral buffer solution comprises 20 mM of Tris, 10.7 mM of NaOAc and 8.7% of sucrose, and has a pH of 7.4-7.6. Preferably, in some embodiments, the neutral buffer solution comprises 20 mM of Tris, 10.7 mM of NaOAc and 8.7% of sucrose, and has a pH of 7.6.

The components of the above neutral buffer solution remain in the product, also known as final product buffer.

In some embodiments, the replacement process is selected from one or more of tangential flow filtration, membrane dialysis, column dialysis, and dilution.

In some embodiments, the two-stage buffer system replacement process comprises dialysis using an acidic buffer solution or a neutral buffer solution in the first stage and dialysis using a neutral buffer solution in the second stage.

In some embodiments, the two-stage buffer system replacement process comprises dilution with an acidic buffer solution in the first stage and dialysis with a neutral buffer solution in the second stage.

In some embodiments, the two-stage buffer system replacement process can be repeated multiple times, that is, it can be a process in which the pH value is alternately increased and decreased multiple times.

In the two-stage buffer system replacement process of the above embodiments, at the end of the first stage, the content of the organic solvent in the buffer system containing the precursor lipid nanoparticles is reduced to less than or equal to 5% (w/w), preferably less than 3% (w/w).

In the two-stage buffer system replacement process of the above embodiments, at the end of the first stage, the pH value of the buffer system containing the precursor lipid nanoparticles is lower than the pKa of the lipid nanoparticles by more than 0.5 units. For example, in some embodiments, the pKa of the lipid nanoparticles is 6.0-7.0, and at the end of the first stage of the two-stage buffer system replacement process, the pH value of the buffer system is lower than the pKa of the lipid nanoparticles by more than 0.5 units, for example, the pH value is 5.0-6.0.

In the two-stage buffer system replacement process of the above embodiments, at the end of the second stage, the content of the organic solvent in the buffer system containing the final product lipid nanoparticles is reduced to less than 1% (w/w), preferably less than 0.1% (w/w).

In the two-stage buffer system replacement process of the above embodiments, at the end of the second stage, the pH value of the buffer system containing the final product lipid nanoparticles is higher than the pKa of the lipid nanoparticles by more than 0.5 units. For example, in some embodiments, the pKa of the lipid nanoparticles is 6.0-7.0, and at the end of the second stage of the two-stage buffer system replacement process, the pH value of the buffer system is greater than the pKa of the lipid nanoparticles by more than 0.5 units, for example, the pH value is 7.0-8.0.

In some embodiments, the buffer system replacement includes a two-step buffer dialysis process. In some embodiments, the two-step buffer dialysis process comprises replacement with an acidic buffer solution in the first stage and replacement with a neutral buffer solution in the second stage; or replacement with a neutral buffer solution in the first stage and replacement with another neutral buffer solution in the second stage. The acidic buffer solution and the neutral buffer solution are as described above. In this process, when the pH value of the system is lower than the pKa of the lipid nanoparticles by more than 0.5 units through the first stage replacement, the content of the organic solvent in the system is reduced to a certain level (for example, less than or equal to 5%), so as to ensure that there is low residual organic solvent in the system when the particles undergo fusion. In the second stage replacement, the particles begin to fuse when the pH value of the system increases to near the pKa of the lipid nanoparticles, and the particle fusion is more complete at low content of organic solvent. When the pH value of the system is higher than the pKa of the lipid nanoparticles, the particle fusion is completed, forming thermodynamically stable particles (i.e., final product lipid nanoparticles).

In some embodiments, the buffer system replacement uses only one neutral buffer solution for dialysis (one-step buffer dialysis process), wherein the neutral buffer solution is as described above. Although this process uses only one neutral buffer solution, the pH value and buffering capacity of the neutral buffer solution can be controlled so that during the dialysis process, when the pH value of the system is lower than the pKa of the lipid nanoparticles by more than 0.5 units, the content of the organic solvent in the system is reduced to a certain level (for example, less than or equal to 5%) (i.e., the first stage replacement). The neutral buffer solution is then used for dialysis (the second stage replacement). When the pH value of the system increases to near the pKa of the lipid nanoparticles, the particles begin to fuse, and the particle fusion is more complete at low content of organic solvent. When the pH value of the system is higher than the pKa of the lipid nanoparticles, particle fusion is completed, forming thermodynamically stable particles (i.e., final product lipid nanoparticles).

In a specific embodiment, the method for the preparation of a lipid nanoparticle composition encapsulating a nucleic acid of the present invention comprises the following steps: (1) dissolving a lipid component (an ionizable cationic lipid, a helper lipid, a structured lipid and a surfactant) in an organic solvent to form a lipid phase; (2) dissolving a nucleic acid in an acidic buffer solution to form an aqueous phase; (3) mixing the lipid phase and the aqueous phase to allow the lipid to encapsulate the nucleic acid to form the precursor lipid nanoparticles; and (4) replacing the organic solvent-acidic buffer system containing the precursor lipid nanoparticles obtained in step (3) with a neutral buffer system in two stages, which comprises replacing with an acidic buffer solution or a neutral buffer solution in the first stage and replacing with a neutral buffer solution in the second stage, to obtain a composition of the final product lipid nanoparticles. In the first stage, the pH value of the system is maintained below the pKa of the lipid nanoparticles, and the content of the organic solvent in the system is reduced to a certain level through replacement with acidic buffer solution or neutral buffer solution, so as to ensure that there is low residual organic solvent in the system when the particles undergo fusion in the second stage. In the second stage, when the pH value of the system increases to near the pKa of the lipid nanoparticles through replacement with neutral buffer solution, the particles begin to fuse, and the particle fusion is more complete under low organic solvent content. When the pH value of the system is higher than the pKa of the lipid nanoparticles, the particle fusion is completed, forming thermodynamically stable particles (i.e., final product lipid nanoparticles).

In some embodiments, the organic solvent in step (1) is selected from C1-C4 low-carbon alcohols, preferably ethanol.

In some embodiments, the total concentration of the lipid component (ionizable cationic lipid, structured lipid, helper lipid and surfactant) in the lipid phase in step (1) is 10-15 mg/ml.

In some embodiments, the concentration of the nucleic acid in the aqueous phase in step (2) is 0.01-1 mg/ml, preferably 0.05-0.5 mg/ml, and more preferably 0.1-0.2 mg/ml.

In some embodiments, the acidic buffer solution in step (2) or step (4) comprises a buffering agent selected from the group consisting of acetates, formates, carbonates, phosphates, borates, succinates, gluconates, lactates, citric acid, glycine, leucine, barbituric acid, phthalic acid, tromethamine (Tris), triethylamine, ammonium salts and combinations thereof. The content/concentration of the buffering agent is determined according to the acidity/alkalinity of the specific type of buffering agent, so that the pH value of the acidic buffer solution is less than the pKa of the lipid nanoparticles. In some embodiments, the pKa of the lipid nanoparticles is 6.0-7.0, and the pH value of the acidic buffer solution in step (2) or step (4) is less than the pKa of the lipid nanoparticles, for example, the pH value of the acidic buffer solution is 3.0-5.0, preferably 4.0-5.0.

In some embodiments, the acidic buffer solution in step (2) or step (4) further comprises an osmotic pressure regulator, which is selected from sodium chloride, potassium chloride, and the like.

In some embodiments, the acidic buffer solution in step (2) or step (4) comprises citric acid and sodium chloride. Preferably, the buffer solution comprises 10-20 mM of citric acid and 120-140 mM of sodium chloride.

In some embodiments, the acidic buffer solution in step (2) or step (4) comprises 10 mM of citric acid and 130 mM of sodium chloride, with a pH of 3.9-4.1, for example, a pH of 4.0. In some embodiments, the acidic buffer solution in step (2) or step (4) comprises 20 mM of citric acid and 130 mM of sodium chloride, with a pH of 3.9-4.1, for example, a pH of 4.0. Preferably, in some embodiments, the acidic buffer solution in step (2) or step (4) comprises 10 mM of citric acid and 130 mM of sodium chloride, with a pH of 4.0; or comprises 20 mM of citric acid and 130 mM of sodium chloride, with a pH of 4.0.

In some embodiments, the volume ratio of the lipid phase and the aqueous phase mixed in step (3) is 1:2-1:9, preferably 1:2-1:5, preferably 1:3.

In some embodiments, the lipid phase and the aqueous phase are mixed in step (3) by a microfluidic mixer, which is selected from a staggered herringbone mixer (SHM), a T-junction mixer, and a microfluidic hydrodynamic focusing (MHF) mixer.

In some embodiments, the particle size of the precursor lipid nanoparticles in step (3) is 20-150 nm, preferably 30-100 nm, or 40-90 nm, or 50-80 nm.

In some embodiments, the particle dispersion index PDI of the precursor lipid nanoparticles in step (3) is 0.01-0.3, preferably 0.01-0.2, and more preferably 0.01-0.15.

In some embodiments, the encapsulation efficiency of the precursor lipid nanoparticles in step (3) is 50-100%, preferably 80%-100%, and more preferably 90%-100%.

In some embodiments, the replacement process in step (4) is selected from one or more of tangential flow filtration, membrane dialysis, column dialysis, and dilution.

In some embodiments, at the end of the first stage of the two-stage replacement process in step (4), the content of the organic solvent in the buffer system containing the precursor lipid nanoparticles is reduced to less than or equal to 5% (w/w), preferably less than 3% (w/w).

In some embodiments, at the end of the first stage of the two-stage replacement process in step (4), the pH value of the buffer system containing the precursor lipid nanoparticles is lower than the pKa of the lipid nanoparticles by more than 0.5 units. For example, in some embodiments, the pKa of the lipid nanoparticles is 6.0-7.0, and at the end of the first stage of the two-stage replacement process in step (4), the pH value of the buffer system is lower than the pKa of the lipid nanoparticles by more than 0.5 units, for example, the pH value of the buffer system is 5.0- 6.0.

In some embodiments, at the end of the second stage of the two-stage replacement process of step (4), the content of the organic solvent in the buffer system containing the final product lipid nanoparticles is reduced to less than 1% (w/w), preferably less than 0.1% (w/w).

In some embodiments, at the end of the second stage of the two-stage replacement process in step (4), the pH value of the buffer system containing the final product lipid nanoparticles is higher than the pKa of the lipid nanoparticles by more than 0.5 units. For example, in some embodiments, the pKa of the lipid nanoparticles is 6.0-7.0, and at the end of the second stage of the two-stage replacement process in step (4), the pH value of the buffer system is greater than the pKa of the lipid nanoparticles by more than 0.5 units, for example, the pH value of the buffer system is 7.0-8.0.

In some embodiments, the two-stage replacement process in step (4) can be repeated multiple times, that is, step (4) can be a process of alternately increasing and decreasing the pH value multiple times. Crossing above and below the pKa of lipid nanoparticles can improve the particle fusion effect.

In some embodiments, the neutral buffer solution in step (4) comprises a buffering agent selected from the group consisting of acetates, formates, carbonates, phosphates, borates, succinates, gluconates, lactates, citric acid, glycine, leucine, barbituric acid, phthalic acid, tromethamine (Tris), triethylamine, ammonium salts, and combinations thereof. The content/concentration of the buffering agent is determined based on the acidity/alkalinity of the specific type of buffering agent, so that the pH value of the neutral buffer solution is greater than the pKa of the lipid nanoparticles. In some embodiments, the pKa of the lipid nanoparticles is 6.0-7.0, and the pH value of the neutral buffer solution in step (4) is greater than the pKa of the lipid nanoparticles, for example, the pH value of the neutral buffer solution is 7.0-8.0.

In some embodiments, the neutral buffer solution in step (4) further comprises a cryoprotectant. The cryoprotectant can be selected from substances such as sugars, polyols, polymers, surfactants, amino acids, and salts, wherein the sugars can be selected from lactose, sucrose, trehalose, galactose, and the like.

In some embodiments, the amount of the cryoprotectant is from 1% w/w to 50% w/w of the buffer solution, such as from 2% w/w to 50% w/w, or from 4% w/w to 45% w/w, or from 6% w/w to 12% w/w, preferably from 6% w/w to 10% w/w, and most preferably from 7% w/w to 9% w/w.

In some embodiments, the neutral buffer solution comprises tromethamine, sodium acetate and sucrose, and has a pH value of 7-8.

In some embodiments, the content of tromethamine is selected from 10-30 mmol/L, preferably 15-25 mmol/L, preferably 15-20 mmol/L, for example, 15 mmol/L, 15.5 mmol/L, 16 mmol/L, 16.5 mmol/L, 17 mmol/L, 17.5 mmol/L, 18 mmol/L, 18.5 mmol/L, 19 mmol/L, 19.5 mmol/L, 20 mmol/L, 20.5 mmol/L, 21 mmol/L, 21.5 mmol/L, 22 mmol/L, 22.5 mmol/L, 23 mmol/L, 23.5 mmol/L, 24 mmol/L, 24.5 mmol/L, 25 mmol/L, and most preferably 20 mmol/L.

In some embodiments, the content of sodium acetate is selected from 0-20 mmol/L, preferably 5-11 mmol/L, for example, 5 mmol/L, 5.5 mmol/L, 6 mmol/L, 6.5 mmol/L, 7 mmol/L, 7.5 mmol/L, 8 mmol/L, 8.5 mmol/L, 9 mmol/L, 9.5 mmol/L, 10 mmol/L, 10.5 mmol/L, 10.6 mmol/L, 10.7 mmol/L, 10.8 mmol/L, 10.9 mmol/L, 11 mmol/L, 11.5 mmol/L, 12 mmol/L, 12.5 mmol/L, 13 mmol/L, and most preferably 10.7 mmol/L.

In some embodiments, the content of sucrose is selected from 5%-15%, preferably 7.5%-10%, more preferably 7.5%-9%, for example, 7.6%, 7.7%, 7.8%, 7.9%, 8.0%, 8.1%, 8.2%, 8.3%, 8.4%, 8.5%, 8.6%, 8.7%, 8.8%, 8.9%, 9%, 9.5%, 10%, and most preferably 8.7%.

In some embodiments, the neutral buffer solution in step (4) comprises 20 mM of Tris, 10.7 mM of NaOAc and 8.7% of sucrose, and has a pH of 7.4-7.6. Preferably, in some embodiments, the neutral buffer solution in step (4) comprises 20 mM of Tris, 10.7 mM of NaOAc and 8.7% of sucrose, and has a pH of 7.6.

The components of the neutral buffer solution of the above step (4) remain in the product, also known as final product buffer.

In some embodiments, the preparation method as described above optionally comprises step (5) concentration, dilution, sterile filtration, aseptic filling and a combination thereof. The concentration, dilution and aseptic filling steps are used to make the nucleic acid drugs meet the specifications and dosages for administration, and the sterile filtration step is used to make the nucleic acid drugs meet the requirements on microorganisms.

The preparation method of the nucleic acid-containing lipid nanoparticle composition of the present invention is characterized in the preparation of the precursor lipid nanoparticles by mixing of lipid phase and aqueous phase and the buffer solution replacement. The formation of the thermodynamically stable nucleic acid-containing lipid nanoparticles depends on the particle fusion during the buffer system replacement process. Reducing the content of the organic solvent in the system to less than or equal to 5% (w/w) is beneficial for complete particle fusion, thereby obtaining thermodynamically stable lipid nanoparticles. Advantageously, by buffer system replacement, the content of organic solvent in the buffer system containing the precursor lipid nanoparticles is reduced to less than or equal to 5% (w/w) when the pH value of the system is lower than the pKa of the lipid nanoparticles by more than 0.5 units.

Beneficial effects of the invention

The present application provides lipid nanoparticles encapsulating nucleic acids with low no-load rate by improving the degree of particle fusion during the preparation process, and provides a new method for improving the stability and in vivo behavior of nucleic acid drugs. After complete fusion, the morphology and structure of the particles are more uniform, and the particles are more stable. The obtained mRNA LNPs can be stored for at least 6 months under refrigeration conditions of 2 to 8°C and at least 12 months under frozen conditions of -15°C to -25°C, showing a stability significantly better than that of the commerical available medicaments, and is convenient for storage, transportation and circulation. Compared with particles prepared by traditional methods, the lipid nanoparticles encapsulating nucleic acids with low no-load rate of the present invention are basically effective loads encapsulating nucleic acids, which is beneficial for improving the efficiency of nucleic acid delivery, reducing the risk of inflammatory adverse reactions caused by lipid components, and improving the effectiveness and safety of nucleic acid drugs. In addition, said lipid nanoparticles encapsulating nucleic acids with low no-load rate are more likely to be retained at the injection site, and have reduced distribution in the blood and non-target organs (such as the heart), thereby reducing the risk of adverse reactions caused by distribution in non-target organs.

**Definitions of terms**

Unless otherwise specified, the terms used in the specification and claims of the present application have the meanings below. It should be understood that, in the case where the terms are not clearly defined herein, they should be given their meanings known in the art. Further, it should be understood that, the terms used herein are only for the purpose of describing specific embodiments and are not intended to limit the scope of protection of the present invention in any way.

Ionizable cationic lipid

The term "ionizable cationic lipid" as used herein refers to a specific type of lipid that is ionizable. Under conditions with a pH lower than the pKa value, it is positively charged and can complex with negatively charged RNA to make RNA encapsulated within LNP for protection. Under neutral physiological conditions with a pH higher than the pKa value, it does not carry any charge, making the LNP electrically neutral and having the ability to be low-toxic and stealth, thereby enabling the RNA to enter the cell through the endosome pathway. In acidic endosomes, the ionizable cationic lipid switches to be positively charged, interacts with the negatively charged phospholipids on the endosome membrane and disrupts the endosome, achieving endosome escape and the release of RNA in the cytoplasm.

pKa of lipid nanoparticles

The term "pKa of lipid nanoparticles" as used herein refers to the apparent ionization constant of lipid nanoparticles comprising a specific ionizable cationic lipid. The pKa value of the lipid nanoparticles comprising a specific ionizable cationic lipid is associated with the tolerability at the injection site, safety in the body, stability and intracellular delivery effect of the lipid nanoparticles. The pKa value of lipid nanoparticles containing a specific ionizable cationic lipid can be determined using the commonly used 6-(p-toluidino)-2-sulfonic acid sodium salt (TNS) probe as reported in literatures. The pH value corresponding to the point at which the TNS fluorescence signal is abruptly changed with pH value is defined as the pKa value of the lipid nanoparticles containing the ionizable cationic lipid.

Precursor lipid nanoparticles

The term "precursor lipid nanoparticles" as used herein refers to lipid nanoparticles in intermediate form presented in the preparation process of LNP containing an ionizable cationic lipid, which are self-assembled by the lipid in the organic phase and the nucleic acid in the aqueous phase after rapid mixing. After mixing, as the pH value of the system is significantly lower than the pKa of the ionizable cationic lipid, the ionizable cationic lipid is protonated and positively charged, and generates electrostatic attraction with the negatively charged nucleic acid. At the same time, due to the poor water solubility of the lipid, hydrophobic interactions are generated, driving the lipid to encapsulate the nucleic acid to form the precursor lipid nanoparticles by self-assembly. The precursor lipid nanoparticles undergo fusion with changes in the pH value of the system during subsequent dialysis or ultrafiltration to form final product lipid nanoparticles.

N:P Ratio

The term "N:P ratio" as used herein refers to the molar ratio of the protonable nitrogen element of the ionizable cationic lipid to the phosphate group of the mRNA. The N:P ratio describes the ratio of the cationic charge of the amino group ($N^+$) in the ionizable cationic lipid to the anionic charge of the phosphate group ($PO_4^-$) in the nucleic acid backbone, and is the basis for the complexation of the ionizable cationic lipid with the nucleic acid through electrostatic interaction. The N:P ratio is a key formulation factor for LNPs, affecting the physicochemical properties of LNPs and the in vivo release of drugs.

Encapsulation efficiency

The term "encapsulation efficiency" as used herein refers to the ratio of the nucleic acids encapsulated inside the lipid nanoparticles to all the nucleic acids. Encapsulation efficiency is usually determined by fluorescence spectrophotometry. Taking RNA as an example, the amount of free RNA outside the lipid nanoparticles in the LNP-RNA solution is firstly determined with RiboGreen fluorescent dye, then the structures of the lipid nanoparticles are destroyed with Triton-100 to release RNA into the external solution, and the total amount of RNA in the solution is determined. The difference between the two amounts is the amount of RNA encapsulated inside the LNP particles, which is further used to obtain the encapsulation efficiency.

No-load rate

The term "no-load rate" as used herein refers to the ratio of the lipid nanoparticles without nucleic acids to all the lipid nanoparticles. In the present invention, the no-load rate is determined by nanoflow cytometry, which comprises firstly determining the number of all lipid nanoparticles in the sample by nanoflow cytometry, then adding Ribozymes in the sample to remove free nucleic acids, adding nucleic acid dyes capable of passing through the membrane, which penetrate the LNP membrane and combine with the nucleic acids inside the LNP to produce fluorescence, so as to obtain the number of lipid nanoparticles encapsulating nucleic acids, and then calculating the ratio of LNPs without nucleic acids, that is, the no-load rate.

Nucleic Acid

The term "nucleic acid" or "nucleic acid molecule" has a meaning recognized and understood by those of ordinary skill in the art. The term "nucleic acid" as used herein refers to a polymer containing at least two deoxyribonucleotides or ribonucleotides in single-stranded or doublestranded form, including DNA and RNA. DNA can be in the following forms: for example, antisense molecules, plasmid DNA, pre-concentrated DNA, PCR products, vectors (P1, PAC, BAC, YAC, artificial chromosomes), expression cassettes, chimeric sequences, chromosomal DNA, or derivatives and combinations thereof. RNA can be in the following forms: siRNA, asymmetric interfering RNA (aiRNA), microRNA (miRNA), mRNA, tRNA, rRNA, tRNA, viral RNA (vRNA), or a combination thereof. Nucleic acid includes those containing known nucleotide analogs or modified backbone residues or bonds, which are synthetic, naturally occurring, or non-naturally occurring, and which have binding properties similar to reference nucleic acids. Examples of the analogs include, but are not limited to, phosphorothioates, phosphoramidates, methyl phosphonates, chiral-methyl phosphonates, 2'-O-methyl ribonucleotides and peptide-nucleic acids (PNAs). Unless otherwise limited, the term includes nucleic acid containing known analogs of natural nucleotides having binding properties similar to those of reference nucleic acids. Unless otherwise indicated, a specific nucleic acid sequence also inherently includes conservatively modified variants thereof (e.g., degenerate codon substitutions), alleles, orthologs, SNPs, and complementary sequences as well as the sequences explicitly indicated. In particular, degenerate codon substitutions can be achieved by generating sequences in which the third position of one or more selected (or all) codons is replaced by mixed basic and/or deoxyinosine residues (Batzer et al., Nucleic Acid Res., 19: 5081 (1991); Ohtsuka et al., J. Biol. Chem., 260: 2605-2608 (1985); Rossolini et al., Mol. Cell. Probes, 8: 91-98 (1994)). "Nucleotide" includes deoxyribonucleic acid (DNA) or ribonucleic acid (RNA), a base, and a phosphate group. The nucleotides are linked together by the phosphate group. "Bases" include purines and pyrimidines, which further include the natural compounds adenine, thymine, guanine, cytosine, uracil, inosine, and natural analogs, as well as synthetic derivatives of purines and pyrimidines, which include, but are not limited to, modifications that introduce new reactive groups such as, but not limited to, amines, alcohols, thiols, carboxylates, and alkyl halides.

DNA that can be used in the present application is not particularly limited and can be appropriately selected according to the purpose of use. Examples thereof include, for example, plasmid DNA, cDNA, antisense DNA, chromosome DNA, PAC, BAC, etc., preferably plasmid DNA, cDNA, antisense DNA, and more preferably plasmid DNA. Circular DNA such as plasmid DNA can also be appropriately digested with restriction enzymes and used in the form of linear DNA.

RNA that can be used in the present application is not particularly limited and can be appropriately selected according to the purpose of use. Examples thereof include, for example, siRNA, miRNA, shRNA, antisense RNA, messenger RNA (mRNA), single-stranded RNA genome, doublestranded RNA genome, RNA replicon, transfer RNA, ribosomal RNA, etc., preferably siRNA, miRNA, shRNA, mRNA, antisense RNA, and RNA replicon.

The source of the nucleic acid that can be used in the present application is not particularly limited and can be appropriately selected according to the purpose of use. For example, commercially available nucleic acid products can be used, or nucleic acids can be prepared with reference to the methods disclosed in the prior art. In some embodiments, the mRNA used in the present application is prepared with reference to the method described in Chinese patent application 202111445859.X filed on November 30, 2021, which is incorporated herein as a whole for all purposes.

Although the numerical ranges and parameter approximations shown in the present invention are within a wide range, the numerical values shown in the specific embodiments are recorded as accurately as possible. However, any numerical value is bound to contain a certain error, which is caused by the standard deviation in their respective measurements. In addition, all ranges disclosed herein should be understood to cover any and all sub-ranges contained therein. For example, the range of "1 to 10" should be considered to include any and all sub-ranges between the minimum value 1 and the maximum value 10 (including the endpoints); that is, all sub-ranges starting with a minimum value of 1 or greater, such as 1 to 6.1, and sub-ranges ending with a maximum value of 10 or less, such as 5.5 to 10. In addition, any reference referred to as "incorporated herein" should be understood to be incorporated in its entirety.

In order to provide a more concise description, some quantitative data in the context are not modified with the term "about". It should be understood that, whether the term "about" is explicitly used or not, each numerical value given here includes not only the actual given value (given value), but also an approximate value of such given value that is reasonably inferred by a person of ordinary skill in the art, including equivalents and approximate values of such given value resulted from experimental and/or measurement conditions. The approximate value is preferably $\pm 20\%$, $\pm 15\%$, $\pm 10\%$, $\pm 8\%$, $\pm 6\%$, $\pm 5\%$, $\pm 4\%$, $\pm 3\%$, 2%, $\pm 1\%$ based on the given value.

It should also be noted that, as used in the specification, singular forms include plural forms of their referents, unless clearly and unambiguously limited to one referent. The term "or" can be used interchangeably with the term "and/or" unless the context clearly indicates otherwise.

Those skilled in the art should understand that the particle size of the particles can be measured using any existing or potential appropriate method, including but not limited to sedimentation method, sieving method, microscopic observation, or laser particle size analyzer. It should also be understood that, when the nanoparticles of the present disclosure are multiple, it is not required that the particle size of each nanoparticle be consistent, as long as the average particle size thereof meets the above limitation, that is, included in the scope covered by the present disclosure. In some specific embodiments, the particle size is determined using a laser particle size analyzer.

Other embodiments of the present invention include:

1. A lipid nanoparticle composition for encapsulating a nucleic acid, characterized in that the ratio of empty lipid nanoparticles containing no nucleic acid to the total number of lipid nanoparticles, that is, the no-load rate is not more than 10%, preferably not more than 9%, more preferably not more than 8.5%, still more preferably not more than 6%, further preferably not more than 3%, or is any value within the above ranges, for example, 2%, 3%, 4%, 5%, 6%, 7%, 8%, 9%, 7.1%, 7.6%, 7.7%, 7.9%, 8.3%.

2. The composition as described in embodiment 1, characterized in that the average particle size of the lipid nanoparticles is 50 nm~150 nm, preferably 70 nm~120 nm, more preferably 90 nm~110 nm, or any value within the above ranges, for example, 96 nm, 97 nm, 98 nm, 99 nm, 100 nm, 101 nm, 102 nm, 103 nm, 104 nm, 105 nm, 106 nm, 107 nm, 109 nm, 110 nm, 111 nm, 112 nm, 113 nm, 114 nm, 115 nm, 116 nm, 117 nm, 119 nm, 120 nm.

3. The composition as described in embodiment 1, characterized in that the encapsulation efficiency of the lipid nanoparticles is above 80%, preferably above 85%, and more preferably above 90%.

4. The composition as described in embodiment 1, characterized in that the nanoparticles comprise (1) a nucleic acid, and (2) a lipid component including an ionizable cationic lipid, a helper lipid, a structured lipid and a surfactant.

5. The composition as described in embodiment 4, characterized in that the lipid component comprises 20-60 mol% of the ionizable cationic lipid, 25-55 mol% of the structured lipid, 2-25 mol% of the helper lipid and 0.5-15 mol% of the surfactant.

6. The composition as described in embodiment 5, characterized in that the cationic lipid is selected from the group consisting of SM-102, ALC-0315, Dlin-MC3-DMA, DODMA, C12-200 and DlinDMA, the structured lipid is selected from cholesterol or cholesterol derivatives, the helper lipid is selected from the group consisting of DSPC, DOPE, DOPC, DOPG and DOPS, and the surfactant is selected from the group consisting of mPEG-DMG-2K, ALC-0159, PEG-DSPE, DTDA-PEG2000 and TPGS.

7. The composition as described in embodiment 5, characterized in that the N:P ratio of the lipid nanoparticles is from about 2:1 to about 30:1, preferably from about 2:1 to about 15:1, more preferably from about 2:1 to about 10:1, and more preferably from about 3:1 to about 6:1.

8. The composition as described in embodiment 4, characterized in that the weight ratio of the ionizable cationic lipid component to the nucleic acid is from about 5:1 to about 100:1, preferably from about 5:1 to about 50:1, preferably from about 5:1 to about 30:1, and more preferably from about 10:1 to about 20:1.

9. The composition as described in embodiment 1, characterized in that the lipid nanoparticle composition further comprises a final product buffer containing a buffering agent and/or a cryoprotectant.

10. The composition as described in embodiment 9, characterized in that the buffering agent is selected from acetates, formates, carbonates, phosphates, borates, succinates, gluconates, lactates, citric acid, glycine, leucine, barbituric acid, phthalic acid, tromethamine (Tris), triethylamine, ammonium salts or a combination thereof, so that the pH value of the buffer solution is 7-8, or

the cryoprotectant is selected from substances such as sugars/polyols, polymers, surfactants, amino acids and salts, wherein the sugars are selected from lactose, sucrose, trehalose, galactose and the like.

11. The composition as described in embodiment 9, characterized in that the final product buffer comprises tromethamine, sodium acetate and sucrose, and has a pH value of 7-8.

12. The composition as described in embodiment 1, characterized in that the nucleic acid is selected from an mRNA comprising a nucleotide sequence having at least 80%, at least 85%, at least 90%, at least 95% or at least 98% or at least 99% or 100% identity to any one of the nucleotide sequence of SEQ ID NO:1 to SEQ ID NO:6, preferably SEQ ID NO:1, or selected from an mRNA encoding the coronavirus antigen which comprises an amino acid sequence having at least 80%, at least 85%, at least 90%, at least 95% or at least 98% or at least 99% or 100% identity to the amino acid sequence of SEQ ID NO:7, or selected from an mRNA comprising a nucleotide sequence having at least 80%, at least 85%, at least 90%, at least 95% or at least 98% or at least 99% or 100% identity to any one of the nucleotide sequence of SEQ ID NOs:8 to 26.

13. The composition as described in embodiment 12, characterized in that the composition comprises lipid nanoparticles encapsulating a nucleic acid, 20 mmol/L of tromethamine, 10.7 mmol/L of sodium acetate and 8.7% of sucrose, and has a pH of 7.0-8.0, wherein the concentration of the mRNA is 100 μg/ml; the lipid nanoparticles comprise 50 mol% of SM-102, 10 mol% of DSPC, 38.5 mol% of cholesterol and 1.5 mol% of mPEG-DMG-2K, and the no-load rate is not more than 10%.

14. A preparation method of the above lipid nanoparticle composition, mainly comprising the following steps:
(1) dissolving the lipid component in an organic solvent to form an organic phase; (2) dissolving the nucleic acid in an acidic buffer solution to form an aqueous phase; (3) mixing the organic phase and the aqueous phase to form precursor lipid nanoparticles by encapsulating the nucleic acid with the lipid; (4) replacing the organic solvent-acidic buffer system containing the precursor lipid nanoparticles obtained in step (3) with a neutral buffer system in two stages to obtain final product lipid nanoparticles, wherein the first stage comprises replacement with an acidic buffer solution or a neutral buffer solution, and the second stage comprises replacement with a neutral buffer solution.

15. The preparation method as described in embodiment 14, characterized in that, in the two-stage replacement process in step (4), the content of the organic solvent in the buffer system containing the precursor lipid nanoparticles is reduced to less than or equal to 5% (w/w), preferably less than 3% (w/w) at the end of the first stage.

16. The preparation method as described in embodiment 14, characterized in that, in the two-stage replacement process in step (4), the pH value of the buffer system containing the precursor lipid nanoparticles is lower than the pKa of the lipid nanoparticles by more than 0.5 units at the end of the first stage.

17. The preparation method as described in embodiment 14, characterized in that, in the two-stage replacement process in step (4), the content of the organic solvent in the buffer system containing the final product lipid nanoparticles is reduced to less than 1% (w/w), preferably less than 0.1% (w/w) at the end of the second stage.

18. The preparation method as described in embodiment 14, characterized in that, in the two-stage replacement process in step (4), the pH value of the buffer system containing the final product lipid nanoparticles is higher than the pKa of the lipid nanoparticles by more than 0.5 units at the end of the second stage.

19. The preparation method as described in embodiment 14, characterized in that the preparation method optionally comprises step (5) concentration, dilution, sterile filtration, aseptic filling and the combination thereof.

20. The preparation method as described in embodiment 14, characterized in that the organic solvent in step (1) is selected from C1-C4 low-carbon alcohols, preferably ethanol.

21. The preparation method as described in embodiment 14, characterized in that the total concentration of the lipid component in the organic phase in step (1) is 10-15 mg/ml.

22. The preparation method as described in embodiment 14, characterized in that the concentration of the nucleic acid in the aqueous phase in step (2) is 0.01-1 mg/ml, preferably 0.05-0.5 mg/ml, preferably 0.1-0.2 mg/ml.

23. The preparation method as described in embodiment 14, characterized in that the acidic buffer solution in step (2) or step (4) comprises a buffering agent, which is selected from acetates, formates, carbonates, phosphates, borates, succinates, gluconates, lactates, citric acid, glycine, leucine, barbituric acid, phthalic acid, tromethamine (Tris), triethylamine ammonium salts, sodium chloride, potassium chloride or a combination thereof, so that the pH value of the acidic buffer solution is less than the pKa of the lipid nanoparticles.

24. The preparation method as described in embodiment 14, characterized in that the acidic buffer solution in step (2) or step (4) further comprises an osmotic pressure regulator, which is selected from sodium chloride, potassium chloride, etc.

25. The preparation method as described in embodiment 14, characterized in that the pKa of the lipid nanoparticles is 6.0-7.0, and the pH value of the acidic buffer solution in step (2) or step (4) is less than the pKa of the lipid nanoparticles, for example, is 3.0-5.0, preferably 4.0-5.0.

26. The preparation method as described in embodiment 14, characterized in that the acidic buffer solution in step (2) or step (4) comprises citric acid and sodium chloride, preferably, comprises 10-20 mM of citric acid and 120-140 mM of sodium chloride.

27. The preparation method as described in embodiment 14, characterized in that the volume ratio of the organic phase and the aqueous phase for mixing in step (3) is 1:2-1:9, preferably 1:2-1:5, preferably 1:3.

28. The preparation method as described in embodiment 14, characterized in that the replacement process in step (4) is selected from one or more of tangential flow filtration, membrane dialysis, column dialysis, and dilution.

29. The preparation method as described in embodiment 14, characterized in that the two-stage replacement process in step (4) can be repeated multiple times, that is, step (4) can be a process in which the pH value is alternately increased and decreased multiple times, and crossing above and below the pKa of the lipid nanoparticles can reduce the no-load rate of the particles.

30. The preparation method as described in embodiment 14, characterized in that the neutral buffer solution in step (4) comprises a buffering agent selected from the group consisting of acetates, formates, carbonates, phosphates, borates, succinates, gluconates, lactates, citric acid, glycine, leucine, barbituric acid, phthalic acid, tromethamine (Tris), triethylamine, ammonium salts, sucrose and combinations thereof, so that the pH value of the neutral buffer solution is greater than the pKa of the lipid nanoparticles.

31. The preparation method as described in embodiment 14, characterized in that the pKa of the lipid nanoparticle is 6.0-7.0, and the pH value of the neutral buffer solution in step (4) is greater than the pKa of the lipid nanoparticles, for example, is 7.0-8.0.

Description of the drawings

FIG.1 shows the structure of the linearized plasmid template of the T7 promoter in Preparation Example 1;

FIG.2 shows the curve of fluorescence intensity as a function of pH value during the determination of the pKa value of lipid nanoparticles containing SM-102 by TNS method;

FIG.3 shows curves of particle size and PDI of lipid nanoparticles containing SM-102 as a function of pH value in the two-stage dialysis process;

FIG.4 shows curves of particle size of lipid nanoparticles containing different cationic lipids as a function of dialysis fold during buffer solution replacement;

FIG. 5 shows curves of PDI of lipid nanoparticles containing different cationic lipids as a function of dialysis fold during buffer solution replacement;

FIG.6 shows the curve of particle size of LNP as a function of dialysis process during reverse dialysis;

FIG.7 shows respectively the fluorescence nanoflow cytometry graphs of intermediates in the LNP preparation process after two-phase mixing (A), after dialysis with pH 4.0 buffer solution (B), and after dialysis with pH 7.6 buffer solution (C) according to embodiment 1-1, wherein the abscissa SS-H represents the peak height of the scattering signal, the ordinate FITC-A represents the peak area of the fluorescence signal, P1 represents the percentage of loaded LNPs, and P2 represents the percentage of no-load LNPs;

FIG.8 shows cryo-TEM images of intermediates in the LNP preparation process after two-phase mixing, after dialysis with pH 4.0 buffer solution, and after dialysis with pH 7.6 buffer solution according to embodiment 1-1;

FIG.9 shows a typical cryo-TEM image of the final product lipid nanoparticles of embodiment 1-1;

FIG.10 shows a typical cryo-TEM image of the final product lipid nanoparticles of comparative embodiment 1-1;

FIG.11 shows the pseudovirus neutralizing antibody activity of immune serum obtained after immunizing mice with the lipid nanoparticle compositions prepared by embodiment 1-1 and comparative embodiment 1-1;

FIG.12 shows a comparison of the in vitro cell transfection efficacy of luciferase mRNA-LNPs prepared according to the methods of embodiment 1-1 and comparative embodiment 1-1;

FIG.13 shows the tissue distribution results of lipid nanoparticles prepared according to the methods of embodiment 1-1 and comparative embodiment 1-1 in mice after administration.

Examples

In order to further understand the present invention, the specific embodiments of the present invention are described in detail below in conjunction with examples. However, it should be understood that the description is only intended to further illustrate the features and advantages of the present invention, and does not constitute any limitation of the present invention.

Preparation Example 1 Preparation of mRNA

An exemplary method for preparing mRNA used in the present application is as follows:

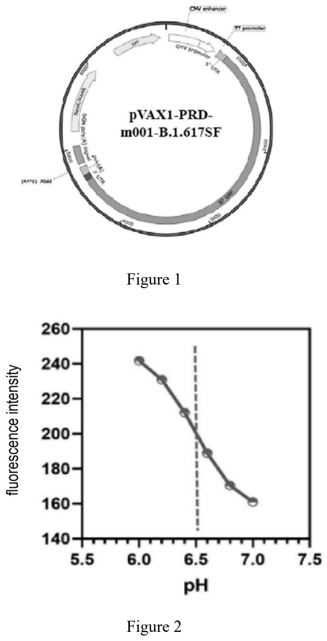

1. Preparing the IVT reaction system according to the instruction of the IVT kit (E131, Novoprotein) by mixing $10\times$ Transcription Buffer solution, ATP, GTP, CTP, 1-N-Me-Pseudo UTP (Cat. No.: WA0992, Hongene BioTech), 5' cap analog m7G(5')ppp(5')(2'OMeA)pG (Cat. No.: GAGNH23C2L1B, Hongene BioTech), water for injection, linearized plasmid template of T7 promoter (customized by GenScript Biotech Co., Ltd., see FIG.1 for the structure) and Enzyme Mix, wherein the selected sequences are as follows:

| mRNA | 5'UTR | ORF | 3'UTR | PolyA sequence and the subsequent elements | Relative expression level |
|---|---|---|---|---|---|
| SEQ ID NO: 1 | alpha-1-globin 5'UTR | optimized ORF-1 GC%=54.98% | gp130 and DH143 3'UTR | 100A | 100% |

2. placing the mixed reaction system at 37°C and reacting for 40 minutes; and

3. adding corresponding percentage of DNase I to terminate the reaction.

S protein mRNA was synthesized in vitro, separated and purified by hydrophobic chromatography and ultrafiltration concentration to obtain high-purity mRNA (>95%). The experimental results showed that high-purity mRNA was obtained through above steps, which can be used for subsequent experiments. Note: Internal standard (25nt, Agilent, RNA 6000 Nano Kit (reorder-no 5067-1511)).

Example 1 Preparation of lipid nanoparticles by two-step dialysis process

This Example illustrates a method for preparing lipid nanoparticles with low no-load rate according to the present invention, wherein the precursor lipid nanoparticles were dialyzed in two steps with a pH 4.0 buffer solution and a pH 7.6 buffer solution to obtain final product lipid nanoparticles. The specific preparation method was shown below.

1. Preparation of precursor lipid nanoparticles

1.1 Preparation of the lipid phase: four lipids of SM-102, mPEG-DMG-2K, DSPC and cholesterol were weighed accurately according to a molar ratio of 50%: 1.5%: 10%: 38.5%, and dissolved in anhydrous ethanol to obtain a lipid phase with a total lipid concentration of 12 mg/ml;

1.2 Preparation of the aqueous phase: the mRNA stock solution obtained in Preparation Example 1 was diluted with 10 mM citric acid-130 mM sodium chloride buffer solution (pH 4.0) to obtain an aqueous phase with mRNA concentration of 0.18 mg/ml;

1.3 Mixing: the lipid phase and the aqueous phase were conveyed by a constant-flux pump and mixed in a microchannel mixer at a volume ratio of 1:3, by which the lipids encapsulated the mRNA to form a suspension of precursor lipid nanoparticles.

2. Preparation of final product lipid nanoparticles

2.1 Dialysis with pH 4.0 buffer solution: the suspension of precursor lipid nanoparticles was dialyzed 1 to 5 times with equal volume using 10 mM citric acid-130 mM sodium chloride buffer solution (pH 4.0) as the first stage dialysis solution through tangential flow filtration (TFF) to reduce the ethanol content in the system and produce a solution after dialysis with pH 4.0 buffer solution;

2.2 Dialysis with pH 7.6 buffer solution: the solution after dialysis with pH 4.0 buffer solution was dialyzed 3 to 6 times with equal volume using 20mM Tris-10.7mM NaOAc-8.7% sucrose solution (pH 7.6) as the second stage dialysis solution through tangential flow filtration (TFF). During the increase of the pH value, the particles fused to form stable final product lipid nanoparticles.

In this method, during the first stage of dialysis with pH 4.0 buffer solution, the pH value of the system was maintained below the pKa of the lipid nanoparticles, and the ethanol content was gradually reduced to a certain level to ensure that the ethanol content in the system was low when the particles fused in the second stage. During the second stage of dialysis with pH 7.6 buffer solution, when the pH value of the system increased to near the pKa of the lipid nanoparticles, the particles begined to fuse, and the particle fusion was more complete at the low ethanol content; when the pH value of the system was higher than the pKa of the lipid nanoparticles, the particle fusion was completed, forming thermodynamically stable final product lipid nanoparticles.

Some specific embodiments of Example 1 were shown in Table 1.

Table 1 Specific embodiments of Example 1

| No. | Concentration of citric acid | Concentration of Tris | Replacement process of the buffer system | | Summary of the embodiment |
|---|---|---|---|---|---|
| | | | Step 1 of the process | Step 2 of the process | |
| Embodiment 1-1 | 10 mM | 20 mM | Dialyzed 3 times with equal volume by TFF using pH4.0 buffer solution | Dialyzed 5 times with equal volume by TFF using pH7.6 buffer solution | Standard two-step dialysis |
| Embodiment 1-2 | 10 mM | 20 mM | Dialyzed 1 time with equal volume by TFF using pH4.0 buffer solution | Dialyzed 6 times with equal volume by TFF using pH7.6 buffer solution | two-step dialysis, the dialysis fold is adjusted |

(continued)

| No. | Concentration of citric acid | Concentration of Tris | Replacement process of the buffer system | | Summary of the embodiment |
| --- | --- | --- | --- | --- | --- |
| | | | Step 1 of the process | Step 2 of the process | |
| Embodiment 1-3 | 10 mM | 20 mM | Dialyzed 2 times with equal volume by TFF using pH4.0 buffer solution | Dialyzed 5 times with equal volume by TFF using pH7.6 buffer solution | two-step dialysis, the dialysis fold is adjusted |

Comparative Example 1 Preparation of lipid nanoparticles by one-step dialysis process

This Comparative Example illustrates a conventional method for preparing lipid nanoparticles, wherein the precursor lipid nanoparticles were dialyzed with a pH 7.6 buffer solution in one step to obtain the final product lipid nanoparticles. The specific preparation method was shown below.

　　1. Preparation of the precursor lipid nanoparticles in a manner the same as that in Example 1.
　　2. Preparation of final product lipid nanoparticles: the suspension of the precursor lipid nanoparticles was dialyzed 5 to 8 times with equal volume using 20mM Tris-10.7mM NaOAc-8.7% sucrose solution (pH 7.6) or a buffer solution at higher concentration as the dialysis solution through tangential flow filtration (TFF). During the increase of the pH value, the particles fused to form stable final product lipid nanoparticles.

In this method, during the one-step dialysis process with pH 7.6 buffer solution, when the pH value of the system increased to near the pKa of lipid nanoparticles, the particles began to fuse. However, the particles could not fuse completely due to the high ethanol content, forming thermodynamically unstable final product lipid nanoparticles.
Some specific embodiments of Comparative Example 1 were shown in Table 2.

Table 2 Specific embodiments of Comparative Example 1

| No. | Concentraion of citric acid | Concentraion of Tris | Replacement process of the buffer system | | |
| --- | --- | --- | --- | --- | --- |
| | | | Step 1 of the process | Step 2 of the process | Summary of the embodiment |
| Comparative embodiment 1-1 | 10 mM | 20 mM | Dialyzed 8 times with equal volume by TFF using pH7.6 buffer solution | N/A | Standard one-step dialysis |
| Comparative embodiment 1-2 | 10 mM | 60 mM | Dialyzed 5 times with equal volume by TFF using pH7.6 buffer solution | N/A | one-step dialysis, improving the buffering ability of the neutral buffer solution |

Example 2 Preparation of lipid nanoparticles by two-step process of dilution followed by dialysis

This Example illustrates an alternative method for preparing the lipid nanoparticles with low no-load rate of the present invention, wherein the the precursor lipid nanoparticles were diluted with a pH 4.0 buffer solution and dialyzed with a pH 7.6 buffer solution to obtain the final product lipid nanoparticles. The specific preparation method was shown below.

　　1. Preparation of the precursor lipid nanoparticles in a manner the same as that in Example 1.
　　2. Preparation of final product lipid nanoparticles

　　　2.1 Dilution with pH 4.0 buffer solution: the suspension of the precursor lipid nanoparticles was diluted 10 times with 10 mM citric acid-130 mM sodium chloride buffer solution (pH 4.0) (during which the ethanol content in the system was reduced without increasing the pH value) to obtain a solution diluted with pH 4.0 buffer solution;
　　　2.2 Dialysis with pH 7.6 buffer solution: the solution diluted with pH 4.0 buffer solution was concentrated 10 times by tangential flow filtration (TFF), then dialyzed 4 to 6 times with equal volume using 20 mM Tris-10.7 mMNaOAc-8.7% sucrose solution (pH 7.6) as the dialysis solution. During the increase of the pH value, the particles fused to form stable final product lipid nanoparticles.

In this method, the pH value of the system was lower than the pKa of the lipid nanoparticles after the dilution with pH 4.0 buffer solution in the first stage, and the ethanol content was reduced to a certain level, ensuring that the ethanol content in the system was low when the particles fused in the second stage. During the dialysis with pH 7.6 buffer solution in the second stage, the particles began to fuse when the pH value of the system increased to near the pKa of the lipid nanoparticles, and the particles fused more completely at the low ethanol content. When the pH value of the system was higher than the pKa of the lipid nanoparticles, the particle fusion was completed, forming thermodynamically stable final product lipid nanoparticles.

Some specific embodiments of Example 2 were shown in Table 3.

Table 3 Specific embodiments of Example 2

| No. | Concentration of citric acid | Concentration of Tris | Replacement process of the buffer system | | |
|---|---|---|---|---|---|
| | | | Step 1 of the process | Step 2 of the process | Summary of the embodiment |
| Embo dimen t 2 | 10cmM | 20cmM | Diluted 10 times by pH 4.0 buffer solution, then conentrated to the original volume | Dialyzed 5 times with equal volume by TFF using pH7.6 buffer solution | Diluted with acidic buffer solution firstly, then dialyzed with neutral buffer solution |

Comparative Example 2 Preparation of lipid nanoparticles by two-step process of dilution followed by dialysis

This Comparative Example illustrates another conventional preparation method of lipid nanoparticles, wherein the precursor lipid nanoparticles were diluted and dialyzed with pH 7.6 buffer solution to obtain final product lipid nanoparticles. The specific preparation method was shown below.

1. Preparation of the precursor lipid nanoparticles in a manner the same as that in Example 1.
2. Preparation of final product lipid nanoparticles

2.1 Dilution with pH 7.6 buffer solution: the suspension of the precursor lipid nanoparticles was diluted 3 times by 20 mM Tris-10.7 mM NaOAc-8.7% sucrose solution (pH 7.6) (during which the ethanol content was reduced while the pH value of the system was increased) to obtain a solution diluted with pH 7.6 buffer solution;
2.2 Dialysis with pH 7.6 buffer solution: the solution diluted with pH 7.6 buffer solution was concentrated 3 times by tangential flow filtration (TFF), then dialyzed 4 to 6 times with equal volume using 20 mM Tris-10.7 Mm NaOAc-8.7% sucrose solution (pH 7.6) as the dialysis solution. During the increase of the pH value, the particles fused to form stable final product lipid nanoparticles.

In this method, after the dilution with pH 7.6 buffer solution in the first stage, the pH value of the system was increased. During the dialysis with pH 7.6 buffer solution in the second stage, the pH value of the system rapidly increased to near the pKa of lipid nanoparticles, the particles began to fuse. Due to the high ethanol content, the particles did not fuse completely, forming thermodynamically unstable final product lipid nanoparticles.

Some specific embodiments of Comparative Example 2 were shown in Table 4.

Table 4 Specific embodiments of Comparative Example 2

| No. | Concentration of citric acid | Concentration of Tris | Replacement process of the buffer system | | |
|---|---|---|---|---|---|
| | | | Step 1 of the process | Step 2 of the process | Summary of the embodiment |
| Comparative embodiment 2 | 10 mM | 20 mM | Diluted 3 times with pH7.6 buffer solution, then concentrated to the original volume | Dialyzed 5 times with equal volume by TFF using pH7.6 buffer solution | Diluted with neutral buffer solution firstly, then dialyzed with neutral buffer solution |

Example 3 Preparation of lipid nanoparticles by one-step dialysis process (improving the buffering capacity of acidic buffer solution)

This Example illustrates a method for the preparation of lipid nanoparticles with low no-load rate according to the present invention, wherein the precursor lipid nanoparticles were dialyzed with a pH 7.6 buffer solution in one step to obtain final product lipid nanoparticles. The specific preparation method was shown below.

1. Preparation of the precursor lipid nanoparticles

1.1 preparation of the lipid phase in a manner the same as that in Example 1;
1.2 Preparation of the aqueous phase: the mRNA stock solution was diluted with 20 mM citric acid-130 mM sodium chloride buffer solution (pH 4.0) to obtain an aqueous phase with an mRNA concentration of 0.18 mg/ml;
1.3 Mixing in a manner the same as that in Example 1.

2. Preparation of final product lipid nanoparticles in a manner the same as that in Comparative Example 1.

In this method, when preparing the precursor lipid nanoparticles, a higher concentration of citric acid buffer system was used, which reduced the increasing rate of pH value of the system during the buffer system replacement process of the one-step dialysis, so that the ethanol content in the system had been reduced to an acceptable level when the particles fused, and the particles fused completely to form thermodynamically stable final product lipid nanoparticles. The one-step dialysis process of this Example comprises two stages. In the first stage, the pH value of the system was lower than the pKa of the lipid nanoparticles, and the ethanol content was reduced to a certain level to ensure that the ethanol content in the system was low when the particles fused in the second stage. In the second stage, when the pH value of the system increased to near the pKa of the lipid nanoparticles, the particles began to fuse, and the particles fused more completely at the low ethanol content. When the pH value of the system was higher than the pKa of the lipid nanoparticles, the particle fusion was completed, forming thermodynamically stable final product lipid nanoparticles.

Some specific embodiments of Example 3 were shown in Table 5.

Table 5 Specific embodiments of Example 3

| No. | Concentration of citric acid | Concentration of Tris | Replacement process of the buffer system | | |
| --- | --- | --- | --- | --- | --- |
| | | | Step 1 of the process | Step 2 of the process | Summary of the embodiment |
| Embodiment 3 | 20 mM | 20 mM | Dialyzed 8 times with equal volume by TFF using pH7.6 buffer solution | N/A | One-step dialysis, improving the bufering capacity of the buffer solution |

Example 4 Preparation of precursor lipid nanoparticles by post-loading process

This Example illustrates an alternative method for preparing the precursor lipid nanoparticles of the present invention. Unlike the standard preparation process of directly mixing the lipid phase with the aqueous phase containing nucleic acids as described in Example 1, in the post-loading process, the lipid phase was firstly mixed with an acidic buffer solution without nucleic acids to form empty lipid nanoparticles, which were then mixed with the aqueous phase containing nucleic acids, allowing the lipids to encapsulate the nucleic acids to form the precursor lipid nanoparticles. The specific preparation method was shown below.

1.1 Preparation of the lipid phase: four lipids of SM-102, mPEG-DMG-2K, DSPC and cholesterol were weighed accurately at a molar ratio of 50%: 1.5%: 10%: 38.5%, and dissolved in anhydrous ethanol to obtain a lipid phase with a total lipid concentration of 12 mg/ml;
1.2 Preparation of the empty lipid nanoparticles: the lipid phase and a 10 mM citric acid-130 mM sodium chloride buffer solution (pH 4.0) were conveyed by a constant-flux pump and mixed at a volume ratio of 1:3 in a microchannel mixer to form a empty lipid nanoparticle suspension;
1.3 Preparation of the aqueous phase: the mRNA stock solution obtained in Prepapration Example 1 was diluted with a 10 mM citric acid-130 mM sodium chloride buffer solution (pH 4.0) to obtain an aqueous phase with mRNA concentration of 0.18 mg/ml;

1.4 Preparation of the precursor lipid nanoparticles: the empty lipid nanoparticle suspension and the aqueous phase were conveyed by a constant-flux pump and mixed at a volume ratio of 4:3 in a microchannel mixer, during which the lipids encapsulated the mRNA to form a suspension of the precursor lipid nanoparticles.

Example 5 Study on the relationship between particle fusion pH value and pKa of the lipid nanoparticles

The fusion process of lipid nanoparticles is closely related to the charge state of LNPs containing ionizable cationic lipids. The charge ratio of the ionizable cationic lipids at different pH values conforms to the Henderson-Hasselbalch equation:

$$\lg \frac{[BH^+]}{[B]} = pKa - pH$$

wherein $[BH^+]$ is the concentration of positively charged ionizable cationic lipids, and $[B]$ is the concentration of neutral ionizable cationic lipids. In this Example, the ionizable cationic lipid SM-102 was taken as an example, and the relationship between the pH value at which the particles begin to fuse or the pH value at which the fusion completes during the replacement process of the buffer system and the pKa of the LNP containing the ionizable cationic lipid was studied by investigating the change in particle size of the particles. In addition, the changes in particle size of LNPs containing the cationic lipid DOTAP, the ionizable cationic lipid SM-102 and Dlin-MC3-DMA respectively during the replacement process of the buffer system were compared to reveal whether different types of cationic lipids would affect the particle fusion process.

1. Determination of pKa of LNPs containing ionizable cationic lipids

The pKa of LNPs containing ionizable cationic lipids was determined using the commonly used sodium 6-(p-toluidino)-2-naphthalenesulfonate (TNS) fluorescent probe. TNS was non-fluorescent in aqueous solution, however, after binding to cationic lipids and entering into a hydrophobic environment, it exhibited strong fluorescence. With the decrease of pH value, the interaction between the negatively charged TNS and the positively charged ionizable cationic lipids increased, resulting in enhanced fluorescence signals. The fluorescence signal intensity-pH value curve is fitted using the Boltzmann function. The fitting results were differentiated, and the pH value at the peak was the pKa of the LNP containing ionizable cationic lipids. The specific determination method was shown below.

1.1 Preparation of SM-102 lipid nanoparticles in a manner the same as that in Example 1.
1.2 Determination of the pKa of SM-102 lipid nanoparticles

1 ml of buffer solutions with different pH gradients (citrates, phosphates or borates buffer solution) were added to centrifuge tubes, and 50 µl of SM-102 lipid nanoparticle dispersions were added to obtain sample solutions with different pH gradients. 200 µl of sample solutions with different pH gradients were added into a 96-well plate. 30 µl (300 µM) of prepared TNS-DMSO Reagent was added to each well, and the plate was kept from light for 5 min. The 96-well plate was placed into a microplate reader, which was set in the fluorescence mode with an excitation wavelength of 321 nm and an emission wavelength of 445 nm. The Boltzmann function was used to fit the fluorescence signal intensity-pH value curve. The fitting results were differentiated, and the pH value at the peak (center of the peak) was determined as 6.52, which was the pKa value of SM-102 lipid nanoparticles. The fitting curve was shown in Figure 2.

2. Relationship between the pH at which particles fuse and pKa of ionizable cationic lipids

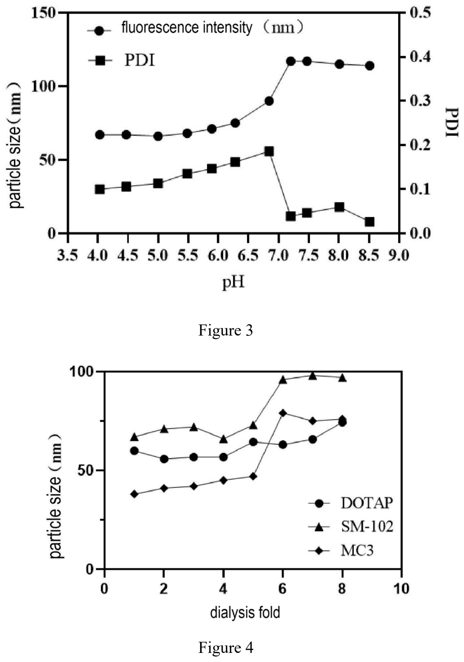

The precursor lipid nanoparticles were prepared according to the method described in Example 1, and then treated by a two-step dialysis process to obtain final product lipid nanoparticles. During the increase of the pH value, the particle size was used as an indicator to reflect the fusion process of ionizable cationic lipid particles and the relationship between the pH value at which fusion occurs and the pKa of the ionizable cationic lipid LNP.
The two-step dialysis process was as follows. The SM-102 precursor lipid nanoparticles were firstly dialyzed 3 times with equal volume using 10mM citric acid-130mM sodium chloride buffer solution (pH 4.0), and then dialyzed with 20mM Tris-10.7mM NaOAc-8.7% sucrose solution (pH 7.6). During the increase of pH value, samples were taken at pH 4.0, 4.5, 5.0, 5.5, 6.0, 6.2, 6.8, 7.2, and 7.4 respectively for determination of the particle size and PDI. The pH value of the dialyzed SM-102 final product lipid nanoparticles was adjusted to 8.0 and 8.5 with sodium hydroxide solution, and samples were taken for determination of particle size and PDI. The results were shown in Table 6 and Figure 3.

Table 6 Change of particle size and PDI of SM-102 lipid nanoparticles with the change of pH value

| pH value | 4.0 | 4.5 | 5.0 | 5.5 | 6.0 | 6.2 | 6.8 | 7.2 | 7.4 | 8.0 | 8.5 |
|---|---|---|---|---|---|---|---|---|---|---|---|
| Particle size (nm) | 67 | 67 | 66 | 68 | 71 | 75 | 90 | 117 | 117 | 115 | 114 |
| PDI | 0.10 | 0.11 | 0.11 | 0.14 | 0.15 | 0.16 | 0.19 | 0.04 | 0.05 | 0.06 | 0.03 |

During the dialysis, when the pH value increased to 6.0, the particle size did not change significantly, indicating that the particles had not yet begun to fuse; when the pH value increased to near the pKa of SM-102 (6.2 and 6.8), the particle size and PDI increased significantly, indicating that the particles were fusing; and when the pH value increased to 7.2 and above, the PDI decreased and the particle size and PDI remained at a stable level, indicating that the fusion of the particles had finished. Therefore, in the two-stage replacement process, the first stage process aims to reduce the concentration of organic solvent in the system, and the pH should be maintained at ≤pKa-0.5, so that the particles do not undergo obvious fusion; the second stage process aims to allow the particles to fuse to form final product lipid nanoparticles, and the pH at the endpoint of the process should be ≥pKa+0.5, so that the fusion of the particles has finished and a steady state has been reached.

3. Particle changes of different cationic lipid LNPs during buffer solution replacement

The precursor lipid nanoparticles were prepared using ionizable cationic lipids SM-102, Dlin-MC3-DMA and permanently charged cationic lipid DOTAP according to the method described in Example 1, and then treated with a two-step dialysis process to obtain final product lipid nanoparticles. During the increase of the pH value, the particle fusion process was studied by using particle size as an indicator.

The precursor lipid nanoparticles were prepared using cationic lipid DOTAP, ionizable cationic lipid SM-102 or Dlin-MC3-DMA respectively according to the method described in Example 1, firstly dialyzed 3 times with equal volume using 10mM citric acid-130mM sodium chloride buffer solution (pH 4.0) to remove ethanol, and then dialyzed with 20mM Tris-10.7mM NaOAc-8.7% sucrose solution (pH 7.6). During the increase of the pH value, samples were taken to determine the ethanol content, particle size and PDI. The results were shown in Table 7 and Figures 4-5.

Table 7 Changes in particle size and PDI of different cationic lipid LNPs during buffer solution replacement

| Replacement process of buffer system | | DOTAP | | | SM-102 | | | Dlin-MC3-DMA | | | Ethanol content (%) |
|---|---|---|---|---|---|---|---|---|---|---|---|
| | | pH | Particle size (nm) | PD1 | pH | Particle size (nm) | PDI | pH | Particle size (nm) | PDI | |
| pH4.0 buffer solution | Dialyzed 1 time | 4.20 | 60 | 0.17 | 4.29 | 67 | 0.07 | 4.26 | 38 | 0.07 | 8 |
| | Dialyzed 2 times | 4.10 | 56 | 0.23 | 4.12 | 71 | 0.10 | 4.08 | 41 | 0.09 | 3 |
| | Dialyzed 3 times | 4.07 | 57 | 0.28 | 4.06 | 72 | 0.10 | 4.03 | 42 | 0.09 | 1 |
| pH7.6 buffer solution | Dialyzed 4 times | 5.37 | 57 | 0.29 | 5.31 | 66 | 0.12 | 5.26 | 45 | 0.12 | 0.4 |
| | Dialyzed 5 times | 6.60 | 64 | 0.31 | 6.36 | 73 | 0.15 | 6.40 | 47 | 0.20 | 0.1 |
| | Dialyzed 6 times | 7.48 | 63 | 0.26 | 7.29 | 96 | 0.07 | 7.30 | 79 | 0.07 | 0.05 |
| | Dialyzed 7 times | 7.62 | 66 | 0.32 | 7.49 | 98 | 0.05 | 7.44 | 75 | 0.06 | 0.02 |
| | Dialyzed 8 times | 7.64 | 74 | 0.36 | 7.54 | 97 | 0.06 | 7.46 | 76 | 0.07 | 0.01 |
| pKa (TNS method) | | N/A | | | 6.52 | | | 6.57 | | | N/A |

From the changes in particle size and PDI during the replacement process of buffer system in the above table, it can be seen that: (1) Lipid nanoparticles containing ionizable cationic lipids SM-102 and Dlin-MC3-DMA began to fuse at pH 6.36 and 6.40 respectively, with the increase of the particle size and PDI; then fusion was completed at pH 7.29 and 7.30 respectively, with the particle size increased significantly and the PDI decreased significantly; after the particle fusion was completed, the particle size and PDI were remained at a stable level; (2) Lipid nanoparticles containing cationic lipid DOTAP were always charged within the pH range of the process, the particle size and PDI continued to increase slowly, and there was no particle fusion process caused by changes in charge during the replacement process of buffer system. These results showed that there was no particle fusion process caused by changes in charge during replacement process of the buffer system for permanently charged cationic lipid LNPs, while particle fusion process existed generally for ionizable cationic lipid LNPs, and the fusion occured when the pH value was near to pKa.

Example 6 Summary of physical and chemical properties of lipid nanoparticles in different Examples and Comparative Examples

1.Determination method for encapsulation efficiency: Ribogreen® fluorescence kit was used to determine the encapsulation efficiency of nucleic acid in the final product lipid nanoparticles. The sample was diluted with TE buffer solution and then added to a black polystyrene 96-well plate, into which TE buffer solution or the same volume of Triton X-100 was added. After incubation, TE-diluted Ribogreen® Fluorescence reagent was added, and the fluorescence intensity was measured at an excitation wavelength of 480nm and an emission wavelength of 520nm. The ratio of the fluorescence signal of the TE diluted sample to that of the Trion X-100 destroyed sample was the percentage of free mRNA.

2. Determination method for particle size: Malvern Zetasizer Nano ZS laser nanoparticle size analyzer was used to determine the particle size and polydispersity index (PDI) of lipid nanoparticles.

3. Comparison of the physical and chemical properties of particles: During the replacement process of the buffer system, the content of the organic solvent during fusion influenced the degree of particle fusion, which in turn had an effect on the key physical and chemical indexes (such as particle size, PDI and encapsulation efficiency) and storage stability of the final product lipid nanoparticles. The key physical and chemical properties of the particles and organic solvent residues before and after fusion in the replacement process of buffer system in different Examples and Comparative Examples were compared statistically. The comparison results of the key physical and chemical properties of the particles before and after fusion were shown in Table 8.

Table 8 Comparison of key physical and chemical properties of particles before and after fusion in different Examples and Comparative Examples

| No. | The stage of the replacement process | pH | Ethanol content (%) | Particle size (nm) | PDI | Encapsulation efficiency (%) |
|---|---|---|---|---|---|---|
| Embodiment 1-1 | at the end of the first stage | 6.03 | 0.1 | 78 | 0.142 | 94 |
| | at the end of the second stage | 7.20 | 0.04 | 107 | 0.073 | 93 |
| Embodiment 1-2 | at the end of the first stage | 5.41 | 2 | 55 | 0.070 | 90 |
| | at the end of the second stage | 7.26 | 0.4 | 99 | 0.069 | 90 |
| Embodiment 1-3 | at the end of the first stage | 6.01 | 0.4 | 61 | 0.111 | 91 |
| | at the end of the second stage | 7.22 | 0.1 | 96 | 0.074 | 90 |
| Embodiment 2 | at the end of the first stage | 5.46 | 0.8 | 72 | 0.239 | 91 |
| | at the end of the second stage | 7.53 | 0.3 | 96 | 0.046 | 93 |
| Embodiment 3 | at the end of the first stage | 5.99 | 3 | 69 | 0.123 | 95 |
| | at the end of the second stage | 7.34 | 0.4 | 99 | 0.050 | 95 |
| Comparative Embodiment 1-1 | at the end of the first stage | 5.89 | 8 | 66 | 0.057 | 95 |
| | at the end of the second stage | 7.22 | 1 | 87 | 0.046 | 96 |

(continued)

| No. | The stage of the replacement process | pH | Ethanol content (%) | Particle size (nm) | PDI | Encapsulation efficiency (%) |
|---|---|---|---|---|---|---|
| Comparative Embodiment 1-2 | at the end of the first stage | 6.01 | 13 | 57 | 0.061 | 90 |
| | at the end the second stage | 7.40 | 3 | 82 | 0.057 | 84 |
| Comparative Embodiment 2 | at the end of the first stage | 5.97 | 7 | 66 | 0.279 | 94 |
| | at the end the second stage | 7.29 | 1 | 88 | 0.180 | 94 |

As can be seen from the above table, compared with the method of the comparative embodiment, the preparation method of the present invention reduced the ethanol content to a lower level (<5%) before particle fusion, making the particle fusion more complete, as shown by the larger particle size after fusion.

Example 7 Effect of ethanol content on particle fusion

The precursor lipid nanoparticles were prepared according to the method described in Example 1. 0.5 ML of suspensions of the precursor lipid nanoparticles were respectively injected into 5 ml G50 Sephadex gel columns pre-equilibrated with 20 mM Tris-10.7 mM NaOAc-8.7% sucrose solutions (pH 7.6) with different ethanol contents, which were then eluted with the corresponding equilibration buffer solutions. Samples were collected when the eluate became milky white, and used for determination of the pH value and particle size of the final product lipid nanoparticles prepared by dialysis on Sephadex gel column, so as to investigate the effect of ethanol content on particle fusion during dialysis on the Sephadex gel column. The results were shown in Table 9.

Table 9 Effect of ethanol content on particle fusion

| Sample | | Particle size (nm) | PDI | pH |
|---|---|---|---|---|
| Before dialysis | | 50 | 0.076 | 4.82 |
| After dialysis | 0% ethanol | 105 | 0.129 | 7.57 |
| | 1% ethanol | 119 | 0.184 | 7.63 |
| | 2.5% ethanol | 108 | 0.069 | 7.54 |
| | 5% ethanol | 110 | 0.082 | 7.57 |
| | 10% ethanol | 88 | 0.063 | 7.54 |
| | 15% ethanol | 90 | 0.052 | 7.52 |

When the ethanol content was $\leq$ 5%, the particles fused, and the increase of the particle size was similar. After dialysis, the particle size was above 105nm, indicating a complete particle fusion. When the ethanol content was $\geq$10%, the increase of the particle size was smaller, the particle size after dialysis was below 90nm, indicating an incompleter particle fusion.

Example 8 Reverse Dialysis

The conclusion that there were stable particles and metastable particles in the fusion process was further verified by reverse dialysis. The specific method of reverse dialysis was as follows. The precursor lipid nanoparticles were firstly prepared according to the method described in Example 1. The suspension of the precursor lipid nanoparticles was dialyzed 5 times with equal volume by tangential flow filtration using 20mM Tris-10.7mM NaOAc-8.7% sucrose solution (pH 7.6), then reversely dialyzed 3 times with equal volume using 10mM citric acid-130mM sodium chloride buffer solution (pH 4.0), and finally dialyzed 3 times with equal volume using 20mM Tris-10.7mM NaOAc-8.7% sucrose solution (pH 7.6).

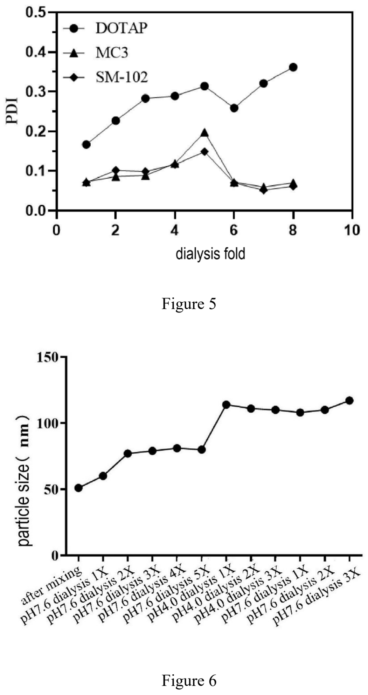

In the process of preparing the final product lipid nanoparticles by reverse dialysis, the system underwent a change from acidic → neutral → acidic → neutral. The first process from pH 4.0 to pH 7.6 was consistent with the one-step dialysis process for preparing the final product lipid nanoparticles in Comparative Example 1. The first stage ended with 1 time of equal volume dialysis, the pH value of the system was below the pKa of the lipid nanoparticles containing ionizable cationic lipids, and the ethanol content was 8%, which was higher than the acceptable standard. The second stage ended with more than 2 times of equal volume dialysis, the pH value of the system increased to above the pKa of the lipid nanoparticles containing ionizable cationic lipids. During the increase of the pH value, the particles fused to form final product lipid nanoparticles. Due to the high ethanol content in the system, the particle fusion was incomplete and the steady state was not reached. The particles obtained after 5 times of dialysis to remove ethanol have a particle size of 80 nm. After reverse dialysis from pH 7.6 to pH 4.0, no-load LNPs and unstable LNPs fused again to reach a steady state, resulting in particles having a particle size of 110nm. The second process from pH 4.0 to pH 7.6 resulted in particles having a particle size of 117nm, which was basically consistent with that after reverse dialysis. The changes in particle size and PDI during the process were shown in Table 10 and Figure 6.

Table 10 Changes in particle size and PDI during reverse dialysis

| Process | Mixing | pH7.6 buffer solution | | | | | | pH4.0 buffer solution | | | pH7.6 buffer solution | | |
|---|---|---|---|---|---|---|---|---|---|---|---|---|---|
| | | Dialyzed 1 time | Dialyzed 2 times | Dialyzed 3 times | Dialyzed 4 times | Dialyzed 5 times | | Dialyzed 1 time | Dialyzed 2 times | Dialyzed 3 times | Dialyzed 1 time | Dialyzed 2 times | Dialyzed 3 times |
| Particle size (nm) | 51 | 60 | 77 | 79 | 81 | 80 | | 114 | 111 | 110 | 108 | 110 | 117 |
| PDI | 0.07 | 0.10 | 0.10 | 0.08 | 0.07 | 0.09 | | 0.15 | 0.14 | 0.14 | 0.14 | 0.14 | 0.10 |
| pH | 4.74 | 5.92 | 7.13 | 7.43 | 7.47 | 7.53 | | 4.76 | 4.24 | 4.06 | 5.17 | 6.73 | 7.35 |
| Ethanol content (%) | 21 | 8 | 3 | 1 | 0.4 | 0.1 | | < 0.1 | | | | | |

The results showed that, when the particles fused, the high ethanol content of the system (>5%) led to incomplete particle fusion. The obtained metastable particles further fused to obtain stable particles at a low ethanol content (<0.1%), indicating that the physical and chemical properties (particle size, etc.) of metastable particles might undergo significant changes during storage.

Example 9 Determination of nucleic acid loading percentage using fluorescence-based nano-flow cytometry

Fluorescence-based nano-flow cytometry was used to determine the percentages of loaded LNP and no-load LNP, so as to compare the particle fusion during the process and the percentages of loaded LNP and no-load LNP in products prepared by different processes.

The nucleic acid loading percentage is determined by fluorescence-based nano-flow cytometry based on the following basic principle. The sample was added with a nucleic acid dye capable of passing through the membrane, which penetrated the LNP membrane and combined with the internal mRNA to produce fluorescence, and the percentages of loaded LNP and no-load LNP in the sample were calculated by the fluorescence signal and the scattering signal. The specific test method comrpises adding ribozyme into the sample to be tested, incubating to remove free mRNA, adding nucleic acid dye and incubating, measuring the scattered light signal of a single LNP with a nano-flow cytometry analyzer, and rapidly distinguishing the empty-loaded LNP and nucleic acid-loaded LNP through dye fluorescence labeling to thereby calculate the percentages of loaded LNP and no-load LNP.

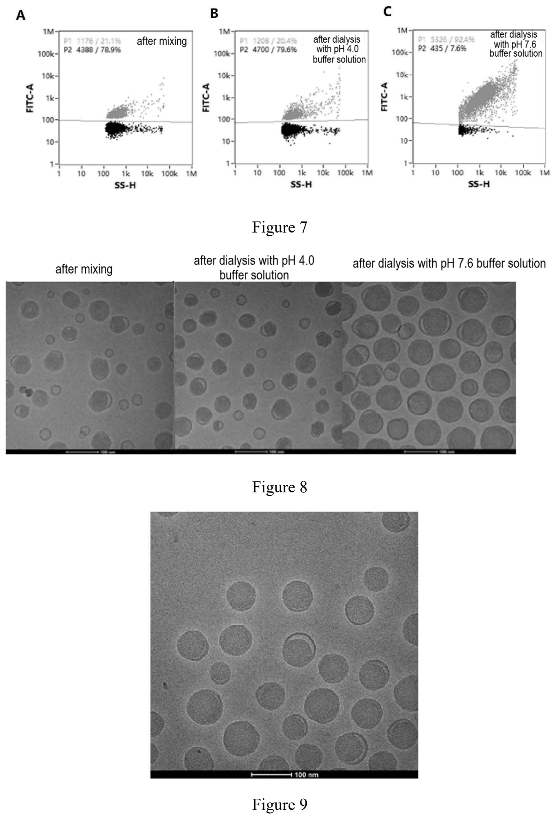

The changes in the percentages of loaded LNP and no-load LNP in the intermediates during the process of the preparation of LNP in embodiment 1-1 were tested by fluorescence-based nano-flow cytometry, and the results were shown in Table 11 and Figure 7. After mixing of the lipid phase and the aqueous phase and dialysis with pH 4.0 buffer solution, the percentage of loaded LNP was about 20%, while after dialysis with pH 7.6 buffer solution, the percentage of particles loaded with mRNA reached above 90%, as the particle fusion had reached a steady state and there was basically no empty LNP in the system.

Table 11 Changes in the percentages of loaded LNP and no-load LNP during the LNP preparation process embodiment 1-1)

| Samples | Overall particle size (nm) | Particle size of loaded LNP (nm) | Percentage of loaded LNP (%) | Percentage of no-load LNP (%) |
|---|---|---|---|---|
| After mixing | 65 | 71 | 21.1 | 78.9 |
| After dialysis with pH4.0 buffer solution | 67 | 75 | 20.4 | 79.6 |
| After dialysis with pH7.6 buffer solution | 94 | 92 | 92.4 | 7.6 |

The percentages of loaded LNP and no-load LNP in the final product lipid nanoparticles obtained in different Examples and Comparative Examples were tested by fluorescence-based nano-flow cytometry, and the results were shown in Table 12. All the Examples showed a low percentage of no-load LNP of below 10%, indicating that the particles fused completely and reached a steady state.

Table 12 Percentages of loaded LNP and no-load LNP in the final product products in different Examples and Comparative Examples

| No. | Embodiment | | | | | Comparative embodiment | | |
|---|---|---|---|---|---|---|---|---|
| | 1-1 | 1-2 | 1-3 | 2 | 3 | 1-1 | 1-2 | 2 |
| Percentage of loaded LNP (%) | 92.4 | 92.9 | 92.1 | 92.9 | 91.7 | 85.4 | 80.0 | 80.6 |
| Percentage of no-load LNP (%) | 7.6 | 7.1 | 7.9 | 7.1 | 8.3 | 14.6 | 20.0 | 19.4 |

Example 10 Characterization by cryo-transmission electron microscopy (Cryo-TEM)

The morphology of lipid nanoparticles was characterized by cryo-transmission electron microscopy (Cryo-TEM), and the changes in particle morphology during the process and the morphology of products prepared by different processes were compared.

A cryo-TEM image of an intermediate during the LNP preparation process of embodiment 1-1 was shown in FIG 8. After mixing of the lipid phase and the aqueous phase and dialysis with pH 4.0 buffer solution, lipid nanoparticles with small particle sizes were present in the system; while after dialysis with pH 7.6 buffer solution, the particles fused to form large and uniform lipid nanoparticles. The cryo-TEM results showed that a particle fusion process occurred during the preparation of LNP. This result was consistent with the results for particle size and fluorescence nanoflow cytometry.

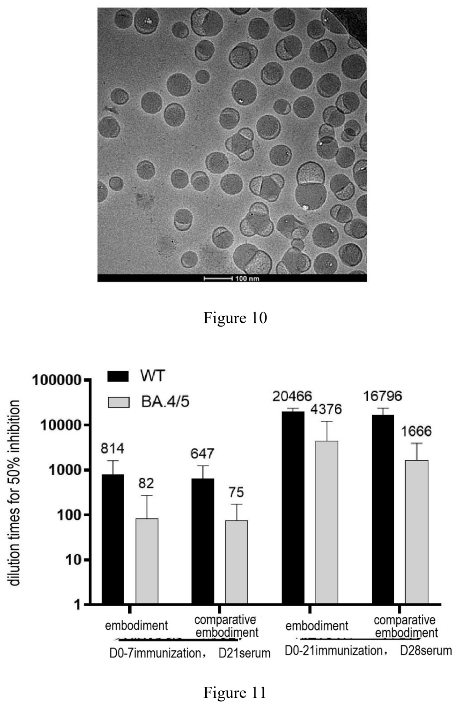

The cryo-TEM image of the final product lipid nanoparticles obtained in embodiment 1-1 was given in FIG 9, which showed that the particles had uniform size and consistent morphology. The cryo-TEM image of the final product lipid nanoparticles obtained in comparative embodiment 1-1 was given in FIG10, which showed that the particles had different morphologies and there were many preliminarily fused LNPs or no-load LNPs, which particles were thermodynamically unstable and the physical and chemical properties thereof might change during storage.

Example 11 Preparation of lipid nanoparticles encapsulating different types of nucleic acids

Lipid nanoparticles can be used to deliver various genetic materials, including siRNA, pDNA and mRNA, etc. Lipid nanoparticles encapsulating siRNA (ordinary siRNA, GenePharm) or pDNA (pVAX.1, GenScript Biotech Co., Ltd.) were prepared according to the method of embodiment 1-1 of the present invention. The physical and chemical properties and loading percentage of the nucleic acids in the obtained products were shown in Table 13. As shown in the results in the table, the product possessed uniform particle size, high encapsulation efficiency and low no-load rate.

Table 13 Physical and chemical properties and loading percentage of nucleic acid of LNP products encapsulating different nucleic acids

| Nucleic acid | Particle size (nm) | PDI | Encapsulation efficiency (%) | Loading percentage of LNP (%) | No-load percentage of LNP (%) |
|---|---|---|---|---|---|
| siRNA | 106 | 0.10 | 94 | 92.3 | 7.7 |
| pDNA | 97 | 0.10 | 92 | 94.0 | 6.0 |

The above siRNA (patisiran sodium , MedChemExpress) has the following sequences: RNA (SEQ ID NO: 28, A-U-G-G-A-A-Um-A-C-U-C-U-U-G-G-U-Um-A-C-dT-dT), binding RNA (SEQ ID NO: 29, G-Um-A-ACm-Cm-A-A-G-A-G-Um-A-Um-Um-Cm-Cm-A-Um-dT-dT) (1:1) sodium salts, wherein m reprsents 2'-Ome modification, dT represents thymidine deoxyribose.

The above pDNA ( pVAX.1 ) is Invitrogen™, catalogue number: V26020.

Other products were prepared according to the method of embodiment 1-1 of the present invention, except that the mRNA stock solution obtained in Preparation Example 1 was replaced with mRNAs shown in other sequences of the present invention (SEQ ID NOs: 2-7, or mRNA shown in SEQ ID NOs: 8-26). The products obtained exhibited desirable properties, including uniform particle size, high encapsulation efficiency and low no-load percentage.

Example 12 Study on storage stability of intermediates in the preparation process of lipid nanoparticles

The intermediates in the preparation process of embodiment 1-1 were placed at 2-8°C for 2 months, and then the particle size thereof was measured. The results showed that, the particles that fused completely (after dialyzed with pH 7.6 buffer solution) had better stability, with no significant changes in average particle size and PDI, whereas the particles that did not fuse completely (after mixing and dialyzed with pH 4.0 buffer solution) had a significantly increased particle size and a larger PDI. The results were shown in Table 14.

Table 14 Storage stability of intermediates in the preparation process of LNP

| Sample | Particle size (nm) | | PDI | | D10 (nm) | | D50 (nm) | | D90 (nm) | |
|---|---|---|---|---|---|---|---|---|---|---|
| | 0 Day | 2 Months | 0 Day | 2 Months | 0 Day | 2 Months | 0 Day | 2 Month | 0 Day | 2 Months |
| After mixing | 62 | 91 | 0.021 | 0.082 | 41 | 64 | 55 | 86 | 78 | 119 |
| After dialysis with pH4.0 buffer solution | 74 | 86 | 0.085 | 0.140 | 43 | 44 | 61 | 65 | 95 | 112 |
| After dialysis with pH7.6 buffer solution | 102 | 105 | 0.019 | 0.013 | 76 | 80 | 104 | 106 | 142 | 141 |

Example 13 Study on freeze-thaw stability of mRNA-LNP

There are differences in the stability of lipid nanoparticles prepared by different preparation processes during storage, transportation and use. For frozen mRNA-LNP, the changes in the physical and chemical properties of the final product lipid nanoparticles prepared by the preparation processes of embodiment 1-1 and comparative embodiment 1-1 after repeated freezing and thawing (-20°C ± 5°C/room temperature) were investigated. The results were shown in Table 15.

Table 15. Stability of mRNA-LNP after repreated freezing and thawing

| Times of repreated freezing and thawing | Comparative embodiment 1-1 | | Embodiment 1-1 | |
|---|---|---|---|---|
| | particle size(nm) | PDI | particle size(nm) | PDI |
| Before freeze-thaw | 85 | 0.04 | 105 | 0.04 |
| 5 | 115 | 0.09 | 111 | 0.04 |
| 10 | 121 | 0.09 | 121 | 0.03 |
| 20 | 132 | 0.14 | 123 | 0.07 |
| 30 | 148 | 0.18 | 124 | 0.09 |

After 30 cycles of freeze-thaw, the increases in particle size and PDI of the mRNA-LNP prepared by embodiment 1-1 were significantly less than those of comparative embodiment 1-1, indicating that the lipid nanoparticles prepared by embodimentl-1 had better freeze-thaw stability.

Example 14 Study on stability of mRNA-LNP under freezing, refrigeration and room temperature conditions

The lipid nanoparticle composition (mRNA-LNP) prepared in embodiment 1-1 was stored under freezing (-20°C ± 5°C), refrigeration (5°C ± 3°C) and room temperature (25°C ± 2°C) conditions, and samples were taken at different time points for analysis of the particle size, PDI, mRNA purity and biological activity. The stability results were shown in Table 16.

Method for detecting mRNA purity: mRNA was extracted and purified by use of PureLink RNA Mini Kit (Invitrogen), concentrated to a concentration of 200 ng/$\mu$l using a 30KD ultrafiltration centrifuge tube (Merck), and analyzed and identified with Nanodrop UV spectrophotometer (Thermo). The purity of mRNA was evaluated with an Agilent 2100 bioanalyzer.

Method for detecting biological activity: healthy, well-grown BALB/c mice aged 6-8 weeks were selected, and randomly divided into groups after classified by weight, with 10 mice in each group, half male and half female. Each animal was given a dose of 5 $\mu$g for each administration, with two doses administered at an interval of 7 days. Blood serum was collected 7 days after the last dose, and the titer of the anti-S protein binding antibody in the serum sample of individual mice was determined by ELISA, and then the geometric mean of the antibody titer of all tested mice was calculated.

Table 16 Storage stability of mRNA-LNP under freezing, refrigeration and room temperature conditions

| Items | 0 Day | -20°C±5°C | | | | 5°C±3°C | | 25°C±2°C | |
|---|---|---|---|---|---|---|---|---|---|
| | | 3 Months | 6 Months | 9 Months | 12 Months | 3 Months | 6 Months | 7 Days | 14 Days |
| Particle size (nm) | 105 | 109 | 110 | 117 | 128 | 108 | 113 | 109 | 107 |
| PDI | 0.06 | 0.05 | 0.04 | 0.03 | 0.04 | 0.04 | 0.11 | 0.01 | 0.01 |
| mRN A purity (%) | 91 | 88 | 93 | 88 | 91 | 85 | 80 | 87 | 78 |
| Biological activity (titer) | $1:10^{6.0}$ | $1:10^{6.1}$ | $1:10^{5.6}$ | $1:10^{5.9}$ | $1:10^{5.8}$ | $1:10^{5.6}$ | $1:10^{5.8}$ | $1:10^{5.5}$ | $1:10^{58}$ |

The results showed that the physical and chemical properties and biological activities of the lipid nanoparticle composition (mRNA-LNP) did not change significantly after being stored in a refrigerator (5°C±3°C) for 6 months, at room temperature (25°C±2°C) for 14 days, and under frozen (-20°C±5°C) for 12 months. Compared with similar products, the mRNA-LNP of the present invention has better thermal stability.

Example 15 Antibody efficacy detection for pseudovirus-neutralizing antibodies

BALB/c mice (6-8 weeks old, Beijing Vital River Laboratory Animal Technology Co., Ltd.) were immunized by intramuscular injection of the lipid nanoparticles prepared by embodiment 1-1 and comparative embodiment 1-1 (5 mice/group, female) according to two immunization procedures, respectively. The two immunization procedures were as follows: (1) administration of 2 doses with an interval of 7 days, collection of serum 14 days after the last administration, with a dosage of 10 $\mu$g; (2) administration of 2 doses with an interval of 21 days, collection of serum 7 days after the last administration, with a dosage of 3 $\mu$g. After immunization, mouse serum was collected and initially diluted 30 times and then continuously diluted 3 times with DMEM complete medium (Hyclone, containing 1% double antibody, 10% FBS, 1% non-essential amino acids, 1% HEPES). The mixture was incubated respectively with 650TCID50 of SARS-CoV-2WT and BA.4/5 pseudovirus strains (Zhongke Guobang (Beijing) Inspection and Testing Co., Ltd.) at 37°C for 1 hour, followed by the addition of 2x $10^4$ Vero cells. After culturing for 20-28 hours, the supernatant was discarded and luciferase detection reagent (Perkin Elmer) was added. Fluorescence values were read, and the 50% neutralizing antibody titer was calculated according to the Reed-Muench method. The results were shown in Figure 11. The results showed that for both immunization procedures, the neutralizing activity of the immune serum of embodiment 1-1 against WT and BA.4/5 pseudoviruses was better than that of comparative embodiment 1-1.

Example 16 Detection of in vitro cell transfection efficacy

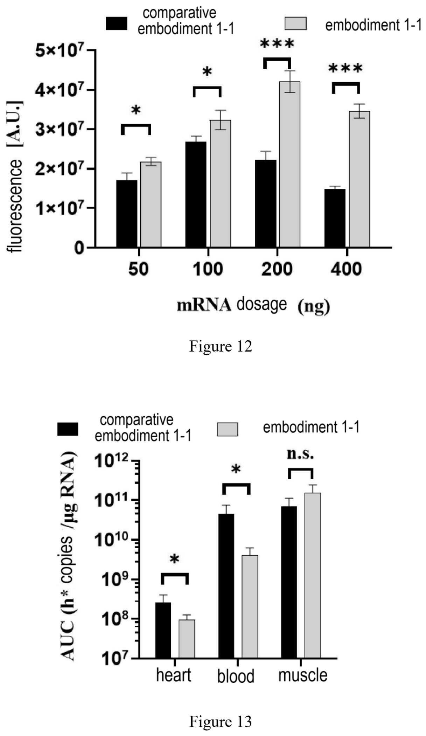

The luciferase mRNA-LNP was prepared according to the method of embodiment 1-1 and comparative embodiment 1-1 of the present invention, except that mRNA of SEQ ID NO: 1 was replaced with a RNA sequence encoding luciferase (SEQ ID NO: 27). The luciferase mRNA-LNP was co-incubated with HEK293T cells (ATCC) in 4 groups, with a dose of 50ng mRNA/well, 100ng mRNA/well, 200ng mRNA/well and 400ng mRNA/well (96-well plate), respectively. After incubation for 24h, luciferase detection reagent (Promega) was added to quantitatively detect the expression of luciferase (n = 3). The data were analyzed by two-tailed unpaired t-test, in which * represents $P < 0.05$, ** represents $P < 0.01$, *** represents $P < 0.001$, n.s. represents no significant difference. The results were shown in Figure 12. The results showed that the in vitro cell transfection efficacy of the luciferase mRNA-LNP prepared by the method of embodiment 1-1 was significantly better than that of the luciferase mRNA-LNP prepared by the method of comparative embodiment 1-1.

Example 17 Experiments on animal tissue distribution

C57BL/6J mice (6-8 weeks old, Beijing Vital River Laboratory Animal Technology Co., Ltd.) were administered with 5 $\mu$g of lipid nanoparticles prepared by embodiment 1-1 and comparative embodiment 1-1 respectively (6 mice/group, half male and half female) by single intramuscular injection. The whole blood of the mice was collected 2h, 24h and 96h after administration (orbital blood collection, EDTA anticoagulation). The heart and local muscle tissue of the mice were taken after perfusion with normal saline. RNA was extracted using the PureLink RNA Mini Kit (Thermo Fisher Scientific), and the RNA concentration was determined by UV spectrophotometry (instrument Nanodrop, Thermo Scientific). RNA was then reverse-transcribed into cDNA using PrimeScript™ RT Master Mix Kit (TaKaRa), Taq Pro HS Universal U+Probe Master Mix (Vazyme) and primers PRD-F6 (SEQ ID NO: 30, AGCGTGCTCTATAACTCGGC), PRD-R6 (SEQ ID NO: 31, TCGGACCTCATCGCCTCTAA), PRD-P6 (SEQ ID NO: 32, ACGGCGTGAGCCCCACAAAG) were added to perform qPCR on a LightCycler 480 real-time fluorescence PCR instrument to quantitatively detect the mRNA levels in the blood and tissues. Data analysis was performed using a two-tailed unpaired t-test, in which * represents $P < 0.05$, ** represents $P < 0.01$, *** represents $P < 0.001$, n.s. represents no significant difference, and the results were shown in Figure 12. The AUC (area under the curve) results of 96 hours after administration showed that the samples prepared using embodiment 1-1 had lower mRNA distribution in heart and blood, which reduced the possibility of myocarditis and systemic adverse reactions.

The relevant sequences involved in this application are as follows:

SEQ ID NO:1

gggagacccaagcuggcuagcguuuaaacuuaagcuugguuaccgagcucgggauccacuaguccagugugguggaauucgaa
uaaacuaguauucuucuggucccacagacucagagagaacccgccaccaugucgguguuccuggugcugcugcccuuagugagcag
ccagugcgucaaccuaacuacuagaacacagcugccuccugccuacacaaacagcuucaccagaggcguguauuaccccgauaagug
uuccggucuucugugcugcacagcaccccggaccuguuucugccuuucuucagcaaugugaccuggguuccacgccauccacgugucu
ggcacaaacggcacaaaaaggguucgacaaccccguucugccuuucaaugacggcguguacuucgcuagcacagaaaagagcaacauca
ucagaggauggaucuucggaaccacccugggauuccaagacacaguccccuguugaucguuauaaugccaccaaugugguggaucaagg
ugugugaguuccaguuuugcaacgauccuuuucgugggcguguauuaccacaagaauaacaaaagcuggaugggaaagcgaguuuaga
guauacucuagcgccaacaacugcaccuucgaguacgucagccagccuuuucugauggaccuggaaggcaagcaggggcaacuucaag
aaucugagagaguucguguucaaaaaacauugacggauauuucaaaaucaacagcaagcacacaccuaucaacuggugcgggaccug
ccacaggggguuuagcgcauuggagccucugcgucgaucugccuaucggcaucaacaucaccccgguuccagacccugccugccugcau
agaagcuaucugaccccuggcgacagcucaagcggcuggaccgccggcgccgccgccuacuacgugggcuaccugcagccuagaaaccu
uucugcugaaguacaacgagaacggcaccauaaccgacgcugugacugcgcccuggacccccugucugaaaccaagugcaccguuaa
gagcuuuaccguggaaaaagggcaucuaccaaacaagcaauuuccggggcagccuaccgagucuaucgucggguuucccaacaucac
caaccugugcccauuccgagaagguguucaacgccaccagauucgccagcgguacgccuggaaccggaagagaaucucuaacugcgu
ggccgauuacagcgugcucuauaacucggcuagcuucagcacauucaagugcuacggcgugagcccccacaaagcugaacgaccugug
cuucaccaacgucuacgccgacagcuucgugauuuagaggcgaugaggguccgacagaucgcccccuggccaaaccggcaagauugcuga
cuacaacuacaagcugccugaugacuucacuggaugugugaucgcuuggaacuccaacaaccuggacucuaaaguggggcggaaacua
caacuuaccgguuaccggcuguuuagaaagaguaaccuuaaaccuuucgagagagauaucagcacagaaaucuaccaggcuggcagcaca
ccuuguuaacggcggcgugcaaggcuucaacugcuauuuccucuccagucuuauuggcuuccuaccuaccaacggcgugggcuaucagccc
uaccgagugguggugcugcuccuucgagcugcugcaugccccuguuaccgugugcgccccuuaagaaaucgaccaaccuggugagaac
aagugcgugaauuuuuaauuucaacggccucaccggcaccggagugcugaccgagagcaacaagaaguuccugcccuuccagcaguuc
ggaagagacaucgccgauaccacagacgccgugcgggacccccaaacacuggaaauccuggacaucacaccuugcagcuucggcggcg
ugagcgugaucaccccuggccaccaacacaagcaaccaggguggccgugcucuaccagggcgugaacugcaccgaagugccaguggccau
ccacgccgaucagcugaccccuaccuggagagugcuacagcaccggcagcaacguguuccagacaagggcugguugcugaucggcgc
cgagcacgugaauaauuccuacgagugcgacaucccuauuggcgccggaaucuguugccagcuaccagacacagacaaacuccccacgu
agagccagauccgucgccagccagagcaucaucgcuuacaccaugagccuggggcgcugaaaacagcguggcuuacagcaacaauucua
ucgccauaccuaccaauuuuacaaucucgugacaaccgagauccugccuguuagcaugaccaagaccagcguggacugcacaaugua
caucugcggagacagcaccgagugcuccaaccugcugcuucaauacgggguccuuucgucuuacacagcugaauagagcccugacaggcau
cgcuguugagcaggacaggacaagaacacccaggaggugguuugcccaagugaagcagaucuacaagacuccgccuaucaaggacuucgccgg
cuucaacuucagccagauccugccagauccuucuuaagccuagcaagcgcaguuucaucgaggaccguuguuuucaacaaggugacccu
ggccgacgccggcuuuaucaagcaguacggcgauugccugggcgacaucgccgcuagagaucugaucugcgcccagaaguucaaugg
acugacagugcugccgccccugcugaccgaugagaugaucgcucaguacaccucugcccugcuggccgggacaaucaccagcggaug
gacauucggggccggcgccgcccugcagauccccuuugccaugcagauggccuacagauucaacgguauuggcgugacccaaaacgu
gcguacgagaaucagaaguuaaucgcaaaccaguucaacagcgccaucggcaagauccaggauucccugaguuccacggccagcgcu
cugggguaagcugcaagacguggugaaccagaacgcacaggcccugaacacccuggucaaacagcugagcuccaacuucggagcgauca
gcagcgugcuuaaugacauccugagcagacuggaucccccccgaggccgaaguccaaaucgaccggcugaucacaggcagguugcaga
gccugcagaccuacgugacccaacagcugaucagagcagccgagaucagagccucagccaaucuggcugcuacgaagaugagcgagug
ugugcuggggacaggacaagcggguggauuuugcggcaaagggguaccaccugaugagcuucucccucagagcgccccucacggccug
uguuccugcacgugacgcugcccgcccaagagaagaacuuccaccacagccccccgucccaucugcacgacgcggaaaagcccacuuccc
ucgugagggcgucuucgugagcaacggcacacacuggguucgugacucagagaaacuucuacgagcccuucagaucaucaccaccgacaac
accuuugugagcggcaacugcgacguggugaucgggaucgugaacaacaccguguacgacccccugcagccugagcuggauagcuuc
aaagaggaacuggacaaguauuucaagaaucacaccucuccagacguggaccuggggcgacaucagcggcauuaacgccucuguggug
aacauccagaaggaaaucgaucggcugaacgaggugguugaagaacuugaacgagagccugaucgaccugcaggagcugggcaaguac
gagcaguacaucaaguggccuuugguacaucuggcugggauucaucgccggccugauugccaucgucauggugaccaucaugcugug
uugcaugacaagcugcugcagcugucugaagggaugguugcucuuguggcucuugcguaaauucgacgaagacgacucggaacccg
ugcugaagggcguuaagcuccacuacaccugaugacucgagcugguacugcaugcacgcaaugcuagcugcccccuuucccguccugg
guaccccgagcucccccgaccucgggucccagguaugcucccaccuccaccugccccacucaccaccucugcuaguuccagacaccu
cccaagcacgcagcaaugcagcucaaaacgcuuagcuagccacaccccccacgggaaacagcagugauuaaccuuuagcaauaaacgaa
aguuuaacuaagcuauacuaaccccagggguuggucaauuucgugccagccacaccucuggagcuagcaaaaaaaaaaaaaaaaaaaaaaa
aaaaaagcauaugacuaaaaaaaaaaaaaaaaaaaaaaaaaaaaaaaaaaaaaaaaaaaaaaaaaaaaaaaaaaaaaaaaaaaaaaaa

SEQ ID NO:2

gggagacccaagcuggcuagcguuuaaacuuaagcuugguuaccgagcucggggauccacuaguccagugugguggaauucgaa
uaaacuaguauucuucuggucccacagacucagagagaacccgccaccaugggguuuguguuccuggucccugcugcccuuagugaagg
ucaguguguugaaccugacaaaccgcacccagcugccccugccuacacuaaacucuuuuaccaggggggaguguacuacccagacaaagug
uuuagaucuucugugcugcacucuacacaggaucuguuucugccauucuucucuaacgugacauggguuccacgccauucacgucagu
ggaacuaacggcacuaagagauucgacaaccccgugcugccuuucaacgacggagugucuacuucgcgagcacgagaaguccaacauca
ucagaggcuggaucuuuggcaccacacuggauagcaagacacagagucugcugauugugaacaacgccacuaaugucugaucaagg
ugugugaguuccaguucugcaacgauccuuuucuggggguguauuaccacaaaaacaacaagaguuggauggagucagaguuuagg
guguauucuuccgcaaacaacugcuacauucgaauacgucucccagccauuucugauggaccuggagggaagcagggcaacuuuaaa
aaucugcgcgaguuugguuuucaagaacaucgaugggguacuuuaagauuuacuccaaacacaccuaucaaccugguugagagaucug
ccacagggauucagcgcccuggagccacugguggaucugccaaucggaaucaauaucacagauuccagacauuacucgcucugcca
gguccuaucugccagguguagcagcagcgguguugacuigcggcuggccagcugcucuacuaugugggauacuugcagcccaagaacau
uccuguugaaauacaacgagaauggaacaaucacagaugcuguggacugcgcagcacccuggauccacugucugagaccaagugcacacuga
agagcuucacugucgagaagggcaucuaucagacaucaacuuuagagugcagcccacagagagcaucgucgauuccccaacaucac
aaaccuguguccuuuuggcgagguguucaacgccacaagauucgcuucugguacgcauggaacaggaagcgaaucucuaacugugu
ggccgauuacagcgugcuguacaacucugcuucuuucuccacauucaaguguuacggagugucaccuacuaaacugaacgaccugug
cuucacuaacgucuaugccgauaguuucgugauccggggagaugaggugaggcagaucgccccaggacagaccgggaaaaucgccga
uuacaacuauaagcugccagacgauuucaccgguugugugaucgccuggaacucaaacaaccuggauagcaaagugggggggcaacua
uaacuacagauaccgacuguucaggaaaaagcaaccugaaaccuuuugagcgcgauaucuccacugagaucuaccaggcaggaucuaca

ccauguaauggagugcagggagauucaacuguuacuuccccuccagucuuuauggcuuucagccuacuaacggagugggauaucagccc
uacagagugguggguccugucccuucgagcugcugcacgcaccgcuaccgugugcggaccuaagaagucaaccaaccuggugaaaaac
aagugguaaacuuuaacuucaacggacucaccggcaccggaguucugacagagcucaacaaaaaguuccugcccuuccagcaguuc
ggcagggauaucgcugacaccacugacgcugugagagaccacagacccuggagauccuggauaucaccccuuguucuuucggcggu
gucagugugaucacaccaggaacuaacaccaguaaccagguggccgugcucuaccagggagugaacuguaccgaggugccuguggcu
auccacgccgaccagcugacccccuaccuggagggguguauagcaccgggaucuaacguguuccagacacgagccggcugccucaucggc
gccgagcacgugaacaacucuuacgagugugacaucccuaucggagcuggaaucugugcuuccuaccagacacagaccaauucuccua
gaagagccagaucugucgcaagccagaguaucaucgcuuacaccaugagccugggagcugagaacagcgugggcuuacagcaacaacag
caucgcaauccuacccaauuucacaaucucagugacuaccgagauccugcccgugagcaugacuaaaaacaucagugugacguacaaug
uauaucugcggagacuccacagagagugucaaaaccugcugcugcaguauggaucuuucuguucuguacucagcugaacagagccugugacagga
aucgcaguggagcaggacaagaacacucaggaggguguuugcccaggugaagcagaucuacaaaacccuccuaucaaagauuuugg
ggauucaacuucagccagauccugccugauccuuccaaaccuuccaaaagaucauuuaucgaggaccuucuguucaacaaggucaccc
uggcugacgcuggcuuuaucaagcaguauggcgauugucuggggugauaucgcagcaagagaucugauuugugcucagaaguuuaac
ggacugacaguucugccuccgcugcugaccgaugagaugaucgcccaguacacaagugcacugcuggcuggcaccaucacauccggc
uggaccuucgagagccggcgccgcccugcagauuccuuucgcuaugcagauggcauaucgcuuuaacggaauuggcgugacucagaac
guccuguacgagaaccagaaacugaucgcaaaccaguucaacagcgcuaucggaaaaaauccaggauagccugucaucuacagccagug
cccuggggaaagcugcaggacguggugaaucagaacgcacaggcccugaacacacuggugaaacagcugagcucaaacuuuggagcua
ucuccucgguccugaacgacaucccugucccagacuggauccgccugaggcugaggugcagaucgacagacugaucacaggaagacugc
agagucugcaaaccuacgugaccagcagcucaucccgcgcugcugagaucagagcguccgcaaaccuggcagccaccaaaaugaguga
gugugugcuccggacagagcaaacgcguggauuucuguggggcaaggaguuucacaugaagcuuucccacagagcgccccacacggagu
gguguuccugcauugacuuacgucccccgcucaggagaaaauuucacaacugcgccugcuaucugccacgaugguaaggcucacuu
uccaagagaggagaguguuugugagcaauggacacauugguucgucaccagagaaacuucuacgagccucagaucaucacuaccga
caauacuuucgugucaggaaacugcgacguggugaucggcaucgucaacaauaccguuuacgacccucugcagccugagcuggacag
uuucaagagggagcuggauaaauacuucaaaaaccacacuagcccagacguggaccuggggagauaucagcggcaucaaugcaucugu
ggugaacauccagaaagagauugacagacugaacgaggugggccaagaaccugaacgaaagucugaucgaucugcaagagcugggaaa
auaugagcaguacaucaaauggccuugguuacauuuggcuggggauucaucgcuggacugaucgcaauugugauggugacaauuaugc
ugugcugcaugacaagcugcuguucuugucucaaaggaugcugcucuuugcgguagcugcuguaaauuugaugaggacgauagugag
ccugugcucaagggagucaaacugcacuauaccuaaugacucgagcugguacugcaugcacgcaaugcuagcugccccuuucccguc
cuggguaccccgagucucccccgaccucgggucccagguaugcucccaccuccaccugcccacucaccaccucugcuaguuccagac
accucccaagcacgcaaugcagcucaaaacgcuuagccuagccccacccccgggaaacagcagugauuaacccuuuugcaauaaa
cgaaaguuuaacuaagcuauacuaaccccaggguuggucaauuucgugccagccacacccuggagcuagcaaaaaaaaaaaaaaaaaaa
aaaaaaaaaaagcauaugacuaaaaaaaaaaaaaaaaaaaaaaaaaaaaaaaaaaaaaaaaaaaaaaaaaaaaaaaaaaaaaaaaaaaaa
a

<u>SEQ ID NO:3</u>

gggagacccaagcuggcuagcguuuaaacuuaagcuugguaccgagcucgggauccacuaguccagugugguggaauucgaa
uaaacuaguauucuucuggucccacagacucagagagaacccgccaccauguucgucguuuccuggugcugcugcccuuagugagcag
ccagugcgucaaccuaacuacuagaacacagcugccuccugccuacacaaacagcuucaccagaggcguguauuaccccgauaagggug
uuccggucuucugugcugcacagcaccccaggaccuguuucugccuuucuucagcaaugugaccugguuccacgccauccacgugucu
ggcacaaacggcacaaaaaggguucgacaaccccgucugccuuucaaugacggcguguacuucgcuagcacagaaaagagcaacauca
ucagaggauggaucuucggaaccacccuggauuccaagacacaguccccuguugaucguuaauaaugccaccaaugugguggaucaagg
ugugugaguuccaguuuugcaacgauccuuuucuggggcguguauuaccacaagaauaacaaaagcuggauggaaagcgaguuuaga
guauacucuagcgccaacaacugcaccuucgaguacgucagccagcccuuuucugauggaccuggaaggcaagcagggcaacuucaag
aaucugagagaguucguguucaaaaacauugacggauauuucaaaaucuacagcaagcacacaccuaucaaucugguggcgggaccug
ccacaggggguuuagcgcauuggagcccucuggucgaucugcccuaucggcaucaacaucacccgguuuccagacccugcuagcgccugcau
agaagcuaucugacccuggccgacagccucaagcggcuggaccgccggccgccgccuacuacggugggcuaccugcagccuagaaccu
uucugcugaaguacaacgagaacggcaccauaaccgacgcugugggacugcgcccuggaccccccugucugaaaccaagcgcacccugaa
gagcuuuaccguggaaaagggcaucuaccaaacaagcaauuuccggggugcagccuaccgagucuaucgugcgguuucccaacaucac
caaccugugcccauucggagaagugguucaacgccaccagauucgccagcgaguacgccuggaaccggaagagaaucucuaacugcgu
ggccgauuacagcgugcucuauaacucggcuagcuucagcacauucaagugcuacggcgugagccccacaaagcugaacgaccugug
cuucaccaacgucuacgccgacagcuucgugauuagaggcgaugaggccgacagaucgcccccuggccaaaccggcaagauugcuga
cuacaacuacaagcugccugaugacuucacuggaugugugaucgcuuggaacuccaacaaccuggacucuaaaguggggcggaaacua
caacuaccgguaccggcuguuuagaaagaguaaccuuaaaccuuucgagagagauaucagcacagaaaucuaccaggcuggcagcaca
ccuuguaacggcgugcaaggcuucaacugcuauuucccucuccagucuuuauggcuuccagccuaccaacggcguggggcuaucagccc
uaccgaguggguggugcucuccugagcugcugcaugcccugcuaccgugugcggcgcccuaagaaaucgaccaaccuggugaagaac
aagugcgugaauuuuaauuucaacggcgcucaccggcaccggaugcugacgagagcaacaagaaguuccugcccuuccagcaguuc
ggaagagacaucgccgauaccacagacgccgugcgggaccccccaaacacuggaaaucuggacaucacaccuugcagcuucggcggcg
ugagcgugaucacccuggcaccaacacaagcaaccaggguggccgugcucuaccagggcgugaacugcaccgaagugccaguggccau
ccacgccgaucagcugacccccuaccuggagaguguacagcaccggcagcaacguguuccagacaagggcugguugucugaucggcgc
cgagcacgugaauaauuccuacgagugcgacaucccuauuggcgccggaaucugugccagcuaccagacacagacaaauccccacgu
agagccagauccgucgccagccagagcaucaucgcuuacaccaugagccuggggcgcugaaaacagcguggcuuacagcaacaauucua
ucgccauaccuaccaauuuuacaaucucggugacaaccgagauccugccuguuagcaugaccaagaccagcguggacugcacaaugua
caucugcggagacagcaccgagugcuccaaccugcugcuucaauacgggguccuucuguacacagcugaauagagcccugacaggcau
cgcuguugagcaggacaagaacacccaggaggguguuugcccaagugaagcagaucuacaagacuccgccuaucaaggacuucggcgg
cuucaacuacagccagauccugcccgauaauuacaagccuagcaagcgcaguuucaucgacuguuguucaacaaggugacccu
ggccgacgccgcuuuaucaagcaguacggcgauuggcuggcgugacaucgccguagagaucgaucgcgcccagaaguucaaugg
acugacagugcugccgcccgcugcugaccgaugagaugaucgcucaguacacccucugcccugcuggccgggacaaucaccagcggaug
gacauucgggggccggcgccgcccugcagaucccuuugccaugcagaugggccuacagauucaacgguauuggcgugacccaaaacgu
gcuguacgagaaucagaaguuaaucgcaaaccaguucaacagcgccaucggcaagauccaggauucccugaguuccacggccagcgcu
cuggguaagcugcaagacguggugaaccagaacgcacaggcccugaacacccuggucaaacagcugagcuccaacuucggagcgauca

gcagcgugcuuaaugacauccugagcagacuggauccccccgaggccgaaguccaaaucgaccggcugaucacaggcagguugcaga
gccugcagaccuacgugacccaacagcugaucagagcagccgagaucagagccucagccaaucuggcugcuacgaagaugagcgagug
ugugcugggacagagcaagcggguggauuuuugcggcaaagggguaccaccugaugagcuucccucagagcgcccucacggcguug
uguuccugcacgugacguacgugcccgcccaagagaagaacuucaccacagccccugccaucugccacgacggaaaagcccacuuccc
ucgugagggcgucuucgugagcaacggcacacacugguucgugacucagagaaacuucuacgagccucagaucaucaccaccgacaac
accuuugugagcggcaacugcgacguguguaucgggaucgugaacaacaccguuacgacccccugcagccugagcuggauagcuuc
aaagaggaacuggacaaguauuucaagaaucacaccucuccagacguggaccuggggcgacaucagcggcauuaacgccucuguggug
aacauccagaaggaaaucgaucggcugaacgagguggccaagaacuugaacgagagccugaucgaccugcaggagcugggcaaguac
gagcaguacaucaaguggccuugguacaucuggcugggauucaucgccggccugauugccaucgucauggugaccaucaugcugug
uugcaugacaagcugcugcagcugucugaaggggauguugcucuugugggcucuugcuguaaauucgacgaagacgacucggaacccg
ugcugaagggcguuaagcuccacuacaccugaugacaagcacgcagcaaugcagcucaaaacgcuuagccuagccacaccccccacgggg
aaacagcagugauuaaccuuuagcaauaaacgaaaguuuaacuaagcuauacuaaccccagggguuggucaauuucgugccagccaccu
cgagcugguacugcaucacgcaaugcuagcugcccccuuucccguccuggguaccccgagcucccccgaccucgggucccagguau
gcucccaccuccaccugccccacucaccaccucugcuaguuccagacaccuccgcucgcuuucuugcuguccaauuucuauuaaaggu
uccuuuguucccuaaguccaacuacuaaacuggggggauauuaugaagggccuugagcacucuggauucugccuaauaaaaaacauuua
uuuucauugcaauugccauguguaugugggguucgcccacauacucugaugaucccccaaucguggcgugucggccugcuucggcagg
cacuggcgccgggaucauucauggcaagcuggagccucgguggccaugcuucuugcccccuugggccucccccccagccccuccucccc
uuccugcacccguaccccguggucuuugaauaaagucugagugggcggcaaaaaaaaaaaaaaaaaaaaaaaaaaaaaaaagcauaug
acuaaaaaaaaaaaaaaaaaaaaaaaaaaaaaaaaaaaaaaaaaaaaaaaaaaaaaaaaaaaaaaaaaaaaaaaaaa

SEQ ID NO:4

gggagacccaagcuggcuagcguuuaaacuuaagcuugguaccgagcucggauccacuaguccagugugguggaauucgaauaaacu
aguauucuucugguccccacagacucagagagaacccgccaccauguucguguuccuggugcugcugcccuuagugagcagccagug
cgucaaccuaacuacuagaacacagcugccuccugccuacacaaacagcuucaccagaggcguguauuaccccgauaaggcguuccgg
ucuucugugcugcacagcacccaggaccuguuucugccuuucuucagcaaugugaccucugguuccacgccauccacgugucuggcaca
aacggcacaaaaaggguucgacaaccccguucugccuuucaaugacggcguguacuucgcuagcacagaaaagagcaacaucaucagag
gauggaucuucggaaccacccuggauuccaagacacagucccguugaucguuaauaaugccaccaugguggugaucaaggugugug
aguuccaguuuugcaacgauccuuuucugggcguguauuaccacaagaauaacaaaagcuggauggaaagcgaguuuagaguauacu
cuagcgccaacaacugcaccuucgaguacgucagccagccuuuucugauggaccuggaaggcaagcagggcaacuucaagaaucuga
gagaguucguguucaaaaacauugacggauauuucaaaaucuacagcaagcacacaccuaucaaucuggugcgggaccugccacagg
gguuuagcgcauuggagccucuggucgaucugccuaucggcaucaacaucacccgguuccagacccugcccugcauagaagcu
aucugaccccuggcgacagcucaagcggcuggaccgccggcgccgccgccuacuacgugggcuaccugcagccuagaaccuuucugc
ugaaguacaacgagaacggcaccauaaccgacgcuguggacugcgcccuggaccccugucugaaaccaagugcaccugaagagcuu
uaccgguggaaaagggcaucuaccaaacaagcaauuuccgggugcagccuaccgagucuaucgugcgguuucccaacaucaccaaccug
ugcccauucggagaaguguucaacgccaccagauucgccagccguguacgccuggaaccggaagagaaucucuaacugcgguggccgau
uacagcgugcucuauaacucggcuagcuucagcacauucaagugcuacggcgugagccccacaaagcugaacgaccugugcuucacca
acgucuacgccgacagcuucgugauuagaggcgaugagguccgacagaucgccccuggccaaaccggcaagauugcugacuacaacu
acaagcugccugaugacuucacuggaugugugaucgcuuggaacuccaacaaccuggacucuaaaguggcggaaacuacaacuacc
gguaccggcuguuuagaaagaguaaccuuaaaccuuucgagagagauaucagcacagaaaucuaccaggcuggcagcacaccuugua
acggcgugcaaggcuucaacugcuauuuucccuccagucuuuaugggcuuccagccuacaacggcgugggcuaucagcccuaccgag
uggugugcuguuccuucgagcugcugcaugccccugcuaccgugugcggcccuaagaaaucgaccaaccuggugaagaacaagugcg
ugaauuuuuaauuucaacggccucaccggcaccggagugcugaccgagacaacaagaaguuccugcccuuccagcaguucggaagag
acaucgccgauaccacagacgccgugcgggacccccaaacacuggaaauccuggacaucacaccuugcagcuucggcggcgugagcgu
gaucaccccuggcaccaacacaagcaaccagguggccgugcucuaccagggcgugaacugcaccgaagugccaguggccauccacgcc
gaucagcugaccccuaccuggagaguguacagcaccggcagcaacguguuccagacaagggcugguugucugaucggcgccgagcac
gugaauaaauuccuacgagugcgacaucccuauuggcgccggaaucugugccagcuaccagacacagacaaacuccccacguagagcca
gauccgucgccagccagagcaucaucgcuuacaccaugagccuggggcgcugaaaacagcguggcuuacagcaacaauuucuaucgccau
accuaccaauuuuacaaucucggugacaaccgagauccugccuguuagcaugaccaagaccagcguggacugcacaauguacaucugc
ggagacagcaccgagugcuccaaccugcugcuucaauacggguccuucguuacacagcugaauagagcccugacaggcaucgcuguu
gagcaggacaagaacacccaggagguguuugcccaagugaagcagaucuacaagacuccgccuaucaaggacuucggcggcuucaacu
ucagccagauccugccagauccuucuuaagccuagcaagcgcaguuucaucggaggaccguuguucaacaaggugacccuggccgacg
ccggcuuuaucaagcaguacggcgauugccugggcgacaucgccgcuagagaucugaucugcgcccagaaguucaauggacugacag
ugcugccgccccugcugaccgaugagaugaucgcucaguacaccucugcccugcuggccgggacaacaccagcggauggacauucg
gggccggcgccgcccugcagauccccuuugccaugcagaugccacagauucaacgguauuggcgugacccaaaacgugcuguacg
agaaucagaaguuaaucgcaaaccaguucaacagcgccaucggcaagauccaggauucccugaguuccacggccagcgcucuggguaa
gcugcaagacguggugaaccagaacgcacaggcccugaacacccuggucaaacagcugagcuccaacuucggagcgaucagcagcgug
cuuaaugacauccugagcagacuggaucccccccgaggccgaaguccaaaucgaccggcugaucacaggcagguugcagagccugcaga
ccuacgugacccaacagcugaucagagcagccgagaucagagccucagccaaucuggcugcuacgaagaugagcgagugugugcugg
gacagagcaagcgggguggauuuuugcggcaaagggguaccaccugaugagcuuccccucagagcgcccccucacggcguugguguuccugc
acgugacguacgugccgcccaagagaagaacuucaccacagccccugccaucugccacgacgaaaagcccacuuccccucgugaggg
cgucuucgugagcaacggcacacaugguucgugacucagagaaacuucuacgagccucagaucaucaccgacaacaccuuugug
agcggcaacugcgacguggugaucgggaucgugaacaacaccguguacgacccccugcagccugagcuggauagcuucaaagaggaa
cuggacaaguauuucaagaaucacaccucuccagacguggaccuggggcgacaucagcggcauuaacgccucguguggugaacauccag
aaggaaaucgaucggcugaacgaggugggccaagaacuugaacgagagccugaucgaccugcaggagcuggggcaaguacgagcaguac
aucaaguggccuugguacaucuggcugggauucaucgccggccugauugccaucgucauggugaccaucaugcuguguugcaugac
aagcugcugcagcugucugaagggauguugcucuuguggcucuugcguuaaauucgacgaagacgacucggaacccgugcugaagg
gcguuaagcuccacuacaccugaugagcucgcuuucuugcuguccaauuucuauuaaaagguuccuuuguucccuaaguccaacuacu
aaacuggggggauauuaugaagggccuugagcaucuggauucugccuaauaaaaaacauuuauuuucauugcaauugccauguguau
gugggguucgcccacauacucugaugauccccaaucguggcgugucggccugcuucggcaggcacuggcgccgggaucauucauggc
aagcuggagccucgguggccaugcuucuugccccuugggccucccccagccccuccuccccuuccugcacccguaccccgugguc
uuugaauaaagucugagugggcggcacucgagcugguacugcaugcacgcaaugcuagcugcccccuuucccguccugggguaccccga

gucucccccgaccucgggucccagguaugcucccaccuccaccugccccacucaccaccucugcuaguuccagacaccuccaaaaaaaa
aaaaaaaaaaaaaaaaaaagcauaugacuaaaaaaaaaaaaaaaaaaaaaaaaaaaaaaaaaaaaaaaaaaaaaaaaaaaaaaaaaa
aaaaaaaaaa

SEQ ID NO:5

gggagacccaagcuggcuagcguuuaaacuuaagcuugguaccgagcucggauccacuaguccagugugguggaauucgaa
uaaacuaguauucuucugguccccacagacucagagagaacccgccaccauguucguguuccuggugcugcugcccuuagugagcag
ccagugcgucaaccuaacuacuagaacacagcugccuccugccuacacaaacagcuucaccagaggcguguauuaccccgauaaggug
uuccggucuucugugcugcacagccaccaggaccuguuucugccuuucuucagcaaugugaccugguuccacgccauccacgugucu
ggcacaaacggcacaaaaaggguucgacaaccccguucugccuuucaaugacggcguguacuucgcuagcacagaaaagagcaacauca
ucagaggauggaucuucggaaccacccuggauuccaagacacaguccccuguugaucguuaauaaugccaccaauguggugaucaagg
ugugugaguuccaguuuugcaacgauccuuuucgggcguguauuaccacaagaauaacaaaagcuggauggaaagcgaguuuaga
guauacucuagcgccaacaacugcaccuucgaguacgucagccagccuuuucugauggaccuggaaggcaagcagggcaacuucaag
aaucugagagaguucguguucaaaaacauugacgggauauuucaaaaucuacagcaagcacacaccuaucaaucuggugcgggaccug
ccacaggggguuuagcgcauuggagccucuggucgaucugccuaucggcaucaacaucacccgguuccagacccugcuagcccugcau
agaagcuaucugaccccuggcgacagcucaagcggcuggaccgccggcgccgccgccuacuacgugggcuaccugcagccuagaaccu
uucugcugaaguacaacgagaacggcaccauaaccgacgcuguggacugcgcccuggaccccugucugaaaccaagugcacccugaa
gagcuuuaccguggaaaagggcaucuaccaaacaagcaauuuccggggugcagccuaccgagucuaucgugcgguuucccaacaucac
caaccugugcccauucggagaagugguucaacgccaccagauucgccagcguguacgccuggaaccggaagagaaucucuaacugcgu
ggccgauuacagcgugcucuauaacucggcuagcuucagcacauucaagugcuacggcgugagccccacaaagcugaacgaccugug
cuucaccaacgucuacgccgacagcuucgugauuagaggcgaugagguccgacagaucgcccccuggccaaaccggcaagauugcuga
cuacaacuacaagcugccugaugacuucacuggaugugugaucgcuuggaacuccaacaaccuggacucuaaaguggggcgggaaacua
caacuaccgguaccggcuguuuagaaagaguaaccuuaaaccuuucgagagagauaucagcacagaaaucuaccaggcuggcagcaca
ccuuguaacgacgaaggcuucaacgucuauuucccucuccagucuuuauggcuuccagccuaccaacggcgugggccuaucagccu
uaccgaguggugguggcuguuccuucgagcugcugcaugccccugcuaccgugugcggcccuaagaaaucgaccaaccuggugaagaac
aagugcgugaauuuaauuuacaacggccucaccggcaccggagugcugaccgagagcaacaagaaguuccugcccuuccagcaguuc
ggaagagacaucgccgauaccacagacgccgugcgggaccccaaacacuggaaauccuggacaucacaccuugcagcuucggcggcg
ugagcgugaucaccccuggccaccaacacaagcaaccaggugggccgugcucuaccagggcgugaacugcaccgaagugccaguggccau
ccacgccgaucagcugaccccuaccuggagagaguguacagcaccggcagcaacguguuccagacaagggcugguugcugaucggcgc
cgagcacgugaauaauuccuacgagugcgacaucccuauuggcgccggaaucugugccagcuaccagacacagacaaacuccccacgu
agagccagauccgucgccagccagagcaucaucgcuuacaccaugagccugggcgcugaaaacagcguggcuuacagcaacaauucua
ucgccauaccuaccaauuuuacaaucucgguagcaaccgagauccugccuguuagcaugaccaagaccagcguggacugcacaaugua
caucugcggagacgccaccgagugccuccaaccuccgucugcuucaauacgggguccuuucguacacagcugaauagagcccugacaggcau
cgcuguugagcaggacaagacacccaggagggcguuugcccaagugaagcagaucuacaagacuccgccucaucaaggacuuccgugcgg
cuucaacuucagccagauccugccagaucculucuaagccuagcaagcgcaguuucaucgaggaccgguuguucaacaaggugacccu
ggccgacgccggcuuuaucaagcaguacggcgauugccugggcgacaucgccgcuagagaucugaucugcgcccagaaguucaauggg
acugacagugcugccgccccugcugaccgaugagaugaucgcucaguacaccucugcccugcuggccgggacaaucaccagcggaug
gacauucggggccggcgccgcccugcagauccccuuugccaugcagauggccuacagauucaacgguauuggcgugacccaaaacgu
gcguacgagaaucagaaguuaaucgcaaaccaguucaacagcgccaucggcaagauccaggauucccugaguuccacggccagcgcu
cugggguaagcugcaagacguggugaaccagaacgcacaggcccugaacacccuggucaaacagcugagcuccaacuucggagcgauca
gcagcgugcuuaaugacauccugagcagacuggaucccccccgaggccgaaguccaaaucgaccggcugaucacaggcagguugcaga
gccugcagaccuacgugacccaacagcugaucagagcagccgagaucagagccucagccaaucuggcugcuacgaagaugagcgagug
ugugcuggggacagagcaagcagcgggguggauuuuuugcggcaaaggguaccaccugaugagcuucccuccugagcgccccucacggcgug
uguuccugcacgugacguacgugcccgcccaagagaagaacuuaccacacagccccugccucaucugccacgacgggaaaagcccacuuccc
ucgugagggcgucuucgugagcaacggcacacacugguucgugacucagagaaacuucuacgagccucagaucaucaccaccgacaac
accuuugugagcggcaacugcgacguggugaucgggaucgugaacaacaccguguacgaccccugcagccugagcuggauagcuuc
aaagaggaacuggacaaguauuucaagaaucacaccucuccagacguggaccuggggcgacaucagcggcauuaacgccucuguggug
aacauccagaaggaaaucgaucggcugaacgaggugggccaagaacuugaacgagagccugaucgaccugcaggagcugggcaaguac
gagcaguacaucaaguggccuugguacaucuggcugggauucaucgccggccugauugccaucgucaugguaccaucaugcugug
uugcaugacaagcugcugcagcugucugaagggauguugcucuuguggcucuugcguaaauucgacgaagacgacucgggaacccg
ugcugaagggcguuaagcuccacuacaccugaugagcucgcuuucuugcuguccaauuucuauuaaagguuccuuuguucccuaag
uccaacuacuaaacgggggggauauuaugaagggccuugagcaucuggauucugccuaauaaaaaacauuuauuuucauugcaauugc
cauguguaugugggguucgcccacauacucugaugauccccaaucggcgugucggccugcuucggcaggcacuggcgccggggaucg
auucaugggcaacucgagcugguacugcaugcacgcaaugcuagcugcccuuucccguccuggguaccccgagucuccccaccuc
ggguccaggguaugcucccaccuccaccugcccacucacaccacucugcuaguuccagacacccucc

SEQ ID NO:6

gggagacccaagcuggcuagcguuuaaacuuaagcuugguaccgagcucggauccacuaguccagugugguggaauucgaa
uaaacuaguauucuucugguccccacagacucagagagaacccgccaccauguucguguuccuggugcugcugcccuuagugagcag
ccagugcgucaaccuaacuacuagaacacagcugccuccugccuacacaaacagcuucaccagaggcguguauuaccccgauaaggug
uuccggucuucugugcugcacagccaccaggaccuguuucugccuuucuucagcaaugugaccugguuccacgccauccacgugucu
ggcacaaacggcacaaaaaggguucgacaaccccguucugccuuucaaugacggcguguacuucgcuagcacagaaaagagcaacauca
ucagaggauggaucuucggaaccacccuggauuccaagacacaguccccuguugaucguuaauaaugccaccaauguggugaucaagg
ugugugaguuccaguuuugcaacgauccuuuucgggcguguauuaccacaagaauaacaaaagcuggauggaaagcgaguuuaga
guauacucuagcgccaacaacugcaccuucgaguacgucagccagccuuuucugauggaccuggaaggcaagcagggcaacuucaag
aaucugagagaguucguguucaaaaacauugacgggauauuucaaaaucuacagcaagcacacaccuaucaaucuggugcgggaccug
ccacaggggguuuagcgcauuggagccucuggucgaucugccuaucggcaucaacaucacccgguuccagacccugcuagcccugcau
agaagcuaucugaccccuggcgacagcucaagcggcuggaccgccggcgccgccgccuacuacgugggcuaccugcagccuagaaccu
uucugcugaaguacaacgagaacggcaccauaaccgacgcuguggacugcgcccuggaccccugucugaaaccaagugcacccugaa
gagcuuuaccguggaaaagggcaucuaccaaacaagcaauuuccggggugcagccuaccgagucuaucgugcgguuucccaacaucac
caaccugugcccauucggagaagugguucaacgccaccagauucgccagcguguacgccuggaaccggaagagaaucucuaacugcgu
ggccgauuacagcgugcucuauaacucggcuagcuucagcacauucaagugcuacggcgugagccccacaaagcugaacgaccugug

cuucaccaacgucuacgccgacagcuucgugauuagaggcgaugagguccgacagaucgccccuggccaaaccggcaagauugcuga
cuacaacuacaagcugccgaugacuucacuggaugugugaucgcuuggaacuccaacaaccuggacucuaaaguggggcggaaacua
caacuaccgguaccggcuguuuagaaagaguaaccuuaaaccuuucgagagagauaucagcacagaaaucuaccaggcuggcagcaca
ccuuguaacggcgugcaaggcuucaacugcuauuucccucuccagucuuauggcuuccagccuaccaacggcgugggcuaucagccc
uaccgaguggguggugcuguccuucgagcugcugcaugccccugcuaccgugugcggcccuaagaaaucgaccaaccuggugaagaac
aagugccgugaauuuuaauuucaacggccucaccggcaccggagugcugaccgagagcaacaagaaguuccugcccuuccagcaguuc
ggaagagacaucgccgauaccacagacgccgugcgggacccccaaacacuggaaauccuggacaucacaccuugcagcuucggcggcg
ugagcgugaucacccccuggcaccaacacaagcaaccagguggccgugcucuaccagggcgugaacugcaccgaagugccaguggccau
ccacgccgaucagcugaccccuaccuggagaguguacagcaccggcagcaacguguuccagacaagggcugguugucugaucggcgc
cgagcgaugaauaaauuccuacgagugcgacaucccuauuggcgccggaaucugugccuaccaccagacacaaucuccccacgu
agagccagauccgucgccagccagagcaucucuuacaccaugagccugggcgcugaaaacagcguggcuuacagcaacaauucua
ucgccauaccuaccaauuuuacaaucucggugacaaccgagauccugccuguuagcaugaccaagaccagcguggacugcacaaugua
caucugcggagacagcaccgagugcuccaaccugcugcuucaauacgggguccuucuguacacagcugaauagagcccugacaggcau
cgcuguugagcaggacaagaacacccaggaggguguuugcccaagugaagcagaucuacaagacuccgccuaucaaggacuucggcgg
cuucaacuucagccagauccugccagauccuucuaagccuagcaagcgcaguuucaucgaggaccuguuguucaacaaggugacccu
ggccgacgccggcuuuaucaagcaguacggcgauugccugggcgacaucgccgcuagagaucugaucugcgcccagaaguucaaugg
acugacagugcugccgccccugcugaccgaugagaugaucgcucaguacaccucugcccugcuggccgggacaaucaccagcggaug
gacauucggggccggcgccgcccugcagaucccuuugccaugcagaugggccuacagauucaacgguauuggcgugacccaaaacgu
gcuguacgagaaucagaaguuaaucgcaaaccaguucaacagcgccaucggcaagauccaggauucccugaguuccacggccagcgcu
cuggguaagcucaagacguggugaaccagaacgcacaggcccugaacaccuggucaaacagcugagcuccaacuucggagcgauca
gcagcgugcuuaaugacaucugagcagacugggaucccccccgaggccgaaguccaaaucgaccggcugaucacaggcagguugcaga
gccugcagaccuacgugacccaacagcugaucagagcagccgagaucagagccucagccaaucuggcugcuacgaagaugagcgagug
ugugcugggacagagcaagcggguggauuuuugcggcaaagggguaccaccugaugagcuucccucagagcgcccccucacggcguug
uguuccugcacgugacguacgugcccgcccaagagaagaacuucaccacagccccugccaucugccacgacggaaaagcccacuuccc
ucgugagggcgucuucgugagcaacggcacacacugguucgugacucagagaaacuucuacgagccucagaucaucaccaccgacaac
accuuugugagcggcaacugcgacguggugaucgggaucgugaacaacaccguguacgacccccugcagccugagcuggauagcuuc
aaagaggaacuggacaaguauuucaagaaucacaccucuccagacguggaccugggcgacaucagcggcauuaacgccucuguggug
aacauccagaaggaaaucgaucggcugaacgagguggccaagaacuugaacgagagccugaucgaccugcaggagcugggcaaguac
uugcaugacaagcugcugcagcugucugaagggauguugcucuuguggcucuugacgaagacgacucggaacccg
ugcugaagggcguuaagcuccacuacaccgaugagcucguucuuugcuguccaauuuucuauuaaaggguuccuuuguuccuaag
uccaacuacuaaacgggggauauuaugaaggggccuugagcaucuggaucugccuaauaaaaacauuuauuuucauugcaauugc
caugguguaugugggguucgcccacauacucugaugaucccaaucguggcgugucggccugcuucggcaggcacuggcgccgggauc
auucauggcaagcuggagccucgguggccaugcuucuugcccuugggccucccccagcccuccucccuuccugcacccguaccc
ccguggucuuugauaaagucugaguggcggcaaaaaaaaaaaaaaaaaaaaaaaaaaaaaaaagcauaugacuaaaaaaaaaaaaaaa
aaaaaaaaaaaaaaaaaaaaaaaaaaaaaaaaaaaaaaaaaaaaaaaaaaaa

SEQ ID NO:7

MFVFLVLLPLVSSQCVNLTTRTQLPPAYTNSFTRGVYYPDKVFRSSVLHSTQDLFLPFFS
NVTWFHAIHVSGTNGTKRFDNPVLPFNDGVYFASTEKSNIIRGWIFGTTLDSKTQSLLIVNNAT
NVVIKVCEFQFCNDPFLGVYYHKNNKSWMESEFRVYSSANNCTFEYVSQPFLMDLEGKQGNF
KNLREFVFKNIDGYFKIYSKHTPINLVRDLPQGFSALEPLVDLPIGINITRFQTLLALHRSYLTPG
DSSSGWTAGAAAYYVGYLQPRTFLLKYNENGTITDAVDCALDPLSETKCTLKSFTVEKGIYQT
SNFRVQPTESIVRFPNITNLCPFGEVFNATRFASVYAWNRKRISNCVADYSVLYNSASFSTFKCY
GVSPTKLNDLCFTNVYADSFVIRGDEVRQIAPGQTGKIADYNYKLPDDFTGCVIAWNSNNLDS
KVGGNYNYRYRLFRKSNLKPFERDISTEIYQAGSTPCNGVQGFNCYFPLQSYGFQPTNGVGYQP
YRVVVLSFELLHAPATVCGPKKSTNLVKNKCVNFNFNGLTGTVLTESNKKFLPFQQFGRDIA
DTTDAVRDPQTLEILDITPCSFGGVSVITPGTNTSNQVAVLYQGVNCTEVPVAIHADQLTPTWR
VYSTGSNVFQTRAGCLIGAEHVNNSYECDIPIGAGICASYQTQTNSPRRARSVASQSIIAYTMSL
GAENSVAYSNNSIAIPTNFTISVTTEILPVSMTKTSVDCTMYICGDSTECSNLLLQYGSFCTQLNR
ALTGIAVEQDKNTQEVFAQVKQIYKTPPIKDFGGFNFSQILPDPSKPSKRSFIEDLLFNKVTLADA
GFIKQYGDCLGDIAARDLICAQKFNGLTVLPPLLTDEMIAQYTSALLAGTITSGWTFGAGAALQ
IPFAMQMAYRFNGIGVTQNVLYENQKLIANQFNSAIGKIQDSLSSTASALGKLQDVVNQNAQA
LNTLVKQLSSNFGAISSVLNDILSRLDPPEAEVQIDRLITGRLQSLQTYVTQQLIRAAEIRASANL
AATKMSECVLGQSKRVDFCGKGYHLMSFPQSAPHGVVFLHVTYVPAQEKNFTTAPAICHDGK
AHFPREGVFVSNGTHWFVTQRNFYEPQIITTDNTFVSGNCDVVIGIVNNTVYDPLQPELDSFKEE
LDKYFKNHTSPDVDLGDISGINASVVNIQKEIDRLNEVAKNLNESLIDLQELGKYEQYIKWPWY
IWLGFIAGLIAIVMVTIMLCCMTSCCSCLKGCCSCGSCCKFDEDDSEPVLKGVKLHYT

(The lineations indicate related mutation sites relative to the WT strain)
SEQ ID NO:8(SF-1-BA.5 BA.5S protein natural sequence)

GGGAGACCCAAGCTGGCTAGCGTTTAAACTTAAGCTTGGTACCGAGCTCGGATCCAC
TAGTCCAGTGTGGTGGAATTCAGAATAAACTAGTATTCTTCTGGTCCCCACAGACTCAGAG
AGAACCCGCCACCATGTTTGTTTTTCTTGTTTTATTGCCACTAGTCTCTAGTCAGTGTGTTAA
TCTTATAACCAGAACTCAATCATACACTAATTCTTTCACACGTGGTGTTTATTACCCTGACA
AAGTTTTCAGATCCTCAGTTTTACATTCAACTCAGGACTTGTTCTTACCTTTCTTTTCCAATG
TTACTTGGTTCCATGCTATCTCTGGGACCAATGGTACTAAGAGGTTTGATAACCCTGTCCTA
CCATTTAATGATGGTGTTTATTTTGCTTCCACTGAGAAGTCTAACATAATAAGAGGCTGGAT
TTTTGGTACTACTTTAGATTCGAAGACCCAGTCCCTACTTATTGTTAATAACGCTACTAATG
TTGTTATTAAAGTCTGTGAATTTCAATTTTGTAATGATCCATTTTTGGATGTTTATTACCACA
AAAACAACAAAAGTTGGATGGAAAGTGAGTTCAGAGTTTATTCTAGTGCGAATAATTGCAC
TTTTGAATATGTCTCTCAGCCTTTTCTTATGGACCTTGAAGGAAAACAGGGTAATTTCAAAA

ATCTTAGGGAATTTGTGTTTAAGAATATTGATGGTTATTTTAAAATATATTCTAAGCACACG
CCTATTAATTTAGGGCGTGATCTCCCTCAGGGTTTTTCGGCTTTAGAACCATTGGTAGATTT
GCCAATAGGTATTAACATCACTAGGTTTCAAACTTTACTTGCTTTACATAGAAGTTATTTGA
CTCCTGGTGATTCTTCTTCAGGTTGGACAGCTGGTGCTGCAGCTTATTATGTGGGTTATCTT
CAACCTAGGACTTTTCTATTAAAATATAATGAAAATGGAACCATTACAGATGCTGTAGACT
GTGCACTTGACCCTCTCTCAGAAACAAAGTGTACGTTGAAATCCTTCACTGTAGAAAAAGG
AATCTATCAAACTTCTAACTTTAGAGTCCAACCAACAGAATCTATTGTTAGATTCCTAATA
TTACAAACTTGTGCCCTTTTGATGAAGTTTTTAACGCCACCAGATTTGCATCTGTTTATGCTT
GGAACAGGAAGAGAATCAGCAACTGTGTTGCTGATTATTCTGTCCTATATAATTTCGCACC
ATTTTTCGCTTTTAAGTGTTATGGAGTGTCTCCTACTAAATTAAATGATCTCTGCTTTACTAA
TGTCTATGCAGATTCATTTGTAATTAGAGGTAATGAAGTCAGCCAAATCGCTCCAGGGCAA
ACTGGAAATATTGCTGATTATAATTATAAATTACCAGATGATTTTACAGGCTGCGTTATAGC
TTGGAATTCTAACAAGCTTGATTCTAAGGTTGGTGGTAATTATAATTACCGGTATAGATTGT
TTAGGAAGTCTAATCTCAAACCTTTTGAGAGAGATATTTCAACTGAAATCTATCAGGCCGG
TAACAAACCTTGTAATGGTGTTGCAGGTGTTAATTGTTACTTTCCTTTACAATCATATGGTT
TCCGACCCACTTATGGTGTTGGTCACCAACCATACAGAGTAGTAGTACTTTCTTTTGAACTT
CTACATGCACCAGCAACTGTTTGTGGACCTAAAAAGTCTACTAATTTGGTTAAAAACAAAT
GTGTCAATTTCAACTTCAATGGTTTAACAGGCACAGGTGTTCTTACTGAGTCTAACAAAAA
GTTTCTGCCTTTCCAACAATTTGGCAGAGACATTGCTGACACTACTGATGCTGTCCGTGATC
CACAGACATTGAGATTCTTGACATTACACCATGTTCTTTTGGTGGTGTCAGTGTTATAACA
CCAGGAACAAATACTTCTAACCAGGTTGCTGTTCTTTATCAGGGTGTTAACTGCACAGAAG
TCCCTGTTGCTATTCATGCAGATCAACTTACTCCTACTTGGCGTGTTTATTCTACAGGTTCTA
ATGTTTTTCAAACGTGCAGGCTGTTTAATAGGGGCTGAATATGTCAACAACTCATATGA
GTGTGACATACCCATTGGTGCAGGTATATGCGCTAGTTATCAGACTCAGACTAAGTCTCAT
CGGCGGGCACGTAGTGTAGCTAGTCAATCCATCATTGCCTACACTATGTCACTTGGTGCAG
AAAATTCAGTTGCTTACTCTAATAACTCTATTGCCATACCCACAAATTTTACTATTAGTGTT
ACCACAGAAATTCTACCAGTGTCTATGACCAAGACATCAGTAGATTGTACAATGTACATTT
GTGGTGATTCAACTGAATGCAGCAATCTTTTGTTGCAATATGGCAGTTTTTGTACACAATTA
AAACGTGCTTTAACTGGAAATAGCTGTTGAACAAGACAAAAACACCCAAGAAGTTTTTGCAC
AAGTCAAACAAATTTACAAAACACCACCAATTAAATATTTTGGTGGTTTTAATTTTTCAACAA
ATATTACCAGATCCATCAAAACCAAGCAAGAGGTCATTTATTGAAGATCTACTTTTCAACA
AAGTGACACTTGCAGATGCTGGCTTCATCAAACAATATGGTGATTGCCTTGGTGATATTGCT
GCTAGAGACCTCATTTGTGCACAAAAGTTTAACGGCCTTACTGTTTTGCCACCTTTGCTCAC
AGATGAAATGATTGCTCAATACACTTCTGCACTGTTAGCGGGTACAATCACTTCTGGTTGG
ACCTTTGGTGCAGGTGCTGCATTACAAATACCATTTGCTATGCAAATGGCTTATAGGTTTAA
TGGTATTGGAGTTACACAGAATGTTCTCTATGAGAACCAAAAATTGATTGCCAACCAATTT
AATAGTGCTATTGGCAAAATTCAAGACTCACTTTCTTCCACAGCAAGTGCACTTGGAAAAC
TTCAAGATGTGGTCAACCATAATGCACAAGCTTTAAACACGCTTGTTAAACAACTTAGCTC
CAAATTTGGTGCAATTTCAAGTGTTTTAAATGATATCCTTTCACGTCTTGACAAAGTTGAGG
CTGAAGTGCAAATTGATAGGTTGATCACAGGCAGACTTCAAAGTTTGCAGACATATGTGAC
TCAACAATTAATTAGAGCTGCAGAAATCAGAGCTTCTGCTAATCTTGCTGCTACTAAAATG
TCAGAGTGTGTACTTGGACAATCAAAAAGAGTTGATTTTTGTGGAAAGGGCTATCATCTTA
TGTCCTTCCCTCAGTCAGCACCTCATGGTGTAGTCTTCTTGCATGTGACTTATGTCCCTGCA
CAAGAAAAGAACTTCACAACTGCTCCTGCCATTTGTCATGATGGAAAAGCACACTTTCCTC
GTGAAGGTGTCTTTGTTTCAAATGGCACACACTGGTTTGTAACACAAAGGAATTTTTATGA
ACCACAAATCATTACTACAGACAACACATTTGTGTCTGGTAACTGTGATGTTGTAATAGGA
ATTGTCAACAACACAGTTTATGATCCTTTGCAACCTGAATTAGATTCATTCAAGGAGGAGTT
AGATAAATATTTTAAGAATCATACATCACCAGATGTTGATTTAGGTGACATCTCTGGCATTA
ATGCTTCAGTTGTAAACATTCAAAAAGAAATTGACCGCCTCAATGAGGTTGCCAAGAATTT
AAATGAATCTCTCATCGATCTCCAAGAACTTGGAAAGTATGAGCAGTATATAAAATGGCCA
TGGTACATTTGGCTAGGTTTTATAGCTGGCTTGATTGCCATAGTAATGGTGACAATTATGCT
TTGCTGTATGACCAGTTGCTGTAGTTGTCTCAAGGGCTGTTGTTCTTGTGGATCCTGCTGCA
AATTTGATGAAGACGACTCTGAGCCAGTGCTCAAAGGAGTCAAATTACATTACACATGATG
ACTCGAGCTGGTACTGCATGCACGCAATGCTAGCTGCCCCTTTCCCGTCCTGGGTACCCCGA
GTCTCCCCCGACCTCGGGTCCCAGGTATGCTCCCACCTCCACCTGCCCCACTCACCACCTCT
GCTAGTTCCAGACACCTCCCAAGCACGCAGCAATGCAGCTCAAAACGCTTAGCCTAGCCAC
ACCCCCACGGGAAACAGCAGTGATTAACCTTTAGCAATAAACGAAAGTTTAACTAAGCTAT
ACTAACCCCAGGGTTGGTCAATTTCGTGCCAGCCACACCCTGGAGCTAGCAAAAAAAAAA
AAAAAAAAAAAAAAAAAAAAAGCATATGACTAAAAAAAAAAAAAAAAAAAAAAAAAAAAA
AAAAAAAAAAAAAAAAAAAAAAAAAAAAAAAAAAAAAAAA

SEQ ID NO.9 (SF-2-BA.5 codon optimization sequence)

GGGAGACCCAAGCTGGCTAGCGTTTAAACTTAAGCTTGGTACCGAGCTCGGATCCAC
TAGTCCAGTGTGGTGGAATTCGGGAAATAAGAGAGAAAGAAGAGTAAGAAGAAATATAA
GAGCCACCATGTTCGTTTTTTTAGTTTTTATTACCTTTAGTGAGCAGCCAGTGCGTGAACCTG
ATCACCCGGACCCAGAGCTACACCAACAGCTTCACCCGGGGGCGTGTACTACCCCGACAAGG
TGTTCCGGAGCAGCGTGCTGCACAGCACCCAGGACCTGTTCCTGCCCTTCTTCAGCAACGT
GACCTGGTTCCACGCCATCAGCGGCACCAACGGCACCAAGCGGTTCGACAACCCCGTGCTG
CCCTTCAACGACGGCGTGTACTTCGCCAGCACCGAGAAGAGCAACATCATCCGGGGCTGGA
TCTTCGGCACCACCCTGGACAGCAAGACCCAGAGCCTGCTGATCGTGAACAACGCCACCAA

CGTGGTGATCAAGGTGTGCGAGTTCCAGTTCTGCAACGACCCCTTCCTGGACGTGTACTAC
CACAAGAACAACAAGAGCTGGATGGAGAGCGAGTTCCGGGTGTACAGCAGCGCCAACAAC
TGCACCTTCGAGTACGTGAGCCAGCCCTTCCTGATGGACCTGGAGGGCAAGCAGGGCAACT
TCAAGAACCTGCGGGAGTTCGTGTTCAAGAACATCGACGGCTACTTCAAGATCTACAGCAA
GCACACCCCCATCAACCTGGGCCGGGACCTGCCCCAGGGCTTCAGCGCCCTGGAGCCCCTG
GTGGACCTGCCCATCGGCATCAACATCACCCGGTTCCAGACCCTGCTGGCCCTGCACCGGA
GCTACCTGACCCCCGGCGACAGCAGCAGCGGCTGGACCGCCGGCGCCGCCGCCTACTACGT
GGGCTACCTGCAGCCCCGGACCTTCCTGCTGAAGTACAACGAGAACGGCACCATCACCGAC
GCCGTGGACTGCGCCCTGGACCCCCTGAGCGAGACCAAGTGCACCCTGAAGAGCTTCACCG
TGGAGAAGGGCATCTACCAGACCAGCAACTTCCGGGTGCAGCCCACCGAGAGCATCGTGC
GGTTCCCCAACATCACCAACCTGTGCCCCTTCGACGAGGTGTTCAACGCCACCCGGTTCGC
CAGCGTGTACGCCTGGAACCGGAAGCGGATCAGCAACTGCGTGGCCGACTACAGCGTGCT
GTACAACTTCGCCCCCTTCTTCGCCTTCAAGTGCTACGGCGTGAGCCCCACCAAGCTGAAC
GACCTGTGCTTCACCAACGTGTACGCCGACAGCTTCGTGATCCGGGGCAACGAGGTGAGCC
AGATCGCCCCCGGCCAGACCGGCAACATCGCCGACTACAACTACAAGCTGCCCGACGACTT
CACCGGCTGCGTGATCGCCTGGAACAGCAACAAGCTGGACAGCAAGGTGGGCGGCAACTA
CAACTACCGGTACCGGCTGTTCCGGAAGAGCAACCTGAAGCCCTTCGAGCGGGACATCAGC
ACCGAGATCTACCAGGCCGGCAACAAGCCCTGCAACGGCGTGGCCGGCGTGAACTGCTAC
TTCCCCCTGCAGAGCTACGGCTTCCGGCCCACCTACGGCGTGGGCCACCAGCCCTACCGGG
TGGTGGTGCTGAGCTTCGAGCTGCTGCACGCCCCCGCCACCGTGTGCGGCCCCAAGAAGAG
CACCAACCTGGTGAAGAACAAGTGCGTGAACTTCAACTTCAACGGCCTGACCGGCACCGGC
GTGCTGACCGAGAGCAACAAGAAGTTCCTGCCCTTCCAGCAGTTCGGCCGGGACATCGCCG
ACACCACCGACGCCGTGCGGGACCCCCAGACCCTGGAGATCCTGGACATCACCCCCTGCAG
CTTCGGCGGCGTGAGCGTGATCACCCCCGGCACCAACACCAGCAACCAGGTGGCCGTGCTG
TACCAGGGCGTGAACTGCACCGAGGTGCCCGTGGCCATCCACGCCGACCAGCTGACCCCCA
CCTGGCGGGTGTACAGCACCGGCAGCAACGTGTTCCAGACCCGCGCCGGCTGCCTGATCGG
CGCCGAGTACGTGAACAACAGCTACGAGTGCGACATCCCCATCGGCGCCGGCATCTGCGCC
TCCTACCAGACCCAGACCAAGAGCCACCGGCGGGCCCGGAGCGTGGCCAGCCAGAGCATC
ATCGCCTACACCATGAGCCTGGGCGCCGAGAACAGCGTGGCCTACAGCAACAACAGCATC
GCCATCCCCACCAACTTCACCATCAGCGTGACCACCGAGATCCTGCCCGTGAGCATGACCA
AGACCAGCGTGGACTGCACCATGTACATCTGCGGCGACAGCACCGAGTGCAGCAACCTGCT
GCTGCAGTACGGCAGCTTCTGCACCCAGCTGAAGCGGGCCCTGACCGGCATCGCCGTGGAG
CAGGACAAGAACACCCAGGAGGTGTTCGCCCAGGTGAAGCAGATCTACAAGACCCCCCCC
ATCAAGTACTTCGGCGGCTTCAACTTCAGCCAGATCCTGCCCGACCCCAGCAAGCCCAGCA
AGCGGAGCTTCATCGAGGACCTGCTGTTCAACAAGGTGACCCTGGCCGACGCCGGCTTCAT
CAAGCAGTACGGCGACTGCCTGGGCGACATCGCCGCCCGGGACCTGATCTGCGCCCAGAA
GTTCAACGGCCTGACCGTGCTGCCCCCCCTGCTGACCGACGAGATGATCGCCCAGTACACC
AGCGCCCTGCTGGCCGGCACCATCACCAGCGGCTGGACCTTCGGCGCCGGCGCCGCCCTGC
AGATCCCCTTCGCCATGCAGATGGCCTACCGGTTCAACGGCATCGGCGTGACCCAGAACGT
GCTGTACGAGAACCAGAAGCTGATCGCCAACCAGTTCAACAGCGCCATCGGCAAGATCCA
GGACAGCCTGAGCAGCACCGCCAGCGCCCTGGGCAAGCTGCAGGACGTGGTGAACCACAA
CGCCCAGGCCCTGAACACCCTGGTGAAGCAGCTGAGCAGCAAGTTCGGCGCCATCAGCAG
CGTGCTGAACGACATCCTGAGCCGGCTGGACCCCCCCGAGGCCGAGGTGCAGATCGACCG
GCTGATCACCGGCCGGCTGCAGAGCCTGCAGACCTACGTGACCCAGCAGCTGATCCGGGCC
GCCGAGATCCGGGCCAGCGCCAACCTGGCCGCCACCAAGATGAGCGAGTGCGTGCTGGGC
CAGAGCAAGCGGGTGGACTTCTGCGGCAAGGGCTACCACCTGATGAGCTTCCCCCAGAGC
GCCCCCCACGGCGTGGTGTTCCTGCACGTGACCTACGTGCCCGCCCAGGAGAAGAACTTCA
CCACCGCCCCCGCCATCTGCCACGACGGCAAGGCCCACTTCCCCCGGGAGGGCGTGTTCGT
GAGCAACGGCACCCACTGGTTCGTGACCCAGCGGAACTTCTACGAGCCCCAGATCATCACC
ACCGACAACACCTTCGTGAGCGGCAACTGCGACGTGGTGATCGGCATCGTGAACAACACC
GTGTACGACCCCCTGCAGCCCGAGCTGGACAGCTTCAAGGAGGAGCTGGACAAGTACTTCA
AGAACCACACCAGCCCCGACGTGGACCTGGGCGACATCAGCGGCATCAACGCCAGCGTGG
TGAACATCCAGAAGGAGATCGACCGGCTGAACGAGGTGGCCAAGAACCTGAACGAGAGCC
TGATCGACCTGCAGGAGCTGGGCAAGTACGAGCAGTACATCAAGTGGCCCTGGTACATCTG
GCTGGGCTTCATCGCCGGCCTGATCGCCATCGTGATGGTGACCATCATGCTGTGCTGCATG
ACCAGCTGCTGCAGCTGCCTGAAGGGCTGCTGCAGCTGCGGCAGCTGCTGCAAGTTCGACG
AGGACGACAGCGAGCCCGTGCTGAAGGGCGTGAAGCTGCACTACACCTGATAATAGGCTG
GAGCCTCGGTGGCCATGCTTCTTGCCCCTTGGGCCTCCCCCCAGCCCCTCCTCCCCTTCCTG
CACCCGTACCCCCGTGGTCTTTGAATAAAGTCTGAGTGGGCGGCAAAAAAAAAAAAAAAA
AAAAAAAAAAAAAAAGCATATGACTAAAAAAAAAAAAAAAAAAAAAAAAAAAAAAAAA
AAAAAAAAAAAAAAAAAAAAAAAAAAAAAAAAA

SEQ ID NO.10 (SF-3-BA.5 codon optimization sequence)

GGGAGACCCAAGCTGGCTAGCGTTTAAACTTAAGCTTGGTACCGAGCTCGGATCCAC
TAGTCCAGTGTGGTGGAATTCGGGAAATAAGAGAGAAAAGAAGAGTAAGAAGAAATATAA
GAGCCACCATGTTCGTTTTTTTAGTTTTATTACCTTTAGTGAGCAGCCAGTGCGTGAACCTG
ATCACCCGGACCCAGAGCTACACCAACAGCTTCACCCGGGGCGTGTACTACCCCGACAAGG
TGTTCCGGAGCAGCGTGCTGCACAGCACCCAGGACCTGTTCCTGCCCTTCTTCAGCAACGT
GACCTGGTTCCACGCCATCAGCGGCACCAACGGCACCAAGCGGTTCGACAACCCCGTGCTG
CCCTTCAACGACGGCGTGTACTTCGCCAGCACCGAGAAGAGCAACATCATCCGGGGCTGGA
TCTTCGGCACCACCCTGGACAGCAAGACCCAGAGCCTGCTGATCGTGAACAACGCCACCAA

CGTGGTGATCAAGGTGTGCGAGTTCCAGTTCTGCAACGACCCCTTCCTGGACGTGTACTAC
CACAAGAACAACAAGAGCTGGATGGAGAGCGAGTTCCGGGTGTACAGCAGCGCCAACAAC
TGCACCTTCGAGTACGTGAGCCAGCCCTTCCTGATGGACCTGGAGGGCAAGCAGGGCAACT
TCAAGAACCTGCGGGAGTTCGTGTTCAAGAACATCGACGGCTACTTCAAGATCTACAGCAA
GCACACCCCCATCAACCTGGGCCGGGACCTGCCCCAGGGCTTCAGCGCCCTGGAGCCCCTG
GTGGACCTGCCCATCGGCATCAACATCACCCGGTTCCAGACCCTGCTGGCCCTGCACCGGA
GCTACCTGACCCCCGGCGACAGCAGCAGCGGCTGGACCGCCGGCGCCGCCGCCTACTACGT
GGGCTACCTGCAGCCCCGGACCTTCCTGCTGAAGTACAACGAGAACGGCACCATCACCGAC
GCCGTGGACTGCGCCCTGGACCCCCTGAGCGAGACCAAGTGCACCCTGAAGAGCTTCACCG
TGGAGAAGGGCATCTACCAGACCAGCAACTTCCGGGTGCAGCCCACCGAGAGCATCGTGC
GGTTCCCCAACATCACCAACCTGTGCCCCTTCGACGAGGTGTTCAACGCCACCCGGTTCGC
CAGCGTGTACGCCTGGAACCGGAAGCGGATCAGCAACTGCGTGGCCGACTACAGCGTGCT
GTACAACTTCGCCCCCTTCTTCGCCTTCAAGTGCTACGGCGTGAGCCCCACCAAGCTGAAC
GACCTGTGCTTCACCAACGTGTACGCCGACAGCTTCGTGATCCGGGGCAACGAGGTGAGCC
AGATCGCCCCCGGCCAGACCGGCAACATCGCCGACTACAACTACAAGCTGCCCGACGACTT
CACCGGCTGCGTGATCGCCTGGAACAGCAACAAGCTGGACAGCAAGGTGGGCGGCAACTA
CAACTACCGGTACCGGCTGTTCCGGAAGAGCAACCTGAAGCCCTTCGAGCGGGACATCAGC
ACCGAGATCTACCAGGCCGGCAACAAGCCCTGCAACGGCGTGGCCGGCGTGAACTGCTAC
TTCCCCCTGCAGAGCTACGGCTTCCGGCCCACCTACGGCGTGGGCCACCAGCCCTACCGGG
TGGTGGTGCTGAGCTTCGAGCTGCTGCACGCCCCCGCCACCGTGTGCGGCCCCAAGAAGAG
CACCAACCTGGTGAAGAACAAGTGCGTGAACTTCAACTTCAACGGCCTGACCGGCACCGGC
GTGCTGACCGAGAGCAACAAGAAGTTCCTGCCCTTCCAGCAGTTCGGCCGGGACATCGCCG
ACACCACCGACGCCGTGCGGGACCCCCAGACCCTGGAGATCCTGGACATCACCCCCTGCAG
CTTCGGCGGCGTGAGCGTGATCACCCCCGGCACCAACACCAGCAACCAGGTGGCCGTGCTG
TACCAGGGCGTGAACTGCACCGAGGTGCCCGTGGCCATCCACGCCGACCAGCTGACCCCCA
CCTGCGGGTGTACAGCACCGGCAGCAACGTGTTCCAGACCCGGGCCGGCTGCCTGATCGG
CGCCGAGTACGTGAACAACAGCTACGAGTGCGACATCCCCATCGGCCGCCGGCATCTGCGCC
AGCTACCAGACCCAGACCAAGAGCCACCGGCGGGCCCGGAGCGTGGCCAGCCAGAGCATC
ATCGCCTACACCATGAGCCTGGGCGCCGAGAACAGCGTGGCCTACAGCAACAACAGCATC
GCCATCCCCACCAACTTCACCATCAGCGTGACCACCGAGATCCTGCCCGTGAGCATGACCA
AGACCAGCGTGGACTGCACCATGTACATCTGCGGCGACAGCACCGAGTGCTCCAACCTGCT
GCTGCAGTACGGCAGCTTCTGCACCCAGCTGAAGCGGGCCCTGACCGGCATCGCCGTGGAG
CAGGACAAGAACACCCAGGAGGTGTTCGCCCAGGTGAAGCAGATCTACAAGACCCCCCCC
ATCAAGTACTTCGGCGGCTTCAACTTCAGCCAGATCCTGCCCGACCCCAGCAAGCCCAGCA
AGCGCAGCTTCATCGAGGACCTGCTGTTCAACAAGGTGACCCTGGCCGACGCCGGCTTCAT
CAAGCAGTACGGCGACTGCCTGGGCGACATCGCCGCCCGGGACCTGATCTGCGCCCAGAA
GTTCAACGGCCTGACCGTGCTGCCCCCCCTGCTGACCGACGAGATGATCGCCCAGTACACC
AGCGCCCTGCTGGCCGGCACCATCACCAGCGGCTGGACCTTCGGCGCCGGCGCCGCCCTGC
AGATCCCCTTCGCCATGCAGATGGCCTACCGGTTCAACGGCATCGGCGTGACCCAGAACGT
GCTGTACGAGAACCAGAAGCTGATCGCCAACCAGTTCAACAGCGCCATCGGCAAGATCCA
GGACAGCCTGAGCAGCACCGCCAGCGCCCTGGGCAAGCTGCAGGACGTGGTGAACCACAA
CGCCCAGGCCCTGAACACCCTGGTGAAGCAGCTGAGCAGCAAGTTCGGCGCCATCAGCAG
CGTGCTGAACGACATCCTGAGCCGGCTGGACCCCCCCGAGGCCGAGGTGCAGATCGACCG
GCTGATCACCGGCCGGCTGCAGAGCCTGCAGACCTACGTGACCCAGCAGCTGATCCGGGCC
GCCGAGATCCGGGCCAGCGCCAACCTGGCCGCCACCAAGATGAGCGAGTGCGTGCTGGGC
CAGAGCAAGCGGGTGGACTTCTGCGGCAAGGGCTACCACCTGATGAGCTTCCCCCAGAGC
GCCCCCCACGGCGTGGTGTTCCTGCACGTGACCTACGTGCCCGCCCAGGAGAAGAACTTCA
CCACCGCCCCCGCCATCTGCCACGACGGCAAGGCCCACTTCCCCCGGGAGGGCGTGTTCGT
GAGCAACGGCACCCACTGGTTCGTGACCCAGCGGAACTTCTACGAGCCCCAGATCATCACC
ACCGACAACACCTTCGTGAGCGGCAACTGCGACGTGGTGATCGGCATCGTGAACAACACC
GTGTACGACCCCCTGCAGCCCGAGCTGGACAGCTTCAAGGAGGAGCTGGACAAGTACTTCA
AGAACCACACCAGCCCCGACGTGGACCTGGGCGACATCAGCGGCATCAACGCCAGCGTGG
TGAACATCCAGAAGGAGATCGACCGGCTGAACGAGGTGGCCAAGAACCTGAACGAGAGCC
TGATCGACCTGCAGGAGCTGGGCAAGTACGAGCAGTACATCAAGTGGCCCTGGTACATCTG
GCTGGGCTTCATCGCCGGCCTGATCGCCATCGTGATGGTGACCATCATGCTGTGCTGCATG
ACCAGCTGCTGCAGCTGCCTGAAGGGCTGCTGCAGCTGCGGCAGCTGCTGCAAGTTCGACG
AGGACGACAGCGAGCCCGTGCTGAAGGGCGTGAAGCTGCACTACACCTGATAATAGGCTG
GAGCCTCGGTGGCCATGCTTCTTGCCCCTTGGGCCTCCCCCCAGCCCCTCCTCCCCTTCCTG
CACCCGTACCCCCGTGGTCTTTGAATAAAGTCTGAGTGGGCGGCAAAAAAAAAAAAAAAA
AAAAAAAAAAAAAAAGCATATGACTAAAAAAAAAAAAAAAAAAAAAAAAAAAAAAAAA
AAAAAAAAAAAAAAAAAAAAAAAAAAAAAAAAAAAA

SEQ ID NO.11 (SF-4-BA.5 codon optimization sequence)

GGGAGACCCAAGCTGGCTAGCGTTTAAACTTAAGCTTGGTACCGAGCTCGGATCCAC
TAGTCCAGTGTGGTGGAATTCAGAATAAACTAGTATTCTTCTGGTCCCCACAGACTCAGAG
AGAACCCGCCACCATGTTCGTTTTTTTAGTTTTATTACCTTTAGTGAGCAGCCAGTGCGTGA
ACCTGATCACCCGGACCCAGAGCTACACCAACAGCTTCACCCGGGGGCGTGTACTACCCCGA
CAAGGTGTTCCGGAGCAGCGTGCTGCACAGCACCCAGGACCTGTTCCTGCCCTTCTTCAGC
AACGTGACCTGGTTCCACGCCATCAGCGGCACCAACGGCACCAAGCGGTTCGACAACCCCG
TGCTGCCCTTCAACGACGGCGTGTACTTCGCCAGCACCGAGAAGAGCAACATCATCCGGGGG
CTGGATCTTCGGCACCACCCTGGACAGCAAGACCCAGAGCCTGCTGATCGTGAACAACGCC

ACCAACGTGGTGATCAAGGTGTGCGAGTTCCAGTTCTGCAACGACCCCTTCCTGGACGTGT
ACTACCACAAGAACAACAAGTCCTGGATGGAGAGCGAGTTCCGGGTGTACAGCAGCGCCA
ACAACTGCACCTTCGAGTACGTGAGCCAGCCCTTCCTGATGGACCTGGAGGGCAAGCAGGG
CAACTTCAAGAACCTGCGGGAGTTCGTGTTCAAGAACATCGACGGCTACTTCAAGATCTAC
AGCAAGCACACCCCCATCAACCTGGGCCGGGACCTGCCCCAGGGCTTCAGCGCCCTGGAGC
CCCTGGTGGACCTGCCCATCGGCATCAACATCACCCGGTTCCAGACCCTGCTGGCCCTGCA
CCGGAGCTACCTGACCCCCGGCGACAGCAGCAGCGGCTGGACCGCCGGCGCCGCCGCCTA
CTACGTGGGCTACCTGCAGCCCCGGACCTTCCTGCTGAAGTACAACGAGAACGGCACCATC
ACCGACGCCGTGGACTGCGCCCTGGACCCCCTGAGCGAGACCAAGTGCACCCTGAAGAGC
TTCACCGTGGAGAAGGGCATCTACCAGACCAGCAACTTCCGGGTGCAGCCCACCGAGAGC
ATCGTGCGGTTCCCCAACATCACCAACCTGTGCCCCTTCGACGAGGTGTTCAACGCCACCC
GGTTCGCCAGCGTGTACGCCTGGAACCGGAAGCGGATCAGCAACTGCGTGGCCGACTACA
GCGTGCTGTACAACTTCGCCCCCTTCTTCGCCTTCAAGTGCTACGGCGTGAGCCCCACCAAG
CTGAACGACCTGTGCTTCACCAACGTGTACGCCGACAGCTTCGTGATCCGGGGCAACGAGG
TGAGCCAGATCGCCCCCGGCCAGACCGGCAACATCGCCGACTACAACTACAAGCTGCCCG
ACGACTTCACCGGCTGCGTGATCGCCTGGAACAGCAACAAGCTGGACAGCAAGGTGGGCG
GCAACTACAACTACCGGTACCGGCTGTTCCGGAAGAGCAACCTGAAGCCCTTCGAGCGGG
ACATCAGCACCGAGATCTACCAGGCCGGCAACAAGCCCTGCAACGGCGTGGCCGGCGTGA
ACTGCTACTTCCCCCTGCAGAGCTACGGCTTCCGGCCCACCTACGGCGTGGGGCCACCAGCC
CTACCGGGTGGTGGTGCTGAGCTTCGAGCTGCTGCACGCCCCCGCCACCGTGTGCGGCCCC
AAGAAGAGCACCAACCTGGTGAAGAACAAGTGCGTGAACTTCAACTTCAACGGCCTGACC
GGCACCGGCGTGCTGACCGAGAGCAACAAGAAGTTCCTGCCCTTCCAGCAGTTCGGCCGGG
ACATCGCCGACACCACCGACGCCGTGCGGGACCCCCAGACCCTGGAGATCCTGGACATCAC
CCCCTGCAGCTTCGGCGGCGTGTCCGTGATCACCCCCGGCACCAACACCAGCAACCAGGTG
GCCGTGCTGTACCAGGGCGTGAACTGCACCGAGGTGCCCGTGGCCATCCACGCCGACCAGC
TGACCCCCACCTGGCGGGTGTACAGCACCGGCAGCAACGTGTTCCAGACCCGGGCCGGCTG
CCTGATCGGCGCCGAGTACGTGAACAACAGCTACGAGTGCGACATCCCCATCGGCGCCGGC
ATCTGCGCCAGCTACCAGACCCAGACCAAGAGCCACCGGCGGGCCCGGAGCGTGGCCAGC
CAGAGCATCATCGCCTACACCATGAGCCTGGGCGCCGAGAACAGCGTGGCCTACAGCAAC
AACAGCATCGCCATCCCCACCAACTTCACCATCAGCGTGACCACCGAGATCCTGCCCGTGA
GCATGACCAAGACCAGCGTGGACTGCACCATGTACATCTGCGGCGACAGCACCGAGTGCA
GCAACCTGCTGCTGCAGTACGGCAGCTTCTGCACCCAGCTGAAGCGGGCCCTGACCGGCAT
CGCCGTGGAGCAGGACAAGAACACCCAGGAGGTGTTCGCCCAGGTGAAGCAGATCTACAA
GACCCCCCCCATCAAGTACTTCGGCGGCTTCAACTTCAGCCAGATCCTGCCCGACCCCAGC
AAGCCCAGCAAGCGGAGCTTCATCGAGGACCTGCTGTTCAACAAGGTGACCCTGGCCGAC
GCCGGCTTCATCAAGCAGTACGGCGACTGCCTGGGCGACATCGCCGCCCGGGACCTGATCT
GCGCCCAGAAGTTCAACGGCCTGACCGTGCTGCCCCCCCTGCTGACCGACGAGATGATCGC
CCAGTACACCAGCGCCCTGCTGGCCGGCACCATCACCAGCGGCTGGACCTTCGGCGCCGGC
GCCGCCCTGCAGATCCCCTTCGCCATGCAGATGGCCTACCGGTTCAACGGCATCGGCGTGA
CCCAGAACGTGCTGTACGAGAACCAGAAGCTGATCGCCAACCAGTTCAACAGCGCCATCG
GCAAGATCCAGGACAGCCTGAGCAGCACCGCCAGCGCCCTGGGCAAGCTGCAGGACGTGG
TGAACCACAACGCCCAGGCCCTGAACACCCTGGTGAAGCAGCTGAGCAGCAAGTTCGGCG
CCATCAGCAGCGTGCTGAACGACATCCTGAGCCGGCTGGACCCCCCCGAGGCCGAGGTGC
AGATCGACCGGCTGATCACCGGCCGGCTGCAGAGCCTGCAGACCTACGTGACCCAGCAGCT
GATCCGGGCCGCCGAGATCCGGGCCAGCGCCAACCTGGCCGCCACCAAGATGAGCGAGTG
CGTGCTGGGCCAGAGCAAGCGGGTGGACTTCTGCGGCAAGGGCTACCACCTGATGAGCTTC
CCCCAGAGCGCCCCCCACGGCGTGGTGTTCCTGCACGTGACCTACGTGCCCGCCCAGGAGA
AGAACTTCACCACCGCCCCCGCCATCTGCCACGACGGCAAGGCCCACTTCCCCCGGGAGGG
CGTGTTCGTGAGCAACGGCACCCACTGGTTCGTGACCCAGCGGAACTTCTACGAGCCCCAG
ATCATCACCACCGACAACACCTTCGTGAGCGGCAACTGCGACGTGGTGATCGGCATCGTGA
ACAACACCGTGTACGACCCCCTGCAGCCCGAGCTGGACAGCTTCAAGGAGGAGCTGGACA
AGTACTTCAAGAACCACACCAGCCCCGACGTGGACCTGGGCGACATCAGCGGCATCAACG
CCAGCGTGGTGAACATCCAGAAGGAGATCGACCGGCTGAACGAGGTGGCCAAGAACCTGA
ACGAGAGCCTGATCGACCTGCAGGAGCTGGGCAAGTACGAGCAGTACATCAAGTGGCCCT
GGTACATCTGGCTGGGCTTCATCGCCGGCCTGATCGCCATCGTGATGGTGACCATCATGCT
GTGCTGCATGACCAGCTGCTGCAGCTGCCTGAAGGGCTGCTGCAGCTGCGGCAGCTGCTGC
AAGTTCGACGAGGACGACAGCGAGCCCGTGCTGAAGGGCGTGAAGCTGCACTACACCTGA
TGACTCGAGCTGGTACTGCATGCACGCAATGCTAGCTGCCCCTTTCCCGTCCTGGGTACCCC
GAGTCTCCCCGACCTCGGGTCCCAGGTATGCTCCCACCTCCACCTGCCCCACTCACCACCT
CTGCTAGTTCCAGACACCTCCCAAGCACGCAGCAATGCAGCTCAAAACGCTTAGCCTAGCC
ACACCCCCACGGGAAACAGCAGTGATTAACCTTTAGCAATAAACGAAAGTTTAACTAAGCT
ATACTAACCCCAGGGTTGGTCAATTTCGTGCCAGCCACACCCTGGAGCTAGCAAAAAAAA
AAAAAAAAAAAAAAAAAAAAAAGCATATGACTAAAAAAAAAAAAAAAAAAAAAAAAAAA
AAAAAAAAAAAAAAAAAAAAAAAAAAAAAAAAAAAAAAAAA

SEQ ID NO.12 (SF-5-BA.5 codon optimization sequence)

GGGAGACCCAAGCTGGCTAGCGTTTAAACTTAAGCTTGGTACCGAGCTCGGATCCAC
TAGTCCAGTGTGGTGGAATTCAGAATAAACTAGTATTCTTCTGGTCCCCACAGACTCAGAG
AGAACCCGCCACCATGTTCGTTTTTTTTAGTTTTATTACCATTAGTGAGCAGCAGTGCGTGA
ACCTGATCACCCGGACCCAGAGCTACACCAACAGCTTCACCCGGGGCGTGTACTACCCCGA
CAAGGTGTTCCGGAGCAGCGTGCTGCACAGCACCCAGGACCTGTTCCTGCCCTTCTTCAGC

AACGTGACCTGGTTCCACGCCATCAGCGGCACCAACGGCACCAAGCGGTTCGACAACCCCG
TGCTGCCCTTCAACGACGGCGTGTACTTCGCCAGCACCGAGAAGAGCAACATCATCCGGGG
CTGGATCTTCGGCACCACCCTGGACAGCAAGACCCAGAGCCTGCTGATCGTGAACAACGCC
ACCAACGTGGTGATCAAGGTGTGCGAGTTCCAGTTCTGCAACGACCCCTTCCTGGACGTGT
ACTACCACAAGAACAACAAGAGCTGGATGGAGAGCGAGTTCCGGGTGTACAGCAGCGCCA
ACAACTGCACCTTCGAGTACGTGAGCCAGCCCTTCCTGATGGACCTGGAGGGCAAGCAGGG
CAACTTCAAGAACCTGCGGGAGTTCGTGTTCAAGAACATCGACGGCTACTTCAAGATCTAC
AGCAAGCACACCCCCATCAACCTGGGCCGGGACCTGCCCCAGGGCTTCAGCGCCCTGGAGC
CCCTGGTGGACCTGCCCATCGGCATCAACATCACCCGGTTCCAGACCCTGCTGGCCCTGCA
CCGGAGCTACCTGACCCCCGGCGACAGCAGCAGCGGCTGGACCGCCGGCGCCGCCGCCTA
CTACGTGGGCTACCTGCAGCCCCGGACCTTCCTGCTGAAGTACAACGAGAACGGCACCATC
ACCGACGCCGTGGACTGCGCCCTGGACCCCCTGAGCGAGACCAAGTGCACCCTGAAGAGC
TTCACCGTGGAGAAGGGCATCTACCAGACCAGCAACTTCCGGGTGCAGCCCACCGAGAGC
ATCGTGCGGTTCCCCAACATCACCAACCTGTGCCCCTTCGACGAGGTGTTCAACGCCACCC
GGTTCGCCAGCGTGTACGCCTGGAACCGGAAGCGGATCAGCAACTGCGTGGCCGACTACA
GCGTGCTGTACAACTTCGCCCCCTTCTTCGCCTTCAAGTGCTACGGCGTGAGCCCCACCAAG
CTGAACGACCTGTGCTTCACCAACGTGTACGCCGACAGCTTCGTGATCCGGGGCAACGAGG
TGAGCCAGATCGCCCCCGGCCAGACCGGCAACATCGCCGACTACAACTACAAGCTGCCCG
ACGACTTCACCGGCTGCGTGATCGCCTGGAACAGCAACAAGCTGGACAGCAAGGTGGGCG
GCAACTACAACTACCGGTACCGGCTGTTCCGGAAGAGCAACCTGAAGCCCTTCGAGCGGG
ACATCAGCACCGAGATCTACCAGGCCGGCAACAAGCCCTGCAACGGCGTGGCCGGCGTGA
ACTGCTACTTCCCCCTGCAGAGCTACGGCTTCCGGCCCACCTACGGCGTGGGCCACCAGCC
CTACCGGGTGGTGGTGCTGAGCTTCGAGCTGCTGCACGCCCCCGCCACCGTGTGCGGCCCC
AAGAAGAGCACCAACCTGGTGAAGAACAAGTGCGTGAACTTCAACTTCAACGGCCTGACC
GGCACCGGCGTGCTGACCGAGAGCAACAAGAAGTTCCTGCCCTTCCAGCAGTTCGGCCGGG
ACATCGCCGACACCACCGACGCCGTGCGGGACCCCCAGACCCTGGAGATCCTGGACATCAC
CCCCTGCAGCTTCGGCGGCGTGAGCGTGATCACCCCCGGCACCAACACCAGCAACCAGGTG
GCCGTGCTGTACCAGGGCGTGAACTGCACCGAGGTGCCCGTGGCCATCCACGCCGACCAGC
TGACCCCCACCTGGCGGGTGTACAGCACCGGCAGCAACGTGTTCCAGACCCGGGCCGGCTG
CCTGATCGGCGCCGAGTACGTGAACAACAGCTACGAGTGCGACATCCCCATCGGCGCCGGC
ATCTGCGCCAGCTACCAGACCCAGACCAAGAGCCACCGGCGGGCCCGGAGCGTGGCCAGC
CAGAGCATCATCGCCTACACCATGAGCCTGGGCGCCGAGAACAGCGTGGCCTACAGCAAC
AACAGCATCGCCATCCCCACCAACTTCACCATCAGCGTGACCACCGAGATCCTGCCCGTGA
GCATGACCAAGACCAGCGTGGACTGCACCATGTACATCTGCGGCGACAGCACCGAGTGCA
GCAACCTGCTGCTGCAGTACGGCAGCTTCTGCACCCAGCTGAAGCGGGCCCTGACCGGCAT
CGCCGTGGAGCAGGACAAGAACACCCAGGAGGTGTTCGCCCAGGTGAAGCAGATCTACAA
GACCCCCCCCATCAAGTACTTCGGCGGCTTCAACTTCAGCCAGATCCTGCCCGACCCCAGC
AAGCCCAGCAAGCGGAGCTTCATCGAGGACCTGCTGTTCAACAAGGTGACCCTGGCCGAC
GCCGGCTTCATCAAGCAGTACGGCGACTGCCTGGGCGACATCGCCGCCCGGGACCTGATCT
GCGCCCAGAAGTTCAACGGCCTGACCGTGCTGCCCCCCCTGCTGACCGACGAGATGATCGC
CCAGTACACCAGCGCCCTGCTGGCCGGCACCATCACCAGCGGCTGGACCTTCGGCGCCGGC
GCCGCCCTGCAGATCCCCTTCGCCATGCAGATGGCCTACCGGTTCAACGGCATCGGCGTGA
CCCAGAACGTGCTGTACGAGAACCAGAAGCTGATCGCCAACCAGTTCAACAGCGCCATCG
GCAAGATCCAGGACAGCCTGAGCAGCACCGCCAGCGCCCTGGGCAAGCTGCAGGACGTGG
TGAACCACAACGCCCAGGCCCTGAACACCCTGGTGAAGCAGCTGAGCAGCAAGTTCGGCG
CCATCAGCAGCGTGCTGAACGACATCCTGAGCCGGCTGGACCCCCCCGAGGCCGAGGTGC
AGATCGACCGGCTGATCACCGGCCGGCTGCAGAGCCTGCAGACCTACGTGACCCAGCAGCT
GATCCGGGCCGCCGAGATCCGGGCCAGCGCCAACCTGGCCGCCACCAAGATGAGCGAGTG
CGTGCTGGGCCAGAGCAAGCGGGTGGACTTCTGCGGCAAGGGCTACCACCTGATGTCCTTC
CCCCAGAGCGCCCCCCACGGCGTGGTGTTCCTGCACGTGACCTACGTGCCCGCCCAGGAGA
AGAACTTCACCACCGCCCCCGCCATCTGCCACGACGGCAAGGCCCACTTCCCCCGGGAGGG
CGTGTTCGTGAGCAACGGCACCCACTGGTTCGTGACCCAGCGGAACTTCTACGAGCCCCAG
ATCATCACCACCGACAACACCTTCGTGAGCGGCAACTGCGACGTGGTGATCGGCATCGTGA
ACAACACCGTGTACGACCCCCTGCAGCCCGAGCTGGACAGCTTCAAGGAGGAGCTGGACA
AGTACTTCAAGAACCACACCAGCCCCGACGTGGACCTGGGCGACATCAGCGGCATCAACG
CCAGCGTGGTGAACATCCAGAAGGAGATCGACCGGCTGAACGAGGTGGCCAAGAACCTGA
ACGAGAGCCTGATCGACCTGCAGGAGCTGGGCAAGTACGAGCAGTACATCAAGTGGCCCT
GGTACATCTGGCTGGGCTTCATCGCCGGCCTGATCGCCATCGTGATGGTGACCATCATGCT
GTGCTGCATGACCAGCTGCTGCAGCTGCCTGAAGGGCTGCTGCAGCTGCGGCAGCTGCTGC
AAGTTCGACGAGGACGACAGCGAGCCCGTGCTGAAGGGCGTGAAGCTGCACTACACCTGA
TGACTCGAGCTGGTACTGCATGCACGCAATGCTAGCTGCCCCTTTCCGTCCTGGGTACCCC
GAGTCTCCCCCGACCTCGGGTCCCAGGTATGCTCCCACCTCCACCTGCCCCACTCACCACCT
CTGCTAGTTCCAGACACCTCCCAAGCACGCAGCAATGCAGCTCAAAACGCTTAGCCTAGCC
ACACCCCCACGGGAAACAGCAGTGATTAACCTTTAGCAATAAACGAAAGTTTAACTAAGCT
ATACTAACCCCAGGGTTGGTCAATTTCGTGCCAGCCACACCCTGGAGCTAGCAAAAAAAAA
AAAAAAAAAAAAAAAAAAAAAGCATATGACTAAAAAAAAAAAAAAAAAAAAAAAAAAAA
AAAAAAAAAAAAAAAAAAAAAAAAAAAAAAAAAAAAAAA

SEQ ID NO.13 (SF-6-XBB-1 codon optimization sequence)

GGGAGACCCAAGCTGGCTAGCGTTTAAACTTAAGCTTGGTACCGAGCTCGGATCCAC
TAGTCCAGTGTGGTGGAATTCAGAATAAACTAGTATTCTTCTGGTCCCCACAGACTCAGAG

AGAACCCGCCACCATGTTCGTTTTTTTAGTTTTATTACCTTTAGTGAGCTCCCAGTGCGTGA
ACCTGATCACCCGGACCCAGAGCTACACCAACAGCTTCACCCGGGGCGTGTACTACCCCGA
CAAGGTGTTCCGGAGCAGCGTGCTGCACAGCACCCAGGACCTGTTCCTGCCCTTCTTCAGC
AACGTGACCTGGTTCCACGCCATCCACGTGAGCGGCACCAACGGCACCAAGCGGTTCGACA
ACCCCGCCCTGCCCTTCAACGACGGCGTGTACTTCGCCAGCACCGAGAAGAGCAACATCAT
CCGGGGCTGGATCTTCGGCACCACCCTGGACAGCAAGACCCAGAGCCTGCTGATCGTGAAC
AACGCCACCAACGTGGTGATCAAGGTGTGCGAGTTCCAGTTCTGCAACGACCCCTTCCTGG
ACGTGTACCAGAAGAACAACAAGAGCTGGATGGAGAGCGAGTTCCGGGTGTACAGCAGCG
CCAACAACTGCACCTTCGAGTACGTGAGCCAGCCCTTCCTGATGGACTGGAGGGCAAGGA
GGGCAACTTCAAGAACCTGCGGGAGTTCGTGTTCAAGAACATCGACGGCTACTTCAAGATC
TACAGCAAGCACACCCCCATCAACCTGGAGCGGGACCTGCCCCAGGGCTTCAGCGCCCTG
AGCCCCTGGTGGACCTGCCCATCGGCATCAACATCACCCGGTTCCAGACCCTGCTGGCCCT
GCACCGGAGCTACCTGACCCCCGTGGACAGCAGCAGCGGCTGGACCGCCGGCGCCGCCGC
CTACTACGTGGGCTACCTGCAGCCCCGGACCTTCCTGCTGAAGTACAACGAGAACGGCACC
ATCACCGACGCCGTGGACTGCGCCCTGGACCCCCTGTCCGAGACCAAGTGCACCCTGAAGA
GCTTCACCGTGGAGAAGGGCATCTACCAGACCAGCAACTTCCGGGTGCAGCCCACCGAGA
GCATCGTGCGGTTCCCCAACATCACCAACCTGTGCCCCTTCCACGAGGTGTTCAACGCCAC
CACCTTCGCCAGCGTGTACGCCTGGAACCGGAAGCGGATCAGCAACTGCGTGGCCGACTAC
AGCGTGATCTACAACTTCGCCCCCTTCTTCGCCTTCAAGTGCTACGGCGTGAGCCCCACCAA
GCTGAACGACCTGTGCTTCACCAACGTGTACGCCGACAGCTTCGTGATCCGGGGCAACGAG
GTGAGCCAGATCGCCCCCGGCCAGACCGGCAACATCGCCGACTACAACTACAAGCTGCCC
GACGACTTCACCGGCTGCGTGATCGCCTGGAACAGCAACAAGCTGGACAGCAAGCCCAGC
GGCAACTACAACTACCTGTACCGGCTGTTCCGGAAGAGCAAGCTGAAGCCCTTCGAGCGGG
ACATCAGCACCGAGATCTACCAGGCCGGCAACAAGCCCTGCAACGGCGTGGCCGGCAGCA
ACTGCTACAGCCCCCTGCGGAGCTACGGCTTCCGGCCCACCTACGGCGTGGGCCACCAGCC
CTACCGGGTGGTGGTGCTGAGCTTCGAGCTGCTGCACGCCCCCGCCACCGTGTGCGGCCCC
AAGAAGAGCACCAACCTGGTGAAGAACAAGTGCGTGAACTTCAACTTCAACGGCCTGACC
GGCACCGGCGTGCTGACCGAGAGCAACAAGAAGTTCCTGCCCTTCCAGCAGTTCGGCCGGG
ACATCGCCGACACCACCGACGCCGTGCGGGACCCCCAGACCCTGGAGATCCTGGACATCAC
CCCCTGCAGCTTCGGCGGCGTGAGCGTGATCACCCCCGGCACCAACACCAGCAACCAGGTG
GCCGTGCTGTACCAGGGCGTGAACTGCACCGAGGTGCCCGTGGCCATCCACGCCGACCAGC
TGACCCCCACCTGGCGGGTGTACAGCACCGGCAGCAACGTGTTCCAGACCCGGGCCGGCTG
CCTGATCGGCGCCGAGTACGTGAACAACAGCTACGAGTGCGACATCCCCATCGGCGCCGGC
ATCTGCGCCAGCTACCAGACCCAGACCAAGAGCCACCGGCGGGCCCGGAGCGTGGCCAGC
CAGAGCATCATCGCCTACACCATGAGCCTGGGCGCCGAGAACAGCGTGGCCTACAGCAAC
AACAGCATCGCCATCCCCACCAACTTCACCATCAGCGTGACCACCGAGATCCTGCCCGTGA
GCATGACCAAGACCAGCGTGGACTGCACCATGTACATCTGCGGCGACAGCACCGAGTGCA
GCAACCTGCTGCTGCAGTACGGCAGCTTCTGCACCCAGCTGAAGCGGGCCCTGACCGGCAT
CGCCGTGGAGCAGGACAAGAACACCCAGGAGGTGTTCGCCCAGGTGAAGCAGATCTACAA
GACCCCCCCCATCAAGTACTTCGGCGGCTTCAACTTCAGCCAGATCCTGCCCGACCCCAGC
AAGCCCAGCAAGCGGAGCTTCATCGAGGACCTGCTGTTCAACAAGGTGACCCTGGCCGAC
GCCGGCTTCATCAAGCAGTACGGCGACTGCCTGGGCGACATCGCCGCCCGGGACCTGATCT
GCGCCCAGAAGTTCAACGGCCTGACCGTGCTGCCCCCCCTGCTGACCGACGAGATGATCGC
CCAGTACACCAGCGCCCTGCTGGCCGGCACCATCACCAGCGGCTGGACCTTCGGCGCCGGC
GCCGCCCTGCAGATCCCCTTCGCCATGCAGATGGCCTACCGGTTCAACGGCATCGGCGTGA
CCCAGAACGTGCTGTACGAGAACCAGAAGCTGATCGCCAACCAGTTCAACAGCGCCATCG
GCAAGATCCAGGACAGCCTGAGCAGCACCGCCAGCGCCCTGGGCAAGCTGCAGGACGTGG
TGAACCACAACGCCCAGGCCCTGAACACCCTGGTGAAGCAGCTGAGCAGCAAGTTCGGCG
CCATCAGCAGCGTGCTGAACGACATCCTGAGCCGGCTGGACCCCCCCGAGGCCGAGGTGC
AGATCGACCGGCTGATCACCGGCCGGCTGCAGAGCCTGCAGACCTACGTGACCCAGCAGCT
GATCCGGGCCGCCGAGATCCGGGCCAGCGCCAACCTGGCCGCCACCAAGATGAGCGAGTG
CGTGCTGGGCCAGAGCAAGCGGGTGGACTTCTGCGGCAAGGGCTACCACCTGATGAGCTTC
CCCCAGAGCGCCCCCCACGGCGTGGTGTTCCTGCACGTGACCTACGTGCCCGCCCAGGAGA
AGAACTTCACCACCGCCCCCGCCATCTGCCACGACGGCAAGGCCCACTTCCCCCGGGAGGG
CGTGTTCGTGAGCAACGGCACCCACTGGTTCGTGACCCAGCGGAACTTCTACGAGCCCCAG
ATCATCACCACCGACAACACCTTCGTGAGCGGCAACTGCGACGTGGTGATCGGCATCGTGA
ACAACACCGTGTACGACCCCCTGCAGCCCGAGCTGGACAGCTTCAAGGAGGAGCTGGACA
AGTACTTCAAGAACCACACCAGCCCCGACGTGGACCTGGGCGACATCAGCGGCATCAACG
CCAGCGTGGTGAACATCCAGAAGGAGATCGACCGGCTGAACGAGGTGGCCAAGAACCTGA
ACGAGAGCCTGATCGACCTGCAGGAGCTGGGCAAGTACGAGCAGTACATCAAGTGGCCCT
GGTACATCTGGCTGGGCTTCATCGCCGGCCTGATCGCCATCGTGATGGTGACCATCATGCT
GTGCTGCATGACCAGCTGCTGCAGCTGCCTGAAGGGCTGCTGCAGCTGCGGCAGCTGCTGC
AAGTGATGACTCGAGCTGGTACTGCATGCACGCAATGCTAGCTGCCCCTTTCCCGTCCTGG
GTACCCCGAGTCTCCCCCGACCTCGGGTCCCAGGTATGCTCCCACCTCCACCTGCCCCACTC
ACCACCTCTGCTAGTTCCAGACACCTCCCAAGCACGCAGCAATGCAGCTCAAAACGCTTAG
CCTAGCCACACCCCCACGGGAAACAGCAGTGATTAACCTTTAGCAATAAACGAAAGTTTAA
CTAAGCTATACTAACCCCAGGGTTGGTCAATTTCGTGCCAGCCACACCCTGGAGCTAGCAA
AAAAAAAAAAAAAAAAAAAAAAAAAAAAAAAAAGCATATGACTAAAAAAAAAAAAAAAAAAAAAA
AAAAAAAAAAAAAAAAAAAAAAAAAAAAAAAAAAAAAAAAAAAAAAAAAAAAAA

SEQ ID NO.14 (SF-7-XBB-2 codon optimization sequence )

GGGAGACCCAAGCTGGCTAGCGTTTAAACTTAAGCTTGGTACCGAGCTCGGATCCAC
TAGTCCAGTGTGGTGGAATTCAGAATAAACTAGTATTCTTCTGGTCCCCACAGACTCAGAG
AGAACCCGCCACCATGTTCGTTTTTTTTAGTTTTATTACCTTTAGTGAGCAGCCAGTGCGTGA
ACCTGATCACCCGGACCCAGAGCTACACCAACAGCTTCACCCGGGGCGTGTACTACCCCGA
CAAGGTGTTCCGGAGCAGCGTGCTGCACAGCACCCAGGACCTGTTCCTGCCCTTCTTCAGC
AACGTGACCTGGTTCCACGCCATCCACGTGAGCGGCACCAACGGCACCAAGCGGTTCGACA
ACCCCGCCCTGCCCTTCAACGACGGCGTGTACTTCGCCAGCACCGAGAAGAGCAACATCAT
CCGGGGCTGGATCTTCGGCACCACCCTGGACAGCAAGACCCAGAGCCTGCTGATCGTGAAC
AACGCCACCAACGTGGTGATCAAGGTGTGCGAGTTCCAGTTCTGCAACGACCCCTTCCTGG
ACGTGTACCAGAAGAACAACAAGAGCTGGATGGAGAGCGAGTTCCGGGTGTACAGCAGCG
CCAACAACTGCACCTTCGAGTACGTGAGCCAGCCCTTCCTGATGGACCTGGAGGGCAAGGA
GGGCAACTTCAAGAACCTGCGGGAGTTCGTGTTCAAGAACATCGACGGCTACTTCAAGATC
TACAGCAAGCACACCCCCATCAACCTGGAGCGGGACCTGCCCCAGGGCTTCAGCGCCCTGG
AGCCCCTGGTGGACCTGCCCATCGGCATCAACATCACCCGGTTCCAGACCCTGCTGGCCCT
GCACCGGAGCTACCTGACCCCCGTGGACAGCAGCAGCGGCTGGACCGCCGGCGCCGCCGC
CTACTACGTGGGCTACCTGCAGCCCCGGACCTTCCTGCTGAAGTACAACGAGAACGGCACC
ATCACCGACGCCGTGGACTGCGCCCTGGACCCCCTGAGCGAGACCAAGTGCACCCTGAAG
AGCTTCACCGTGGAGAAGGGCATCTACCAGACCAGCAACTTCCGGGTGCAGCCCACCGAG
AGCATCGTGCGCTTCCCCAACATCACCAACCTGTGCCCCTTCCACGAGGTGTTCAACGCCA
CCACCTTCGCCAGCGTGTACGCCTGGAACGGCAAGCGCATCAGCAACTGCGTGGCCGACTA
CAGCGTGATCTACAACTTCGCCCCCTTCTTCGCCTTCAAGTGCTACGGCGTGAGCCCCACCA
AGCTGAACGACCTGTGCTTCACCAACGTGTACGCCGACAGCTTCGTGATCCGGGGCAACGA
GGTGAGCCAGATCGCCCCCGGCCAGACCGGCAACATCGCCGACTACAACTACAAGCTGCC
CGACGACTTCACCGGCTGCGTGATCGCCTGGAACAGCAACAAGCTGGACAGCAAGCCCAG
CGGCAACTACAACTACCTGTACCGGCTGTTCCGGAAGAGCAAGCTGAAGCCCTTCGAGCGG
GACATCAGCACCGAGATCTACCAGGCCGGCAACAAGCCCTGCAACGGCGTGGCCGGCAGC
AACTGCTACAGCCCCCTGCGGAGCTACGGCTTCCGGCCCACCTACGGCGTGGGCCACCAGC
CCTACCGGGTGGTGGTGCTGAGCTTCGAGCTGCTGCACGCCCCCGCCACCGTGTGCGGCCC
CAAGAAGAGCACCAACCTGGTGAAGAACAAGTGCGTGAACTTCAACTTCAACGGCCTGAC
CGGCACCGGCGTGCTGACCGAGAGCAACAAGAAGTTCCTGCCCTTCCAGCAGTTCGGCCGG
GACATCGCCGACACCACCGACGCCGTGCGGGACCCCCAGACCCTGGAGATCCTGGACATC
ACCCCCTGCAGCTTCGGCGGCGTGAGCGTGATCACCCCCGGCACCAACACCAGCAACCAGG
TGGCCGTGCTGTACCAGGGCGTGAACTGCACCGAGGTGCCCGTGGCCATCCACGCCGACCA
GCTGACCCCCACCTGGCGGGTGTACAGCACCGGCAGCAACGTGTTCCAGACCCGGGCCGGC
TGCCTGATCGGCGCCGAGTACGTGAACAACAGCTACGAGTGCGACATCCCCATCGGCGCCG
GCATCTGCGCCAGCTACCAGACCCAGACCAAGAGCCACCGGCGGGCCCGGAGCGTGGCCA
GCCAGAGCATCATCGCCTACACCATGAGCCTGGGCGCCGAGAACAGCGTGGCCTACAGCA
ACAACAGCATCGCCATCCCCACCAACTTCACCATCAGCGTGACCACCGAGATCCTGCCCGT
GAGCATGACCAAGACCAGCGTGGACTGCACCATGTACATCTGCGGCGACAGCACCGAGTG
CAGCAACCTGCTGCTGCAGTACGGCAGCTTCTGCACCCAGCTGAAGCGGGCCCTGACCGGC
ATCGCCGTGGAGCAGGACAAGAACACCCAGGAGGTGTTCGCCCAGGTGAAGCAGATCTAC
AAGACCCCCCCCATCAAGTACTTCGGCGGCTTCAACTTCAGCCAGATCCTGCCCGACCCCA
GCAAGCCCAGCAAGCGGAGCTTCATCGAGGACCTGCTGTTCAACAAGGTGACCCTGGCCG
ACGCCGGCTTCATCAAGCAGTACGGCGACTGCCTGGGCGACATCGCCGCCCGGGACCTGAT
CTGCGCCCAGAAGTTCAACGGCCTGACCGTGCTGCCCCCCCTGCTGACCGACGAGATGATC
GCCCAGTACACCAGCGCCCTGCTGGCCGGCACCATCACCAGCGGCTGGACCTTCGGCGCCG
GCGCCGCCCTGCAGATCCCCTTCGCCATGCAGATGGCCTACCGGTTCAACGGCATCGGCGT
GACCCAGAACGTGCTGTACGAGAACCAGAAGCTGATCGCCAACCAGTTCAACAGCGCCAT
CGGCAAGATCCAGGACAGCCTGAGCAGCACCGCCAGCGCCCTGGGCAAGCTGCAGGACGT
GGTGAACCACAACGCCCAGGCCCTGAACACCCTGGTGAAGCAGCTGAGCAGCAAGTTCGG
CGCCATCAGCAGCGTGCTGAACGACATCCTGAGCCGGCTGGACCCCCCCGAGGCCGAGGT
GCAGATCGACCGGCTGATCACCGGCCGGCTGCAGAGCCTGCAGACCTACGTGACCCAGCA
GCTGATCCGGGCCGCCGAGATCCGGGCCAGCGCCAACCTGGCCGCCACCAAGATGAGCGA
GTGCGTGCTGGGCCAGAGCAAGCGGGTGGACTTCTGCGGCAAGGGCTACCACCTGATGAG
CTTCCCCCAGAGCGCCCCCCACGGCGTGGTGTTCCTGCACGTGACCTACGTGCCCGCCCAG
GAGAAGAACTTCACCACCGCCCCCGCCATCTGCCACGACGGCAAGGCCCACTTCCCCCGGG
AGGGCGTGTTCGTGAGCAACGGCACCCACTGGTTCGTGACCCAGCGGAACTTCTACGAGCC
CCAGATCATCACCACCGACAACACCTTCGTGAGCGGCAACTGCGACGTGGTGATCGGCATC
GTGAACAACACCGTGTACGACCCCCTGCAGCCCGAGCTGGACAGCTTCAAGGAGGAGCTG
GACAAGTACTTCAAGAACCACACCAGCCCCGACGTGGACCTGGGCGACATCAGCGGCATC
AACGCCAGCGTGGTGAACATCCAGAAGGAGATCGACCGGCTGAACGAGGTGGCCAAGAAC
CTGAACGAGAGCCTGATCGACCTGCAGGAGCTGGGCAAGTACGAGCAGTACATCAAGTGG
CCCTGGTACATCTGGCTGGGCTTCATCGCCGGCCTGATCGCCATCGTGATGGTGACCATCAT
GCTGTGCTGCATGACCAGCTGCTGCAGCTGCCTGAAGGGCTGCTGCAGCTGCGGCAGCTGC
TGCAAGTGATGACTCGAGCTGGTACTGCATGCACGCAATGCTAGCTGCCCCTTTCCCGTCCT
GGGTACCCCGAGTCTCCCCCGACCTCGGGTCCCAGGTATGCTCCCACCTCCACCTGCCCCAC
TCACCACCTCTGCTAGTTCCAGACACCTCCCAAGCACGCAGCAATGCAGCTCAAAACGCTT
AGCCTAGCCACACCCCCACGGGAAACAGCAGTGATTAACCTTTAGCAATAAACGAAAGTTT
AACTAAGCTATACTAACCCCAGGGTTGGTCAATTTCGTGCCAGCCACACCCTGGAGCTAGC
AAAAAAAAAAAAAAAAAAAAAAAAAAAAAAAGCATATGACTAAAAAAAAAAAAAAAAAAA

AAAAAAAAAAAAAAAAAAAAAAAAAAAAAAAAAAAAAAAAAAAAAAAAAAAA

SEQ ID NO.15 (SF-S-BA.2.75-1 codon optimization sequence)

GGGAGACCCAAGCTGGCTAGCGTTTAAACTTAAGCTTGGTACCGAGCTCGGATCCAC
TAGTCCAGTGTGGTGGAATTCAGAATAAACTAGTATTCTTCTGGTCCCCACAGACTCAGAG
AGAACCCGCCACCATGTTCGTTTTTTTAGTTTTATTACCTTTAGTGAGCAGCCAGTGCGTGA
ACCTGATCACCCGGACCCAGAGCTACACCAACAGCTTCACCCGGGGCGTGTACTACCCCGA
CAAGGTGTTCCGGAGCAGCGTGCTGCACAGCACCCAGGACCTGTTCCTGCCCTTCTTCAGC
AACGTGACCTGGTTCCACGCCATCCACGTGAGCGGCACCAACGGCACCAAGCGGTTCGACA
ACCCCGTGCTGCCCTTCAACGACGGCGTGTACTTCGCCAGCACCGAGAAGAGCAACATCAT
CCGGGGGCTGGATCTTCGGCACCACCCTGGACAGCAAGACCCAGAGCCTGCTGATCGTGAAC
AACGCCACCAACGTGGTGATCAAGGTGTGCGAGTTCCAGTTCTGCAACGACCCCTTCCTGG
ACGTGTACTACCACGAGAACAACAAGAGCCGGATGGAGAGCGAGCTGCGGGTGTACAGCA
GCGCCAACAACTGCACCTTCGAGTACGTGAGCCAGCCCTTCCTGATGGACCTGGAGGGCAA
GCAGGGCAACTTCAAGAACCTGCGGGAGTTCGTGTTCAAGAACATCGACGGCTACTTCAAG
ATCTACAGCAAGCACACCCCCGTGAACCTGGGCCGGGACCTGCCCCAGGGCTTCAGCGCCC
TGGAGCCCCTGGTGGACCTGCCCATCGGCATCAACATCACCCGGTTCCAGACCCTGCTGGC
CCTGCACCGGAGCTACCTGACCCCCGGCGACAGCAGCAGCAGCTGGACCGCCGGCGCCGC
CGCCTACTACGTGGGCTACCTGCAGCCCCGGACCTTCCTGCTGAAGTACAACGAGAACGGC
ACCATCACCGACGCCGTGGACTGCGCCCTGGACCCCCTGAGCGAGACCAAGTGCACCCTGA
AGAGCTTCACCGTGGAGAAGGGCATCTACCAGACCAGCAACTTCCGGGTGCAGCCCACCG
AGAGCATCGTGCGGTTCCCCAACATCACCAACCTGTGCCCCTTCCACGAGGTGTTCAACGC
CACCCGGTTCGCCTCCGTGTACGCCTGGAACCGGAAGCGGATCAGCAACTGCGTGGCCGAC
TACAGCGTGCTGTACAACTTCGCCCCCTTCTTCGCCTTCAAGTGCTACGGCGTGAGCCCCAC
CAAGCTGAACGACCTGTGCTTCACCAACGTGTACGCCGACAGCTTCGTGATCCGGGGCAAC
GAGGTGAGCCAGATCGCCCCCGGCCAGACCGGCAACATCGCCGACTACAACTACAAGCTG
CCCGACGACTTCACCGGCTGCGTGATCGCCTGGAACAGCAACAAGCTGGACAGCAAGGTG
AGCGGCAACTACAACTACCTGTACCGGCTGTTCCGGAAGAGCAAGCTGAAGCCCTTCGAGC
GGGACATCAGCACCGAGATCTACCAGGCCGGCAACAAGCCCTGCAACGGCGTGGCCGGCT
TCAACTGCTACTTCCCCCTGCGGAGCTACGGCTTCCGGCCCACCTACGGCGTGGGCCACCA
GCCCTACCGGGTGGTGGTGCTGAGCTTCGAGCTGCTGCACGCCCCCGCCACCGTGTGCGGC
CCCAAGAAGAGCACCAACCTGGTGAAGAACAAGTGCGTGAACTTCAACTTCAACGGCCTG
ACCGGCACCGGCGTGCTGACCGAGAGCAACAAGAAGTTCCTGCCCTTCCAGCAGTTCGGCC
GGGACATCGCCGACACCACCGACGCCGTGCGGGACCCCCAGACCCTGGAGATCCTGGACA
TCACCCCCTGCAGCTTCGGCGGCGTGAGCGTGATCACCCCCGGCACCAACACCAGCAACCA
GGTGGCCGTGCTGTACCAGGGCGTGAACTGCACCGAGGTGCCCGTGGCCATCCACGCCGAC
CAGCTGACCCCCACCTGGCGGGTGTACAGCACCGGCAGCAACGTGTTCCAGACCCGGGCCG
GCTGCCTGATCGGCGCCGAGTACGTGAACAACAGCTACGAGTGCGACATCCCCATCGGCGC
CGGCATCTGCGCCAGCTACCAGACCCAGACCAAGAGCCACCGGCGGGCCCGGAGCGTGGC
CAGCCAGAGCATCATCGCCTACACCATGAGCCTGGGCGCCGAGAACAGCGTGGCCTACAG
CAACAACAGCATCGCCATCCCCACCAACTTCACCATCAGCGTGACCACCGAGATCCTGCCC
GTGAGCATGACCAAGACCAGCGTGGACTGCACCATGTACATCTGCGGCGACAGCACCGAG
TGCAGCAACCTGCTGCTGCAGTACGGCAGCTTCTGCACCCAGCTGAAGCGGGCCCTGACCG
GCATCGCCGTGGAGCAGGACAAGAACACCCAGGAGGTGTTCGCCCAGGTGAAGCAGATCT
ACAAGACCCCCCCCATCAAGTACTTCGGCGGCTTCAACTTCAGCCAGATCCTGCCCGACCC
CAGCAAGCCCAGCAAGCGGAGCTTCATCGAGGACCTGCTGTTCAACAAGGTGACCCTGGCC
GACGCCGGCTTCATCAAGCAGTACGGCGACTGCCTGGGCGACATCGCCGCCCGGGACCTGA
TCTGCGCCCAGAAGTTCAACGGCCTGACCGTGCTGCCCCCCCTGCTGACCGACGAGATGAT
CGCCCAGTACACCAGCGCCCTGCTGGCCGGCACCATCACCAGCGGCTGGACCTTCGGCGCC
GGCGCCGCCCTGCAGATCCCCTTCGCCATGCAGATGGCCTACCGGTTCAACGGCATCGGCG
TGACCCAGAACGTGCTGTACGAGAACCAGAAGCTGATCGCCAACCAGTTCAACAGCGCCA
TCGGCAAGATCCAGGACAGCCTGAGCAGCACCGCCAGCGCCCTGGGCAAGCTGCAGGACG
TGGTGAACCACAACGCCCAGGCCCTGAACACCCTGGTGAAGCAGCTGAGCAGCAAGTTCG
GCGCCATCAGCAGCGTGCTGAACGACATCCTGAGCCGGCTGGACCCCCCCGAGGCCGAGG
TGCAGATCGACCGGCTGATCACCGGCCGGCTGCAGAGCCTGCAGACCTACGTGACCCAGCA
GCTGATCCGGGCCGCCGAGATCCGGGCCAGCGCCAACCTGGCCGCCACCAAGATGAGCGA
GTGCGTGCTGGGCCAGAGCAAGCGGGTGGACTTCTGCGGCAAGGGCTACCACCTGATGAG
CTTCCCCCAGAGCGCCCCCCACGGCGTGGTGTTCCTGCACGTGACCTACGTGCCCGCCCAG
GAGAAGAACTTCACCACCGCCCCCGCCATCTGCCACGACGGCAAGGCCCACTTCCCCCGGG
AGGGCGTGTTCGTGAGCAACGGCACCCACTGGTTCGTGACCCAGCGGAACTTCTACGAGCC
CCAGATCATCACCACCGACAACACCTTCGTGAGCGGCAACTGCGACGTGGTGATCGGCATC
GTGAACAACACCGTGTACGACCCCCTGCAGCCCGAGCTGGACAGCTTCAAGGAGGAGCTG
GACAAGTACTTCAAGAACCACACCAGCCCCGACGTGGACCTGGGCGACATCTCCGGCATCA
ACGCCAGCGTGGTGAACATCCAGAAGGAGATCGACCGGCTGAACGAGGTGGCCAAGAACC
TGAACGAGAGCCTGATCGACCTGCAGGAGCTGGGCAAGTACGAGCAGTACATCAAGTGGC
CCTGGTACATCTGGCTGGGCTTCATCGCCGGCCTGATCGCCATCGTGATGGTGACCATCATG
CTGTGCTGCATGACCAGCTGCTGCAGCTGCCTGAAGGGCTGCTGCAGCTGCGGCAGCTGCT
GCAAGTGATGACTCGAGCTGGTACTGCATGCACGCAATGCTAGCTGCCCCTTTCCCGTCCT
GGGTACCCCGAGTCTCCCCCGACCTCGGGTCCCAGGTATGCTCCCACCTCCACCTGCCCCAC
TCACCACCTCTGCTAGTTCCAGACACCTCCCAAGCACGCAGCAATGCAGCTCAAAACGCTT
AGCCTAGCCACACCCCACGGGAAACAGCAGTGATTAACCTTTAGCAATAAACGAAAGTTT

AACTAAGCTATACTAACCCCAGGGTTGGTCAATTTCGTGCCAGCCACACCCTGGAGCTAGC
AAAAAAAAAAAAAAAAAAAAAAAAAAAAAGCATATGACTAAAAAAAAAAAAAAAAAAA
AAAAAAAAAAAAAAAAAAAAAAAAAAAAAAAAAAAAAAAAAAAAAAAAAAAA

SEQ ID NO.16 (SF-9-BA.2.75-2 codon optimization sequence)

GGGAGACCCAAGCTGGCTAGCGTTTAAACTTAAGCTTGGTACCGAGCTCGGATCCAC
TAGTCCAGTGTGGTGGAATTCAGAATAAACTAGTATTCTTCTGGTCCCCACAGACTCAGAG
AGAACCCGCCACCATGTTCGTTTTTTTTAGTTTTATTACCATTAGTGAGCAGCCAGTGCGTGA
ACCTGATCACCCGGACCCAGAGCTACACCAACAGCTTCACCCGGGGCGTGTACTACCCCGA
CAAGGTGTTCCGGAGCAGCGTGCTGCACAGCACCCAGGACCTGTTCCTGCCCTTCTTCAGC
AACGTGACCTGGTTCCACGCCATCCACGTGAGCGGCACCAACGGCACCAAGCGGTTCGACA
ACCCCGTGCTGCCCTTCAACGACGGCGTGTACTTCGCCAGCACCGAGAAGAGCAACATCAT
CCGGGGCTGGATCTTCGGCACCACCCTGGACAGCAAGACCCAGAGCCTGCTGATCGTGAAC
AACGCCACCAACGTGGTGATCAAGGTGTGCGAGTTCCAGTTCTGCAACGACCCCTTCCTGG
ACGTGTACTACCACGAGAACAACAAGAGCCGGATGGAGAGCGAGCTGCGGGTGTACAGCA
GCGCCAACAACTGCACCTTCGAGTACGTGAGCCAGCCCTTCCTGATGGACCTGGAGGGCAA
GCAGGGCAACTTCAAGAACCTGCGGGAGTTCGTGTTCAAGAACATCGACGGCTACTTCAAG
ATCTACAGCAAGCACACCCCCGTGAACCTGGGCCGGGACCTGCCCCAGGGCTTCAGCGCCC
TGGAGCCCCTGGTGGACCTGCCCATCGGCATCAACATCACCCGGTTCCAGACCCTGCTGGC
CCTGCACCGGAGCTACCTGACCCCCGGCGACAGCAGCAGCAGCTGGACCGCCGGCGCCGC
CGCCTACTACGTGGGCTACCTGCAGCCCCGGACCTTCCTGCTGAAGTACAACGAGAACGGC
ACCATCACCGACGCCGTGGACTGCGCCCTGGACCCCCTGAGCGAGACCAAGTGCACCCTGA
AGAGCTTCACCGTGGAGAAGGGCATCTACCAGACCAGCAACTTCCGGGTGCAGCCCACCG
AGAGCATCGTGCGGTTCCCCAACATCACCAACCTGTGCCCCTTCCACGAGGTGTTCAACGC
CACCCGGTTCGCCAGCGTGTACGCCTGGAACCGGAAGCGGATCAGCAACTGCGTGGCCGA
CTACAGCGTGCTGTACAACTTCGCCCCCTTCTTCGCCTTCAAGTGCTACGGCGTGAGCCCCA
CCAAGCTGAACGACCTGTGCTTCACCAACGTGTACGCCGACAGCTTCGTGATCCGGGGCAA
CGAGGTGAGCCAGATCGCCCCCGGCCAGACCGGCAACATCGCCGACTACAACTACAAGCT
GCCCGACGACTTCACCGGCTGCGTGATCGCCTGGAACAGCAACAAGCTGGACAGCAAGGT
GAGCGGCAACTACAACTACCTGTACCGGCTGTTCCGGAAGAGCAAGCTGAAGCCCTTCGAG
CGGGACATCAGCACCGAGATCTACCAGGCCGGCAACAAGCCCTGCAACGGCGTGGCCGGC
TTCAACTGCTACTTCCCCCTGCGGAGCTACGGCTTCCGGCCCACCTACGGCGTGGGCCACC
AGCCCTACCGGGTGGTGGTGCTGAGCTTCGAGCTGCTGCACGCCCCCGCCACCGTGTGCGG
CCCCAAGAAGAGCACCAACCTGGTGAAGAACAAGTGCGTGAACTTCAACTTCAACGGCCT
GACCGGCACCGGCGTGCTGACCGAGAGCAACAAGAAGTTCCTGCCCTTCCAGCAGTTCGGC
CGGGACATCGCCGACACCACCGACGCCGTGCGGGACCCCCAGACCCTGGAGATCCTGGAC
ATCACCCCCTGCAGCTTCGGCGGCGTGAGCGTGATCACCCCCGGCACCAACACCAGCAACC
AGGTGGCCGTGCTGTACCAGGGCGTGAACTGCACCGAGGTGCCCGTGGCCATCCACGCCGA
CCAGCTGACCCCCACCTGGCGGGTGTACAGCACCGGCAGCAACGTGTTCCAGACCCGGGCC
GGCTGCCTGATCGGCGCCGAGTACGTGAACAACAGCTACGAGTGCGACATCCCCATCGGCG
CCGGCATCTGCGCCAGCTACCAGACCCAGACCAAGAGCCACCGGCGGGCCCGGAGCGTGG
CCAGCCAGAGCATCATCGCCTACACCATGAGCCTGGGCGCCGAGAACAGCGTGGCCTACA
GCAACAACAGCATCGCCATCCCCACCAACTTCACCATCAGCGTGACCACCGAGATCCTGCC
CGTGAGCATGACCAAGACCAGCGTGGACTGCACCATGTACATCTGCGGCGACAGCACCGA
GTGCAGCAACCTGCTGCTGCAGTACGGCAGCTTCTGCACCCAGCTGAAGCGGGCCCTGACC
GGCATCGCCGTGGAGCAGGACAAGAACACCCAGGAGGTGTTCGCCCAGGTGAAGCAGATC
TACAAGACCCCCCCCATCAAGTACTTCGGCGGCTTCAACTTCAGCCAGATCCTGCCCGACC
CCAGCAAGCCCAGCAAGCGGAGCTTCATCGAGGACCTGCTGTTCAACAAGGTGACCCTGGC
CGACGCCGGCTTCATCAAGCAGTACGGCGACTGCCTGGGCGACATCGCCGCCCGGGACCTG
ATCTGCGCCCAGAAGTTCAACGGCCTGACCGTGCTGCCCCCCCTGCTGACCGACGAGATGA
TCGCCCAGTACACCAGCGCCCTGCTGGCCGGCACCATCACCAGCGGCTGGACCTTCGGCGC
CGGCGCCGCCCTGCAGATCCCCTTCGCCATGCAGATGGCCTACCGGTTCAACGGCATCGGC
GTGACCCAGAACGTGCTGTACGAGAACCAGAAGCTGATCGCCAACCAGTTCAACAGCGCC
ATCGGCAAGATCCAGGACAGCCTGAGCAGCACCGCCAGCGCCCTGGGCAAGCTGCAGGAC
GTGGTGAACCACAACGCCCAGGCCCTGAACACCCTGGTGAAGCAGCTGAGCAGCAAGTTC
GGCGCCATCAGCAGCGTGCTGAACGACATCCTGAGCCGGCTGGACCCCCCCGAGGCCGAG
GTGCAGATCGACCGGCTGATCACCGGCCGGCTGCAGAGCCTGCAGACCTACGTGACCCAGC
AGCTGATCCGGGCCGCCGAGATCCGGGCCAGCGCCAACCTGGCCGCCACCAAGATGAGCG
AGTGCGTGCTGGGCCAGAGCAAGCGGGTGGACTTCTGCGGCAAGGGCTACCACCTGATGTC
CTTCCCCCAGAGCGCCCCCCACGGCGTGGTGTTCCTGCACGTGACCTACGTGCCCGCCCAG
GAGAAGAACTTCACCACCGCCCCCGCCATCTGCCACGACGGCAAGGCCCACTTCCCCCGGG
AGGGCGTGTTCGTGAGCAACGGCACCCACTGGTTCGTGACCCAGCGGAACTTCTACGAGCC
CCAGATCATCACCACCGACAACACCTTCGTGAGCGGCAACTGCGACGTGGTGATCGGCATC
GTGAACAACACCGTGTACGACCCCCTGCAGCCCGAGCTGGACAGCTTCAAGGAGGAGCTG
GACAAGTACTTCAAGAACCACACCAGCCCCGACGTGGACCTGGGCGACATCAGCGGCATC
AACGCCAGCGTGGTGAACATCCAGAAGGAGATCGACCGGCTGAACGAGGTGGCCAAGAAC
CTGAACGAGAGCCTGATCGACCTGCAGGAGCTGGGCAAGTACGAGCAGTACATCAAGTGG
CCCTGGTACATCTGGCTGGGCTTCATCGCCGGCCTGATCGCCATCGTGATGGTGACCATCAT
GCTGTGCTGCATGACCAGCTGCTGCAGCTGCCTGAAGGGCTGCTGCAGCTGCGGCAGCTGC
TGCAAGTGATGACTCGAGCTGGTACTGCATGCACGCAATGCTAGCTGCCCCTTTCCCGTCCT
GGGTACCCCGAGTCTCCCCCGACCTCGGGTCCCAGGTATGCTCCCACCTCCACCTGCCCCAC

TCACCACCTCTGCTAGTTCCAGACACCTCCCAAGCACGCAGCAATGCAGCTCAAAACGCTT
AGCCTAGCCACACCCCCACGGGAAACAGCAGTGATTAACCTTTAGCAATAAACGAAAGTTT
AACTAAGCTATACTAACCCCAGGGTTGGTCAATTTCGTGCCAGCCACACCCTGGAGCTAGC
AAAAAAAAAAAAAAAAAAAAAAAAAAAAAAAGCATATGACTAAAAAAAAAAAAAAAAAAAA
AAAAAAAAAAAAAAAAAAAAAAAAAAAAAAAAAAAAAAAAAAAAAAAAAA

SEQ ID NO.17 (SF-10-BO.1-01 codon optimization sequence)

GGGAGACCCAAGCTGGCTAGCGTTTAAACTTAAGCTTGGTACCGAGCTCGGATCCAC
TAGTCCAGTGTGGTGGAATTCAGAATAAACTAGTATTCTTCTGGTCCCCACAGACTCAGAG
AGAACCCGCCACCATGTTCGTTTTTTTAGTTTTATTACCTTTAGTGAGCAGCCAGTGCGTGA
ACCTGATCACCCGGACCCAGAGCTACACCAACAGCTTCACCCGGGGCGTGTACTACCCCGA
CAAGGTGTTCCGGAGCAGCGTGCTGCACAGCACCCAGGACCTGTTCCTGCCCTTCTTCAGC
AACGTGACCTGGTTCCACGCCATCAGCGGCACCAACGGCACCAAGCGGTTCGACAACCCCG
TGCTGCCCTTCAACGACGGCGTGTACTTCGCCAGCACCGAGAAGAGCAACATCATCCGGGG
CTGGATCTTCGGCACCACCCTGGACAGCAAGACCCAGAGCCTGCTGATCGTGAACAACGCC
ACCAACGTGGTGATCAAGGTGTGCGAGTTCCAGTTCTGCAACGACCCCTTCCTGGACGTGT
ACCACAAGAACAACAAGTCCTGGATGGAGAGCGAGTTCCGGGTGTACAGCAGCGCCAACA
ACTGCACCTTCGAGTACGTGAGCCAGCCCTTCCTGATGGACCTGGAGGGCAAGCAGGGCAA
CTTCAAGAACCTGCGGGAGTTCGTGTTCAAGAACATCGACGGCTACTTCAAGATCTACAGC
AAGCACACCCCCATCAACCTGGGCCGGGACCTGCCCCAGGGCTTCAGCGCCCTGGAGCCCC
TGGTGGACCTGCCCATCGGCATCAACATCACCCGGTTCCAGACCCTGCTGGCCCTGCACCG
GAGCTACCTGACCCCCGGCGACAGCAGCAGCGGCTGGACCGCCGGCGCCGCCGCCTACTA
CGTGGGCTACCTGCAGCCCCGGACCTTCCTGCTGAAGTACAACGAGAACGGCACCATCACC
GACGCCGTGGACTGCGCCCTGGACCCCCTGAGCGAGACCAAGTGCACCCTGAAGAGCTTCA
CCGTGGAGAAGGGCATCTACCAGACCAGCAACTTCCGGGTGCAGCCCACCGAGAGCATCG
TGCGGTTCCCCAACATCACCAACCTGTGCCCCTTCGACGAGGTGTTCAACGCCACCCGGTTC
GCCAGCGTGTACGCCTGGAACCGGAAGCGGATCAGCAACTGCGTGGCCGACTACAGCGTG
CTGTACAACTTCGCCCCCCTTCTTCGCCTTCAAGTGCTACGGCGTGAGCCCCACCAAGCTGAA
CGACCTGTGCTTCACCAACGTGTACGCCGACAGCTTCGTGATCCGGGGCAACGAGGTGAGC
CAGATCGCCCCCGGCCAGACCGGCAACATCGCCGACTACAACTACAAGCTGCCCGACGACT
TCACCGGCTGCGTGATCGCCTGGAACAGCAACAAGCTGGACAGCAccGTGGGCGGCAACTA
CAACTACCGGTACCGGCTGTTCCGGAAGAGCAAGCTGAAGCCCTTCGAGCGGGACATCAG
CACCGAGATCTACCAGGCCGGCAACAAGCCCTGCAACGGCGTGGCCGGCGTGAACTGCTA
CTTCCCCCTGCAGAGCTACGGCTTCCGGCCCACCTACGGCGTGGGCCACCAGCCCTACCGG
GTGGTGGTGCTGAGCTTCGAGCTGCTGCACGCCCCCGCCACCGTGTGCGGCCCCAAGAAGA
GCACCAACCTGGTGAAGAACAAGTGCGTGAACTTCAACTTCAACGGCCTGACCGGCACCG
GCGTGCTGACCGAGAGCAACAAGAAGTTCCTGCCCTTCCAGCAGTTCGGCCGGGACATCGC
CGACACCACCGACGCCGTGCGGGACCCCCAGACCCTGGAGATCCTGGACATCACCCCCTGC
AGCTTCGGCGGCGTGTCCGTGATCACCCCCGGCACCAACACCAGCAACCAGGTGGCCGTGC
TGTACCAGGGCGTGAACTGCACCGAGGTGCCCGTGGCCATCCACGCCGACCAGCTGACCCC
CACCTGGCGGGTGTACAGCACCGGCAGCAACGTGTTCCAGACCCGGGCCGGCTGCCTGATC
GGCGCCGAGTACGTGAACAACAGCTACGAGTGCGACATCCCCATCGGCGCCGGCATCTGC
GCCAGCTACCAGACCCAGACCAAGAGCCACCGGCGGGCCCGGAGCGTGGCCAGCCAGAGC
ATCATCGCCTACACCATGAGCCTGGGCGCCGAGAACAGCGTGGCCTACAGCAACAACAGC
ATCGCCATCCCCACCAACTTCACCATCAGCGTGACCACCGAGATCCTGCCCGTGAGCATGA
CCAAGACCAGCGTGGACTGCACCATGTACATCTGCGGCGACAGCACCGAGTGCAGCAACC
TGCTGCTGCAGTACGGCAGCTTCTGCACCCAGCTGAAGCGGGCCCTGACCGGCATCGCCGT
GGAGCAGGACAAGAACACCCAGGAGGTGTTCGCCCAGGTGAAGCAGATCTACAAGACCCC
CCCCATCAAGTACTTCGGCGGCTTCAACTTCAGCCAGATCCTGCCCGACCCCAGCAAGCCC
AGCAAGCGGAGCTTCATCGAGGACCTGCTGTTCAACAAGGTGACCCTGGCCGACGCCGGCT
TCATCAAGCAGTACGGCGACTGCCTGGGCGACATCGCCGCCCGGGACCTGATCTGCGCCCA
GAAGTTCAACGGCCTGACCGTGCTGCCCCCCCTGCTGACCGACGAGATGATCGCCCAGTAC
ACCAGCGCCCTGCTGGCCGGCACCATCACCAGCGGCTGGACCTTCGGCGCCGGCGCCGCCC
TGCAGATCCCCTTCGCCATGCAGATGGCCTACCGGTTCAACGGCATCGGCGTGACCCAGAA
CGTGCTGTACGAGAACCAGAAGCTGATCGCCAACCAGTTCAACAGCGCCATCGGCAAGAT
CCAGGACAGCCTGAGCAGCACCGCCAGCGCCCTGGGCAAGCTGCAGGACGTGGTGAACCA
CAACGCCCAGGCCCTGAACACCCTGGTGAAGCAGCTGAGCAGCAAGTTCGGCGCCATCAG
CAGCGTGCTGAACGACATCCTGAGCCGGCTGGACCCCCCCGAGGCCGAGGTGCAGATCGA
CCGGCTGATCACCGGCCGGCTGCAGAGCCTGCAGACCTACGTGACCCAGCAGCTGATCCGG
GCCGCCGAGATCCGGGCCAGCGCCAACCTGGCCGCCACCAAGATGAGCGAGTGCGTGCTG
GGCCAGAGCAAGCGGGTGGACTTCTGCGGCAAGGGCTACCACCTGATGAGCTTCCCCCAG
AGCGCCCCCCACGGCGTGGTGTTCCTGCACGTGACCTACGTGCCCGCCCAGGAGAAGAACT
TCACCACCGCCCCCGCCATCTGCCACGACGGCAAGGCCCACTTCCCCCGGGAGGGCGTGTT
CGTGAGCAACGGCACCCACTGGTTCGTGACCCAGCGGAACTTCTACGAGCCCCAGATCATC
ACCACCGACAACACCTTCGTGAGCGGCAACTGCGACGTGGTGATCGGCATCGTGAACAAC
ACCGTGTACGACCCCCTGCAGCCCGAGCTGGACAGCTTCAAGGAGGAGCTGGACAAGTAC
TTCAAGAACCACACCAGCCCCGACGTGGACCTGGGCGACATCAGCGGCATCAACGCCAGC
GTGGTGAACATCCAGAAGGAGATCGACCGGCTGAACGAGGTGGCCAAGAACCTGAACGAG
AGCCTGATCGACCTGCAGGAGCTGGGCAAGTACGAGCAGTACATCAAGTGGCCCTGGTAC
ATCTGGCTGGGCTTCATCGCCGGCCTGATCGCCATCGTGATGGTGACCATCATGCTGTGCTG
CATGACCAGCTGCTGCAGCTGCCTGAAGGGCTGCTGCAGCTGCGGCAGCTGCTGCAAGTTC

GACGAGGACGACAGCGAGCCCGTGCTGAAGGGCGTGAAGCTGCACTACACCTGATGACTC
GAGCTGGTACTGCATGCACGCAATGCTAGCTGCCCCTTTCCCGTCCTGGGTACCCCGAGTCT
CCCCCGACCTCGGGTCCCAGGTATGCTCCCACCTCCACCTGCCCCACTCACCACCTCTGCTA
GTTCCAGACACCTCCCAAGCACGCAGCAATGCAGCTCAAAACGCTTAGCCTAGCCACACCC
CCACGGGAAACAGCAGTGATTAACCTTTAGCAATAAACGAAAGTTTAACTAAGCTATACTA
ACCCCAGGGTTGGTCAATTTCGTGCCAGCCACACCCTGGAGCTAGCAAAAAAAAAAAAAAA
AAAAAAAAAAAAAAAAGCATATGACTAAAAAAAAAAAAAAAAAAAAAAAAAAAAAAAAAA
AAAAAAAAAAAAAAAAAAAAAAAAAAAAAAAAAAAA

SEQ ID NO.18 (SF-11-BQ.1-02 codon optimization sequence)

GGGAGACCCAAGCTGGCTAGCGTTTAAACTTAAGCTTGGTACCGAGCTCGGATCCAC
TAGTCCAGTGTGGTGGAATTCAGAATAAACTAGTATTCTTCTGGTCCCCACAGACTCAGAG
AGAACCCGCCACCATGTTCGTTTTTTTTAGTTTTATTACCATTAGTGAGCAGCCAGTGCGTGA
ACCTGATCACCCGGACCCAGAGCTACACCAACAGCTTCACCCGGGGCGTGTACTACCCCGA
CAAGGTGTTCCGGAGCAGCGTGCTGCACAGCACCCAGGACCTGTTCCTGCCCTTCTTCAGC
AACGTGACCTGGTTCCACGCCATCAGCGGCACCAACGGCACCAAGCGGTTCGACAACCCCG
TGCTGCCCTTCAACGACGGCGTGTACTTCGCCAGCACCGAGAAGAGCAACATCATCCGGGG
CTGGATCTTCGGCACCACCCTGGACAGCAAGACCCAGAGCCTGCTGATCGTGAACAACGCC
ACCAACGTGGTGATCAAGGTGTGCGAGTTCCAGTTCTGCAACGACCCCTTCCTGGACGTGT
ACCACAAGAACAACAAGAGCTGGATGGAGAGCGAGTTCCGGGTGTACAGCAGCGCCAACA
ACTGCACCTTCGAGTACGTGAGCCAGCCCTTCCTGATGGACCTGGAGGGCAAGCAGGGCAA
CTTCAAGAACCTGCGGGAGTTCGTGTTCAAGAACATCGACGGCTACTTCAAGATCTACAGC
AAGCACACCCCCATCAACCTGGGCCGGGACCTGCCCCAGGGCTTCAGCGCGCTGGAGCCCC
TGGTGGACCTGCCCATCGGCATCAACATCACCCGGTTCCAGACCCTGCTGGCCCTGCACCG
GAGCTACCTGACCCCCGGCGACAGCAGCAGCGGCTGGACCGCCGGCGCCGCCGCCTACTA
CGTGGGCTACCTGCAGCCCCGGACCTTCCTGCTGAAGTACAACGAGAACGGCACCATCACC
GACGCCGTGGACTGCGCCCTGGACCCCCTGAGCGAGACCAAGTGCACCCTGAAGAGCTTCA
CCGTGGAGAAGGGCATCTACCAGACCAGCAACTTCCGGGTGCAGCCCACCGAGAGCATCG
TGCGGTTCCCCAACATCACCAACCTGTGCCCCTTCGACGAGGTGTTCAACGCCACCCGGTTC
GCCAGCGTGTACGCCTGGAACCGGAAGCGGATCAGCAACTGCGTGGCCGACTACAGCGTG
CTGTACAACTTCGCCCCCTTCTTCGCCTTCAAGTGCTACGGCGTGAGCCCCACCAAGCTGAA
CGACCTGTGCTTCACCAACGTGTACGCCGACAGCTTCGTGATCCGGGGCAACGAGGTGAGC
CAGATCGCCCCCGGCCAGACCGGCAACATCGCCGACTACAACTACAAGCTGCCCGACGACT
TCACCGGCTGCGTGATCGCCTGGAACAGCAACAAGCTGGACAGCACCGTGGGCGGCAACT
ACAACTACCGGTACCGGCTGTTCCGGAAGAGCAAGCTGAAGCCCTTCGAGCGGGACATCA
GCACCGAGATCTACCAGGCCGGCAACAAGCCCTGCAACGGCGTGGCCGGCGTGAACTGCT
ACTTCCCCCTGCAGAGCTACGGCTTCCGGCCCACCTACGGCGTGGGCCACCAGCCCTACCG
GGTGGTGGTGCTGAGCTTCGAGCTGCTGCACGCCCCCGCCACCGTGTGCGGCCCCAAGAAG
AGCACCAACCTGGTGAAGAACAAGTGCGTGAACTTCAACTTCAACGGCCTGACCGGCACC
GGCGTGCTGACCGAGAGCAACAAGAAGTTCCTGCCCTTCCAGCAGTTCGGCCGGGACATCG
CCGACACCACCGACGCCGTGCGGGACCCCCAGACCCTGGAGATCCTGGACATCACCCCCTG
CAGCTTCGGCGGCGTGAGCGTGATCACCCCCGGCACCAACACCAGCAACCAGGTGGCCGT
GCTGTACCAGGGCGTGAACTGCACCGAGGTGCCCGTGGCCATCCACGCCGACCAGCTGACC
CCCACCTGGCGGGTGTACAGCACCGGCAGCAACGTGTTCCAGACCCGGGCCGGCTGCCTGA
TCGGCGCCGAGTACGTGAACAACAGCTACGAGTGCGACATCCCCATCGGCGCCGGCATCTG
CGCCAGCTACCAGACCCAGACCAAGAGCCACCGGCGGGCCCGGAGCGTGGCCAGCCAGAG
CATCATCGCCTACACCATGAGCCTGGGCGCCGAGAACAGCGTGGCCTACAGCAACAACAG
CATCGCCATCCCCACCAACTTCACCATCTCCGTGACCACCGAGATCCTGCCCGTGAGCATG
ACCAAGACCAGCGTGGACTGCACCATGTACATCTGCGGCGACAGCACCGAGTGCAGCAAC
CTGCTGCTGCAGTACGGCAGCTTCTGCACCCAGCTGAAGCGGGCCCTGACCGGCATCGCCG
TGGAGCAGGACAAGAACACCCAGGAGGTGTTCGCCCAGGTGAAGCAGATCTACAAGACCC
CCCCCATCAAGTACTTCGGCGGCTTCAACTTCAGCCAGATCCTGCCCGACCCCAGCAAGCC
CAGCAAGCGGAGCTTCATCGAGGACCTGCTGTTCAACAAGGTGACCCTGGCCGACGCCGGC
TTCATCAAGCAGTACGGCGACTGCCTGGGCGACATCGCCGCCCGGGACCTGATCTGCGCCC
AGAAGTTCAACGGCCTGACCGTGCTGCCCCCCCTGCTGACCGACGAGATGATCGCCCAGTA
CACCAGCGCCCTGCTGGCCGGCACCATCACCAGCGGCTGGACCTTCGGCGCCGGCGCCGCC
CTGCAGATCCCCTTCGCCATGCAGATGGCCTACCGGTTCAACGGCATCGGCGTGACCCAGA
ACGTGCTGTACGAGAACCAGAAGCTGATCGCCAACCAGTTCAACAGCGCCATCGGCAAGA
TCCAGGACAGCCTGAGCAGCACCGCCAGCGCCCTGGGCAAGCTGCAGGACGTGGTGAACC
ACAACGCCCAGGCCCTGAACACCCTGGTGAAGCAGCTGAGCAGCAAGTTCGGCGCCATCA
GCAGCGTGCTGAACGACATCCTGAGCCGGCTGGACCCCCCCGAGGCCGAGGTGCAGATCG
ACCGGCTGATCACCGGCCGGCTGCAGAGCCTGCAGACCTACGTGACCCAGCAGCTGATCCG
GGCCGCCGAGATCCGGGCCAGCGCCAACCTGGCCGCCACCAAGATGAGCGAGTGCGTGCT
GGGCCAGAGCAAGCGGGTGGACTTCTGCGGCAAGGGCTACCACCTGATGAGCTTCCCCCA
GAGCGCCCCCCACGGCGTGGTGTTCCTGCACGTGACCTACGTGCCCGCCCAGGAGAAGAAC
TTCACCACCGCCCCCGCCATCTGCCACGACGGCAAGGCCCACTTCCCCCGGGAGGGCGTGT
TCGTGAGCAACGGCACCCACTGGTTCGTGACCCAGCGGAACTTCTACGAGCCCCAGATCAT
CACCACCGACAACACCTTCGTGAGCGGCAACTGCGACGTGGTGATCGGCATCGTGAACAAC
ACCGTGTACGACCCCCTGCAGCCCGAGCTGGACAGCTTCAAGGAGGAGCTGGACAAGTAC
TTCAAGAACCACACCAGCCCCGACGTGGACCTGGGCGACATCAGCGGCATCAACGCCAGC
GTGGTGAACATCCAGAAGGAGATCGACCGGCTGAACGAGGTGGCCAAGAACCTGAACGAG

AGCCTGATCGACCTGCAGGAGCTGGGCAAGTACGAGCAGTACATCAAGTGGCCCTGGTAC
ATCTGGCTGGGCTTCATCGCCGGCCTGATCGCCATCGTGATGGTGACCATCATGCTGTGCTG
CATGACCAGCTGCTGCAGCTGCCTGAAGGGCTGCTGCAGCTGCGGCAGCTGCTGCAAGTTC
GACGAGGACGACAGCGAGCCCGTGCTGAAGGGCGTGAAGCTGCACTACACCTGATGACTC
GAGCTGGTACTGCATGCACGCAATGCTAGCTGCCCCTTTCCCGTCCTGGGTACCCCGAGTCT
CCCCCGACCTCGGGTCCCAGGTATGCTCCCACCTCCACCTGCCCCACTCACCACCTCTGCTA
GTTCCAGACACCTCCCAAGCACGCAGCAATGCAGCTCAAAACGCTTAGCCTAGCCACACCC
CCACGGGAAACAGCAGTGATTAACCTTTAGCAATAAACGAAAGTTTAACTAAGCTATACTA
ACCCCAGGGTTGGTCAATTCGTGCCAGCCACACCCTGGAGCTAGCAAAAAAAAAAAAAA
AAAAAAAAAAAAAAAAAGCATATGACTAAAAAAAAAAAAAAAAAAAAAAAAAAAAAAA
AAAAAAAAAAAAAAAAAAAAAAAAAAAAAAAAAAAAAAA

SEQ ID NO.19 (SF-12-BF.7-01 codon optimization sequence)

GGGAGACCCAAGCTGGCTAGCGTTTAAACTTAAGCTTGGTACCGAGCTCGGATCCAC
TAGTCCAGTGTGGTGGAATTCAGAATAAACTAGTATTCTTCTGGTCCCCACAGACTCAGAG
AGAACCCGCCACCATGTTCGTTTTTTTAGTTTTATTACCTTTAGTGAGCAGCCAGTGCGTGA
ACCTGATCACCCGAGACCCAGAGCTACACCAACAGCTTCACCCGGGGCGTGTACTACCCCGA
CAAGGTGTTCCGGAGCAGCGTGCTGCACAGCACCCAGGACCTGTTCCTGCCCTTCTTCAGC
AACGTGACCTGGTTCCACGCCATCAGCGGCACCAACGGCACCAAGCGGTTCGACAACCCCG
TGCTGCCCTTCAACGACGGCGTGTACTTCGCCAGCACCGAGAAGAGCAACATCATCCGGGG
CTGGATCTTCGGCACCACCCTGGACAGCAAGACCCAGAGCCTGCTGATCGTGAACAACGCC
ACCAACGTGGTGATCAAGGTGTGCGAGTTCCAGTTCTGCAACGACCCCTTCCTGGACGTGT
ACTACCACAAGAACAACAAGTCCTGGATGGAGAGCGAGTTCCGGGTGTACAGCAGCGCCA
ACAACTGCACCTTCGAGTACGTGAGCCAGCCCTTCCTGATGGACCTGGAGGGCAAGCAGGG
CAACTTCAAGAACCTGCGGGAGTTCGTGTTCAAGAACATCGACGGCTACTTCAAGATCTAC
AGCAAGCACACCCCCATCAACCTGGGCCGGGACCTGCCCCAGGGCTTCAGCGCCCTGGAGC
CCCTGGTGGACCTGCCCCATCGGCATCAACATCACCCGGTTCCAGACCCTGCTGGCCCTGCA
CCGGAGCTACCTGACCCCCGGCGACAGCAGCAGCGGCTGGACCGCCGGCGCCGCCGCCTA
CTACGTGGGCTACCTGCAGCCCCGGACCTTCCTGCTGAAGTACAACGAGAACGGCACCATC
ACCGACGCCGTGGACTGCGCCCTGGACCCCCTGAGCGAGACCAAGTGCACCCTGAAGAGC
TTCACCGTGGAGAAGGGCATCTACCAGACCAGCAACTTCCGGGTGCAGCCCACCGAGAGC
ATCGTGCGGTTCCCCAACATCACCAACCTGTGCCCCTTCGACGAGGTGTTCAACGCCACCA
CCTTCGCCAGCGTGTACGCCTGGAACCGGAAGCGGATCAGCAACTGCGTGGCCGACTACAG
CGTGCTGTACAACTTCGCCCCCTTCTTCGCCTTCAAGTGCTACGGCGTGAGCCCCACCAAGC
TGAACGACCTGTGCTTCACCAACGTGTACGCCGACAGCTTCGTGATCCGGGGCAACGAGGT
GAGCCAGATCGCCCCCGGCCAGACCGGCAACATCGCCGACTACAACTACAAGCTGCCCGA
CGACTTCACCGGCTGCGTGATCGCCTGGAACAGCAACAAGCTGGACAGCAAGGTGGGCGG
CAACTACAACTACCGGTACCGGCTGTTCCGGAAGAGCAACCTGAAGCCCTTCGAGCGGGAC
ATCAGCACCGAGATCTACCAGGCCGGCAACAAGCCCTGCAACGGCGTGGCCGGCGTGAAC
TGCTACTTCCCCCTGCAGAGCTACGGCTTCCGGCCCACCTACGGCGTGGGCCACCAGCCCT
ACCGGGTGGTGGTGCTGAGCTTCGAGCTGCTGCACGCCCCCGCCACCGTGTGCGGCCCCAA
GAAGAGCACCAACCTGGTGAAGAACAAGTGCGTGAACTTCAACTTCAACGGCCTGACCGG
CACCGGCGTGCTGACCGAGAGCAACAAGAAGTTCCTGCCCTTCCAGCAGTTCGGCCGGGAC
ATCGCCGACACCACCGACGCCGTGCGGGACCCCCAGACCCTGGAGATCCTGGACATCACCC
CCTGCAGCTTCGGCGGCGTGTCCGTGATCACCCCCGGCACCAACACCAGCAACCAGGTGGC
CGTGCTGTACCAGGGCGTGAACTGCACCGAGGTGCCCGTGGCCATCCACGCCGACCAGCTG
ACCCCCACCTGGCGGGTGTACAGCACCGGCAGCAACGTGTTCCAGACCCGGGCCGGCTGCC
TGATCGGCGCCGAGTACGTGAACAACAGCTACGAGTGCGACATCCCCATCGGCGCCGGCAT
CTGCGCCAGCTACCAGACCCAGACCAAGAGCCACCGGCGGGCCCGGAGCGTGGCCAGCCA
GAGCATCATCGCCTACACCATGAGCCTGGGCGCCGAGAACAGCGTGGCCTACAGCAACAA
CAGCATCGCCATCCCCACCAACTTCACCATCAGCGTGACCACCGAGATCCTGCCCGTGAGC
ATGACCAAGACCAGCGTGGACTGCACCATGTACATCTGCGGCGACAGCACCGAGTGCAGC
AACCTGCTGCTGCAGTACGGCAGCTTCTGCACCCAGCTGAAGCGGGCCCTGACCGGCATCG
CCGTGGAGCAGGACAAGAACACCCAGGAGGTGTTCGCCCAGGTGAAGCAGATCTACAAGA
CCCCCCCCATCAAGTACTTCGGCGGCTTCAACTTCAGCCAGATCCTGCCCGACCCCAGCAA
GCCCAGCAAGCGGAGCTTCATCGAGGACCTGCTGTTCAACAAGGTGACCCTGGCCGACGCC
GGCTTCATCAAGCAGTACGGCGACTGCCTGGGCGACATCGCCGCCCGGGACCTGATCTGCG
CCCAGAAGTTCAACGGCCTGACCGTGCTGCCCCCCCTGCTGACCGACGAGATGATCGCCCA
GTACACCAGCGCCCTGCTGGCCGGCACCATCACCAGCGGCTGGACCTTCGGCGCCGGCGCC
GCCCTGCAGATCCCCTTCGCCATGCAGATGGCCTACCGGTTCAACGGCATCGGCGTGACCC
AGAACGTGCTGTACGAGAACCAGAAGCTGATCGCCAACCAGTTCAACAGCGCCATCGGCA
AGATCCAGGACAGCCTGAGCAGCACCGCCAGCGCCCTGGGCAAGCTGCAGGACGTGGTGA
ACCACAACGCCCAGGCCCTGAACACCCTGGTGAAGCAGCTGAGCAGCAAGTTCGGCGCCA
TCAGCAGCGTGCTGAACGACATCCTGAGCCGGCTGGACCCCCCCGAGGCCGAGGTGCAGA
TCGACCGGCTGATCACCGGCCGGCTGCAGAGCCTGCAGACCTACGTGACCCAGCAGCTGAT
CCGGGCCGCCGAGATCCGGGCCAGCGCCAACCTGGCCGCCACCAAGATGAGCGAGTGCGT
GCTGGGCCAGAGCAAGCGGGTGGACTTCTGCGGCAAGGGCTACCACCTGATGAGCTTCCCC
CAGAGCGCCCCCCACGGCGTGGTGTTCCTGCACGTGACCTACGTGCCCGCCCAGGAGAAGA
ACTTCACCACCGCCCCCGCCATCTGCCACGACGGCAAGGCCCACTTCCCCCGGGAGGGCGT
GTTCGTGAGCAACGGCACCCACTGGTTCGTGACCCAGCGGAACTTCTACGAGCCCCAGATC

```
ATCACCACCGACAACACCTTCGTGAGCGGCAACTGCGACGTGGTGATCGGCATCGTGAACA
ACACCGTGTACGACCCCCTGCAGCCCGAGCTGGACAGCTTCAAGGAGGAGCTGGACAAGT
ACTTCAAGAACCACACCAGCCCCGACGTGGACCTGGGCGACATCAGCGGCATCAACGCCA
GCGTGGTGAACATCCAGAAGGAGATCGACCGGCTGAACGAGGTGGCCAAGAACCTGAACG
AGAGCCTGATCGACCTGCAGGAGCTGGGCAAGTACGAGCAGTACATCAAGTGGCCCTGGT
ACATCTGGCTGGGCTTCATCGCCGGCCTGATCGCCATCGTGATGGTGACCATCATGCTGTGC
TGCATGACCAGCTGCTGCAGCTGCCTGAAGGGCTGCTGCAGCTGCGGCAGCTGCTGCAAGT
TCGACGAGGACGACAGCGAGCCCGTGCTGAAGGGCGTGAAGCTGCACTACACCTGATGAC
TCGAGCTGGTACTGCATGCACGCAATGCTAGCTGCCCCTTTCCCGTCCTGGGTACCCCGAGT
CTCCCCCGACCTCGGGTCCCAGGTATGCTCCCACCTCCACCTGCCCCACTCACCACCTCTGC
TAGTTCCAGACACCTCCCAAGCACGCAGCAATGCAGCTCAAAACGCTTAGCCTAGCCACAC
CCCCACGGGAAACAGCAGTGATTAACCTTTAGCAATAAACGAAAGTTTAACTAAGCTATAC
TAACCCCAGGGTTGGTCAATTTCGTGCCAGCCACACCCTGGAGCTAGCAAAAAAAAAAAA
AAAAAAAAAAAAAAAAAAAGCATATGACTAAAAAAAAAAAAAAAAAAAAAAAAAAAAAA
AAAAAAAAAAAAAAAAAAAAAAAAAAAAAAAAAAAAAA
```

SEQ ID NO.20 (SF-13-BF.7-02 codon optimization sequence)

GGGAGACCCAAGCTGGCTAGCGTTTAAACTTAAGCTTGGTACCGAGCTCGGATCCAC
TAGTCCAGTGTGGTGGAATTCAGAATAAACTAGTATTCTTCTGGTCCCCACAGACTCAGAG
AGAACCCGCCACCATGTTCGTTTTTTTTAGTTTTATTACCATTAGTGAGCAGCCAGTGCGTGA
ACCTGATCACCCGGACCCAGAGCTACACCAACAGCTTCACCCGGGGCGTGTACTACCCCGA
CAAGGTGTTCCGGAGCAGCGTGCTGCACAGCACCCAGGACCTGTTCCTGCCCTTCTTCAGC
AACGTGACCTGGTTCCACGCCATCAGCGGCACCAACGGCACCAAGCGGTTCGACAACCCCG
TGCTGCCCTTCAACGACGGCGTGTACTTCGCCAGCACCGAGAAGAGCAACATCATCCGGGG
CTGGATCTTCGGCACCACCCTGGACAGCAAGACCCAGAGCCTGCTGATCGTGAACAACGCC
ACCAACGTGGTGATCAAGGTGTGCGAGTTCCAGTTCTGCAACGACCCCTTCCTGGACGTGT
ACTACCACAAGAACAACAAGAGCTGGATGGAGAGCGAGTTCCGGGTGTACAGCAGCGCCA
ACAACTGCACCTTCGAGTACGTGAGCCAGCCCTTCCTGATGGACCTGGAGGGCAAGCAGGG
CAACTTCAAGAACCTGCGGGAGTTCGTGTTCAAGAACATCGACGGCTACTTCAAGATCTAC
AGCAAGCACACCCCCATCAACCTGGGCCGGGACCTGCCCCAGGGCTTCAGCGCCCTGGAGC
CCCTGGTGGACCTGCCCATCGGCATCAACATCACCCGGTTCCAGACCCTGCTGGCCCTGCA
CCGGAGCTACCTGACCCCCGGCGACAGCAGCAGCGGCTGGACCGCCGGCGCCGCCGCCTA
CTACGTGGGCTACCTGCAGCCCCGGACCTTCCTGCTGAAGTACAACGAGAACGGCACCATC
ACCGACGCCGTGGACTGCGCCCTGGACCCCCTGAGCGAGACCAAGTGCACCCTGAAGAGC
TTCACCGTGGAGAAGGGCATCTACCAGACCAGCAACTTCCGGGTGCAGCCCACCGAGAGC
ATCGTGCGGTTCCCCAACATCACCAACCTGTGCCCCTTCGACGAGGTGTTCAACGCCACCA
CCTTCGCCAGCGTGTACGCCTGGAACCGGAAGCGGATCAGCAACTGCGTGGCCGACTACAG
CGTGCTGTACAACTTCGCCCCCTTCTTCGCCCTTCAAGTGCTACGGCGTGAGCCCCACCAAGC
TGAACGACCTGTGCTTCACCAACGTGTACGCCGACAGCTTCGTGATCCGGGGCAACGAGGT
GAGCCAGATCGCCCCCGGCCAGACCGGCAACATCGCCGACTACAACTACAAGCTGCCCGA
CGACTTCACCGGCTGCGTGATCGCCTGGAACAGCAACAAGCTGGACAGCAAGGTGGGCGG
CAACTACAACTACCGGTACCGGCTGTTCCGGAAGAGCAACCTGAAGCCCTTCGAGCGGGAC
ATCAGCACCGAGATCTACCAGGCCGGCAACAAGCCCTGCAACGGCGTGGCCGGCGTGAAC
TGCTACTTCCCCCTGCAGAGCTACGGCTTCCGGCCCACCTACGGCGTGGGCCACCAGCCCT
ACCGGGTGGTGGTGCTGAGCTTCGAGCTGCTGCACGCCCCCGCCACCGTGTGCGGCCCCAA
GAAGAGCACCAACCTGGTGAAGAACAAGTGCGTGAACTTCAACTTCAACGGCCTGACCGG
CACCGGCGTGCTGACCGAGAGCAACAAGAAGTTCCTGCCCTTCCAGCAGTTCGGCCGGGAC
ATCGCCGACACCACCGACGCCGTGCGGGACCCCCAGACCCTGGAGATCCTGGACATCACCC
CCTGCAGCTTCGGCGGCGTGAGCGTGATCACCCCCGGCACCAACACCAGCAACCAGGTGGC
CGTGCTGTACCAGGGCGTGAACTGCACCGAGGTGCCCGTGGCCATCCACGCCGACCAGCTG
ACCCCCACCTGGCGGGTGTACAGCACCGGCAGCAACGTGTTCCAGACCCGGGCCGGCTGCC
TGATCGGCGCCGAGTACGTGAACAACAGCTACGAGTGCGACATCCCCATCGGCGCCGGCAT
CTGCGCCAGCTACCAGACCCAGACCAAGAGCCACCGGCGGGCCCGGAGCGTGGCCAGCCA
GAGCATCATCGCCTACACCATGAGCCTGGGCGCCGAGAACAGCGTGGCCTACAGCAACAA
CAGCATCGCCATCCCCACCAACTTCACCATCAGCGTGACCACCGAGATCCTGCCCGTGAGC
ATGACCAAGACCAGCGTGGACTGCACCATGTACATCTGCGGCGACAGCACCGAGTGCAGC
AACCTGCTGCTGCAGTACGGCAGCTTCTGCACCCAGCTGAAGCGGGCCCTGACCGGCATCG
CCGTGGAGCAGGACAAGAACACCCAGGAGGTGTTCGCCCAGGTGAAGCAGATCTACAAGA
CCCCCCCCATCAAGTACTTCGGCGGCTTCAACTTCAGCCAGATCCTGCCCGACCCCAGCAA
GCCCAGCAAGCGGAGCTTCATCGAGGACCTGCTGTTCAACAAGGTGACCCTGGCCGACGCC
GGCTTCATCAAGCAGTACGGCGACTGCCTGGGCGACATCGCCGCCCGGGACCTGATCTGCG
CCCAGAAGTTCAACGGCCTGACCGTGCTGCCCCCCCTGCTGACCGACGAGATGATCGCCCA
GTACACCAGCGCCCTGCTGGCCGGCACCATCACCAGCGGCTGGACCTTCGGCGCCGGCGCC
GCCCTGCAGATCCCCTTCGCCATGCAGATGGCCTACCGGTTCAACGGCATCGGCGTGACCC
AGAACGTGCTGTACGAGAACCAGAAGCTGATCGCCAACCAGTTCAACAGCGCCATCGGCA
AGATCCAGGACAGCCTGAGCAGCACCGCCAGCGCCCTGGGCAAGCTGCAGGACGTGGTGA
ACCACAACGCCCAGGCCCTGAACACCCTGGTGAAGCAGCTGAGCAGCAAGTTCGGCGCCA
TCAGCAGCGTGCTGAACGACATCCTGAGCCGGCTGGACCCCCCCGAGGCCGAGGTGCAGA
TCGACCGGCTGATCACCGGCCGGCTGCAGAGCCTGCAGACCTACGTGACCCAGCAGCTGAT
CCGGGCCGCCGAGATCCGGGCCAGCGCCAACCTGGCCGCCACCAAGATGAGCGAGTGCGT
GCTGGGCCAGAGCAAGCGGGTGGACTTCTGCGGCAAGGGCTACCACCTGATGTCCTTCCCC

CAGAGCGCCCCCCACGGCGTGGTGTTCCTGCACGTGACCTACGTGCCCGCCCAGGAGAAGA
ACTTCACCACCGCCCCCGCCATCTGCCACGACGGCAAGGCCCACTTCCCCCGGGAGGGCGT
GTTCGTGAGCAACGGCACCCACTGGTTCGTGACCCAGCGGAACTTCTACGAGCCCCAGATC
ATCACCACCGACAACACCTTCGTGAGCGGCAACTGCGACGTGGTGATCGGCATCGTGAACA
ACACCGTGTACGACCCCCTGCAGCCCGAGCTGGACAGCTTCAAGGAGGAGCTGGACAAGT
ACTTCAAGAACCACACCAGCCCCGACGTGGACCTGGGCGACATCAGCGGCATCAACGCCA
GCGTGGTGAACATCCAGAAGGAGATCGACCGGCTGAACGAGGTGGCCAAGAACCTGAACG
AGAGCCTGATCGACCTGCAGGAGCTGGGCAAGTACGAGCAGTACATCAAGTGGCCCTGGT
ACATCTGGCTGGGCTTCATCGCCGGCCTGATCGCCATCGTGATGGTGACCATCATGCTGTGC
TGCATGACCAGCTGCTGCAGCTGCCTGAAGGGCTGCTGCAGCTGCGGCAGCTGCTGCAAGT
TCGACGAGGACGACAGCGAGCCCGTGCTGAAGGGCGTGAAGCTGCACTACACCTGATGAC
TCGAGCTGGTACTGCATGCACGCAATGCTAGCTGCCCCTTTCCCGTCCTGGGTACCCCGAGT
CTCCCCCGACCTCGGGTCCCAGGTATGCTCCCACCTCCACCTGCCCCACTCACCACCTCTGC
TAGTTCCAGACACCTCCCAAGCACGCAGCAATGCAGCTCAAAACGCTTAGCCTAGCCACAC
CCCCACGGGAAACAGCAGTGATTAACCTTTAGCAATAAACGAAAGTTTAACTAAGCTATAC
TAACCCCAGGGTTGGTCAATTTCGTGCCAGCCACACCCTGGAGCTAGCAAAAAAAAAAAA
AAAAAAAAAAAAAAAAAAAAGCATATGACTAAAAAAAAAAAAAAAAAAAAAAAAAAAAAA
AAAAAAAAAAAAAAAAAAAAAAAAAAAAAAAAAAAAA

SEQ ID NO.21 (SF-14-Ch.1.1-01 codon optimization sequence)

GGGAGACCCAAGCTGGCTAGCGTTTAAACTTAAGCTTGGTACCGAGCTCGGATCCAC
TAGTCCAGTGTGGTGGAATTCAGAATAAACTAGTATTCTTCTGGTCCCCACAGACTCAGAG
AGAACCCGCCACCATGTTCGTTTTTTTAGTTTTATTACCTTTAGTGTCCAGCCAGTGCGTGA
ACCTGATCACCCGGACCCAGAGCTACACCAACAGCTTCACCCGGGGCGTGTACTACCCCGA
CAAGGTGTTCCGGAGCAGCGTGCTGCACAGCACCCAGGACCTGTTCCTGCCCTTCTTCAGC
AACGTGACCTGGTTCCACGCCATCCACGTGAGCGGCACCAACGGCACCAAGCGGTTCGACA
ACCCCGTGCTGCCCTTCAACGACGGCGTGTACTTCGCCAGCACCGAGAAGAGCAACATCAT
CCGGGGCTGGATCTTCGGCACCACCCTGGACAGCAAGACCCAGAGCCTGCTGATCGTGAAC
AACGCCACCAACGTGGTGATCAAGGTGTGCGAGTTCCAGTTCTGCAACGACCCCTTCCTGG
ACGTGTACTACCACGAGAACAACAAGAGCCGGATGGAGAGCGAGCTGCGGGTGTACAGCA
GCGCCAACAACTGCACCTTCGAGTACGTGAGCCAGCCCTTCCTGATGGACCTGGAGGGCAA
GCAGGGCAACTTCAAGAACCTGCGGGAGTTCGTGTTCAAGAACATCGACGGCTACTTCAAG
ATCTACAGCAAGCACACCCCCGTGAACCTGGGCCGGGACCTGCCCCAGGGCTTCAGCGCCC
TGGAGCCCCTGGTGGACCTGCCCATCGGCATCAACATCACCCGGTTCCAGACCCTGCTGGC
CCTGCACCGGAGCTACCTGACCCCCGGCGACAGCAGCAGCAGCTGGACCGCCGGCGCCGC
CGCCTACTACGTGGGCTACCTGCAGCCCCGGACCTTCCTGCTGAAGTACAACGAGAACGGC
ACCATCACCGACGCCGTGGACTGCGCCCTGGACCCCCTGAGCGAGACCAAGTGCACCCTGA
AGAGCTTCACCGTGGAGAAGGGCATCTACCAGACCAGCAACTTCCGGGTGCAGCCCACCG
AGAGCATCGTGCGGTTCCCCAACATCACCAACCTGTGCCCCTTCCACGAGGTGTTCAACGC
CACCACCTTCGCCAGCGTGTACGCCTGGAACCGGAAGCGGATCAGCAACTGCGTGGCCGAC
TACAGCGTGCTGTACAACTTCGCCCCCTTCTTCGCCTTCAAGTGCTACGGCGTGAGCCCCAC
CAAGCTGAACGACCTGTGCTTCACCAACGTGTACGCCGACAGCTTCGTGATCCGGGGCAAC
GAGGTGAGCCAGATCGCCCCCGGCCAGACCGGCAACATCGCCGACTACAACTACAAGCTG
CCCGACGACTTCACCGGCTGCGTGATCGCCTGGAACAGCAACAAGCTGGACAGCACCGTG
AGCGGCAACTACAACTACCGGTACCGGCTGTTCCGGAAGAGCAAGCTGAAGCCCTTCGAG
CGGGACATCAGCACCGAGATCTACCAGGCCGGCAACAAGCCCTGCAACGGCGTGGCCGGC
AGCAACTGCTACTTCCCCCTGCAGAGCTACGGCTTCCGGCCCACCTACGGCGTGGGCCACC
AGCCCTACCGGGTGGTGGTGCTGAGCTTCGAGCTGCTGCACGCCCCCGCCACCGTGTGCGG
CCCCAAGAAGAGCACCAACCTGGTGAAGAACAAGTGCGTGAACTTCAACTTCAACGGCCT
GACCGGCACCGGCGTGCTGACCGAGAGCAACAAGAAGTTCCTGCCCTTCCAGCAGTTCGGC
CGGGACATCGCCGACACCACCGACGCCGTGCGGGACCCCCAGACCCTGGAGATCCTGGAC
ATCACCCCCTGCAGCTTCGGCGGCGTGAGCGTGATCACCCCCGGCACCAACACCAGCAACC
AGGTGGCCGTGCTGTACCAGGGCGTGAACTGCACCGAGGTGCCCGTGGCCATCCACGCCGA
CCAGCTGACCCCCACCTGGCGGGTGTACAGCACCGGCAGCAACGTGTTCCAGACCCGGGCC
GGCTGCCTGATCGGCGCCGAGTACGTGAACAACAGCTACGAGTGCGACATCCCCATCGGCG
CCGGCATCTGCGCCAGCTACCAGACCCAGACCAAGAGCCACCGGCGGGCCCGGAGCGTGG
CCAGCCAGAGCATCATCGCCTACACCATGTCCCTGGGCGCCGAGAACAGCGTGGCCTACAG
CAACAACAGCATCGCCATCCCCACCAACTTCACCATCAGCGTGACCACCGAGATCCTGCCC
GTGAGCATGACCAAGACCAGCGTGGACTGCACCATGTACATCTGCGGCGACAGCACCGAG
TGCAGCAACCTGCTGCTGCAGTACGGCAGCTTCTGCACCCAGCTGAACGGGCCCTGACCG
GCATCGCCGTGGAGCAGGACAAGAACACCCAGGAGGTGTTCGCCCAGGTGAAGCAGATCT
ACAAGACCCCCCCCATCAAGTACTTCGGCGGCTTCAACTTCAGCCAGATCCTGCCCGACCC
CAGCAAGCCCAGCAAGCGGAGCTTCATCGAGGACCTGCTGTTCAACAAGGTGACCCTGGCC
GACGCCGGCTTCATCAAGCAGTACGGCGACTGCCTGGGCGACATCGCCGCCCGGGACCTGA
TCTGCGCCCAGAAGTTCAACGGCCTGACCGTGCTGCCCCCCCTGCTGACCGACGAGATGAT
CGCCCAGTACACCAGCGCCCTGCTGGCCGGCACCATCACCAGCGGCTGGACCTTCGGCGCC
GGCGCCGCCCTGCAGATCCCCTTCGCCATGCAGATGGCCTACCGGTTCAACGGCATCGGCG
TGACCCAGAACGTGCTGTACGAGAACCAGAAGCTGATCGCCAACCAGTTCAACAGCGCCA
TCGGCAAGATCCAGGACAGCCTGAGCAGCACCGCCAGCGCCCTGGGCAAGCTGCAGGACG
TGGTGAACCACAACGCCCAGGCCCTGAACACCCTGGTGAAGCAGCTGAGCAGCAAGTTCG
GCGCCATCAGCAGCGTGCTGAACGACATCCTGAGCCGGCTGGACCCCCCCGAGGCCGAGG

TGCAGATCGACCGGCTGATCACCGGCCGGCTGCAGAGCCTGCAGACCTACGTGACCCAGCA
GCTGATCCGGGCCGCCGAGATCCGGGCCAGCGCCAACCTGGCCGCCACCAAGATGAGCGA
GTGCGTGCTGGGCCAGAGCAAGCGGGTGGACTTCTGCGGCAAGGGCTACCACCTGATGAG
CTTCCCCCAGAGCGCCCCCCACGGCGTGGTGTTCCTGCACGTGACCTACGTGCCCGCCCAG
GAGAAGAACTTCACCACCGCCCCCGCCATCTGCCACGACGGCAAGGCCCACTTCCCCCGGG
AGGGCGTGTTCGTGAGCAACGGCACCCACTGGTTCGTGACCCAGCGGAACTTCTACGAGCC
CCAGATCATCACCACCGACAACACCTTCGTGAGCGGCAACTGCGACGTGGTGATCGGCATC
GTGAACAACACCGTGTACGACCCCCTGCAGCCCGAGCTGGACAGCTTCAAGGAGGAGCTG
GACAAGTACTTCAAGAACCACACCAGCCCCGACGTGGACCTGGGCGACATCAGCGGCATC
AACGCCAGCGTGGTGAACATCCAGAAGGAGATCGACCGGCTGAACGAGGTGGCCAAGAAC
CTGAACGAGAGCCTGATCGACCTGCAGGAGCTGGGCAAGTACGAGCAGTACATCAAGTGG
CCCTGGTACATCTGGCTGGGCTTCATCGCCGGCCTGATCGCCATCGTGATGGTGACCATCAT
GCTGTGCTGCATGACCAGCTGCTGCAGCTGCCTGAAGGGCTGCTGCAGCTGCGGCAGCTGC
TGCAAGTTCGACGAGGACGACAGCGAGCCCGTGCTGAAGGGCGTGAAGCTGCACTACACC
TGATGACTCGAGCTGGTACTGCATGCACGCAATGCTAGCTGCCCCTTTCCCGTCCTGGGTAC
CCCGAGTCTCCCCCGACCTCGGGTCCCAGGTATGCTCCCACCTCCACCTGCCCCACTCACCA
CCTCTGCTAGTTCCAGACACCTCCCAAGCACGCAGCAATGCAGCTCAAAACGCTTAGCCTA
GCCACACCCCCACGGGAAACAGCAGTGATTAACCTTTAGCAATAAACGAAAGTTTAACTAA
GCTATACTAACCCCAGGGTTGGTCAATTTCGTGCCAGCCACACCCTGGAGCTAGCAAAAAA
AAAAAAAAAAAAAAAAAAAAAAAAAGCATATGACTAAAAAAAAAAAAAAAAAAAAAAAA
AAAAAAAAAAAAAAAAAAAAAAAAAAAAAAAAAAAAAAAAAAAA

SEQ ID NO.22 (SF-15-XBB.1.5 codon optimization sequence)

GGGAGACCCAAGCTGGCTAGCGTTTAAACTTAAGCTTGGTACCGAGCTCGGATCCAC
TAGTCCAGTGTGGTGGAATTCAGAATAAACTAGTATTCTTCTGGTCCCCACAGACTCAGAG
AGAACCCGCCACCATGTTCGTTTTTTTTAGTTTTATTACCTTTAGTGAGCAGCCAGTGCGTGA
ACCTGATCACCCGGACCCAGAGCTACACCAACAGCTTCACCCGGGGCGTGTACTACCCCGA
CAAGGTGTTCCGGAGCAGCGTGCTGCACAGCACCCAGGACCTGTTCCTGCCCTTCTTCAGC
AACGTGACCTGGTTCCACGCCATCCACGTGAGCGGCACCAACGGCACCAAGCGGTTCGACA
ACCCCGCCCTGCCCTTCAACGACGGCGTGTACTTCGCCAGCACCGAGAAGAGCAACATCAT
CCGGGGCTGGATCTTCGGCACCACCCTGGACAGCAAGACCCAGAGCCTGCTGATCGTGAAC
AACGCCACCAACGTGGTGATCAAGGTGTGCGAGTTCCAGTTCTGCAACGACCCCTTCCTGG
ACGTGTACCAGAAGAACAACAAGAGCTGGATGGAGAGCGAGTTCCGGGTGTACAGCAGCG
CCAACAACTGCACCTTCGAGTACGTGAGCCAGCCCTTCCTGATGGACCTGGAGGGCAAGGA
GGGCAACTTCAAGAACCTGCGGGAGTTCGTGTTCAAGAACATCGACGGCTACTTCAAGATC
TACAGCAAGCACACCCCCATCAACCTGGAGCGGGACCTGCCCCAGGGCTTCAGCGCCCTGG
AGCCCCTGGTGGACCTGCCCATCGGCATCAACATCACCCGGTTCCAGACCCTGCTGGCCCT
GCACCGGAGCTACCTGACCCCCGTGGACAGCAGCAGCGGCTGGACCGCCGGCGCCGCCGC
CTACTACGTGGGCTACCTGCAGCCCCGGACCTTCCTGCTGAAGTACAACGAGAACGGCACC
ATCACCGACGCCGTGGACTGCGCCCTGGACCCCCTGAGCGAGACCAAGTGCACCCTGAAG
AGCTTCACCGTGGAGAAGGGCATCTACCAGACCAGCAACTTCCGGGTGCAGCCCACCGAG
AGCATCGTGCGGTTCCCCAACATCACCAACCTGTGCCCCTTCCACGAGGTGTTCAACGCCA
CCACCTTCGCCAGCGTGTACGCCTGGAACCGGAAGCGGATCAGCAACTGCGTGGCCGACTA
CAGCGTGATCTACAACTTCGCCCCCTTCTTCGCCTTCAAGTGCTACGGCGTGAGCCCCACCA
AGCTGAACGACCTGTGCTTCACCAACGTGTACGCCGACAGCTTCGTGATCCGGGGCAACGA
GGTGAGCCAGATCGCCCCCGGCCAGACCGGCAACATCGCCGACTACAACTACAAGCTGCC
CGACGACTTCACCGGCTGCGTGATCGCCTGGAACAGCAACAAGCTGGACAGCAAGCCCAG
CGGCAACTACAACTACCTGTACCGGCTGTTCCGGAAGAGCAAGCTGAAGCCCTTCGAGCGG
GACATCAGCACCGAGATCTACCAGGCCGGCAACAAGCCCTGCAACGGCGTGGCCGGCCCC
AACTGCTACAGCCCCCTGCAGAGCTACGGCTTCCGGCCCACCTACGGCGTGGGCCACCAGC
CCTACCGGGTGGTGGTGCTGAGCTTCGAGCTGCTGCACGCCCCCGCCACCGTGTGCGGCCC
CAAGAAGAGCACCAACCTGGTGAAGAACAAGTGCGTGAACTTCAACTTCAACGGCCTGAC
CGGCACCGGCGTGCTGACCGAGAGCAACAAGAAGTTCCTGCCCTTCCAGCAGTTCGGCCGG
GACATCGCCGACACCACCGACGCCGTGCGGGACCCCCAGACCCTGGAGATCCTGGACATC
ACCCCCTGCAGCTTCGGCGGCGTGAGCGTGATCACCCCCGGCACCAACACCAGCAACCAGG
TGGCCGTGCTGTACCAGGGCGTGAACTGCACCGAGGTGCCCGTGGCCATCCACGCCGACCA
GCTGACCCCCACCTGGCGGGTGTACAGCACCGGCAGCAACGTGTTCCAGACCCGGGCCGGC
TGCCTGATCGGCGCCGAGTACGTGAACAACAGCTACGAGTGCGACATCCCCATCGGCGCCG
GCATCTGCGCCAGCTACCAGACCCAGACCAAGAGCCACCGGCGGGCCCGGAGCGTGGCCA
GCCAGAGCATCATCGCCTACACCATGAGCCTGGGCGCCGAGAACAGCGTGGCCTACAGCA
ACAACGCTACGCCATCCCCACCAACTTCACCATCTCCGTGACCACCGAGATCCTGCCCGT
GAGCATGACCAAGACCAGCGTGGACTGCACCATGTACATCTGCGGCGACAGCACCGAGTG
CAGCAACCTGCTGCTGCAGTACGGCAGCTTCTGCACCCAGCTGAAGCGGGCCCTGACCGGC
ATCGCCGTGGAGCAGGACAAGAACACCCAGGAGGTGTTCGCCCAGGTGAAGCAGATCTAC
AAGACCCCCCCCATCAAGTACTTCGGCGGCTTCAACTTCAGCCAGATCCTGCCCGACCCCA
GCAAGCCCAGCAAGCGGAGCTTCATCGAGGACCTGCTGTTCAACAAGGTGACCCTGGCCG
ACGCCGGCTTCATCAAGCAGTACGGCGACTGCCTGGGCGACATCGCCGCCCGGGACCTGAT
CTGCGCCCAGAAGTTCAACGGCCTGACCGTGCTGCCCCCCCTGCTGACCGACGAGATGATC
GCCCAGTACACCAGCGCCCTGCTGGCCGGCACCATCACCAGCGGCTGGACCTTCGGCGCCG
GCGCCGCCCTGCAGATCCCCTTCGCCATGCAGATGGCCTACCGGTTCAACGGCATCGGCGT
GACCCAGAACGTGCTGTACGAGAACCAGAAGCTGATCGCCAACCAGTTCAACAGCGCCAT

CGGCAAGATCCAGGACAGCCTGAGCAGCACCGCCAGCGCCCTGGGCAAGCTGCAGGACGT
GGTGAACCACAACGCCCAGGCCCTGAACACCCTGGTGAAGCAGCTGAGCAGCAAGTTCGG
CGCCATCAGCAGCGTGCTGAACGACATCCTGAGCCGGCTGGACCCCCCCGAGGCCGAGGT
GCAGATCGACCGCCTGATCACCGGCCGGCTGCAGAGCCTGCAGACCTACGTGACCCAGCA
GCTGATCCGGGCCGCCGAGATCCGGGCCAGCGCCAACCTGGCCGCCACCAAGATGAGCGA
GTGCGTGCTGGGCCAGAGCAAGCGGGTGGACTTCTGCGGCAAGGGCTACCACCTGATGAG
CTTCCCCCAGAGCGCCCCCCACGGCGTGGTGTTCCTGCACGTGACCTACGTGCCCGCCCAG
GAGAAGAACTTCACCACCGCCCCCGCCATCTGCCACGACGGCAAGGCCCACTTCCCCCGGG
AGGGCGTGTTCGTGAGCAACGGCACCCACTGGTTCGTGACCCAGCGGAACTTCTACGAGCC
CCAGATCATCACCACCGACAACACCTTCGTGAGCGGCAACTGCGACGTGGTGATCGGCATC
GTGAACAACACCGTGTACGACCCCCTGCAGCCCGAGCTGGACAGCTTCAAGGAGGAGCTG
GACAAGTACTTCAAGAACCACACCAGCCCCGACGTGGACCTGGGCGACATCAGCGGCATC
AACGCCAGCGTGGTGAACATCCAGAAGGAGATCGACCGGCTGAACGAGGTGGCCAAGAAC
CTGAACGAGAGCCTGATCGACCTGCAGGAGCTGGGCAAGTACGAGCAGTACATCAAGTGG
CCCTGGTACATCTGGCTGGGCTTCATCGCCGGCCTGATCGCCATCGTGATGGTGACCATCAT
GCTGTGCTGCATGACCAGCTGCTGCAGCTGCCTGAAGGGCTGCTGCAGCTGCGGCAGCTGC
TGCAAGTTCGACGAGGACGACAGCGAGCCCGTGCTGAAGGGCGTGAAGCTGCACTACACC
TGATGACTCGAGCTGGTACTGCATGCACGCAATGCTAGCTGCCCCTTTCCCGTCCTGGGTAC
CCCGAGTCTCCCCCGACCTCGGGTCCCAGGTATGCTCCCACCTCCACCTGCCCCACTCACCA
CCTCTGCTAGTTCCAGACACCTCCCAAGCACGCAGCAATGCAGCTCAAAACGCTTAGCCTA
GCCACACCCCCACGGGAAACAGCAGTGATTAACCTTTAGCAATAAACGAAAGTTTAACTAA
GCTATACTAACCCCAGGGTTGGTCAATTTCGTGCCCAGCCACACCCTGGAGCTAGCAAAAA
AAAAAAAAAAAAAAAAAAAAAAAAGCATATGACTAAAAAAAAAAAAAAAAAAAAAAAAAA
AAAAAAAAAAAAAAAAAAAAAAAAAAAAAAAAAAAAAAAAAAAA

SEQ ID NO.23 (SF-16-XBB.1.16 codon optimization sequence)

GGGAGACCCAAGCTGGCTAGCGTTTAAACTTAAGCTTGGTACCGAGCTCGGATCCAC
TAGTCCAGTGTGGTGGAATTCAGAATAAACTAGTATTCTTCTGGTCCCCACAGACTCAGAG
AGAACCCGCCACCATGTTCGTTTTTTTAGTTTTATTACCTTTAGTGAGCAGCCAGTGCGTGA
ACCTGATCACCCGGACCCAGAGCTACACCAACAGCTTCACCCGGGGCGTGTACTACCCCGA
CAAGGTGTTCCGGAGCAGCGTGCTGCACAGCACCCAGGACCTGTTCCTGCCCTTCTTCAGC
AACGTGACCTGGTTCCACGCCATCCACGTGAGCGGCACCAACGGCACCAAGCGGTTCGACA
ACCCCGCCCTGCCCTTCAACGACGGCGTGTACTTCGCCAGCACCGAGAAGAGCAACATCAT
CCGGGGCTGGATCTTCGGCACCACCCTGGACAGCAAGACCCAGAGCCTGCTGATCGTGAAC
AACGCCACCAACGTGGTGATCAAGGTGTGCGAGTTCCAGTTCTGCAACGACCCCTTCCTGG
ACGTGTACCAGAAGAACAACAAGAGCTGGATGGAGAGCGAGTTCCGGGTGTACAGCAGCG
CCAACAACTGCACCTTCGAGTACGTGAGCCAGCCCTTCCTGATGGACCTGGTGGGCAAGGA
GGGCAACTTCAAGAACCTGCGGGAGTTCGTGTTCAAGAACATCGACGGCTACTTCAAGATC
TACAGCAAGCACACCCCCATCAACCTGGAGCGGGACCTGCCCCAGGGCTTCAGCGCCCTGG
AGCCCCTGGTGGACCTGCCCATCGGCATCAACATCACCCGGTTCCAGACCCTGCTGGCCCT
GCACCGGAGCTACCTGACCCCCGTGGACAGCAGCAGCGGCTGGACCGCCGGCGCCGCCGC
CTACTACGTGGGCTACCTGCAGCCCCGGACCTTCCTGCTGAAGTACAACGAGAACGGCACC
ATCACCGACGCCGTGGACTGCGCCCTGGACCCCCTGAGCGAGACCAAGTGCACCCTGAAG
AGCTTCACCGTGGAGAAGGGCATCTACCAGACCAGCAACTTCCGGGTGCAGCCCACCGAG
AGCATCGTGCGGTTCCCCAACATCACCAACCTGTGCCCCTTCCACGAGGTGTTCAACGCCA
CCACCTTCGCCAGCGTGTACGCCTGGAACCGGAAGCGGATCAGCAACTGCGTGGCCGACTA
CAGCGTGATCTACAACTTCGCCCCCTTCTTCGCCTTCAAGTGCTACGGCGTGAGCCCCACCA
AGCTGAACGACCTGTGCTTCACCAACGTGTACGCCGACAGCTTCGTGATCCGGGGCGACGA
GGTGAGCCAGATCGCCCCCGGCCAGACCGGCAACATCGCCGACTACAACTACAAGCTGCC
CGACGACTTCACCGGCTGCGTGATCGCCTGGAACAGCAACAAGCTGGACAGCAAGCCCAG
CGGCAACTACAACTACCTGTACCGGCTGTTCCGGAAGAGCAAGCTGAAGCCCTTCGAGCGG
GACATCAGCACCGAGATCTACCAGGCCGGCAACCGGCCCTGCAACGGCGTGGCCGGCCCC
AACTGCTACAGCCCCCTGCAGAGCTACGGCTTCCGGCCCACCTACGGCGTGGGCCACCAGC
CCTACCGGGTGGTGGTGCTGAGCTTCGAGCTGCTGCACGCCCCCGCCACCGTGTGCGGCCC
CAAGAAGAGCACCAACCTGGTGAAGAACAAGTGCGTGAACTTCAACTTCAACGGCCTGAC
CGGCACCGGCGTGCTGACCGAGAGCAACAAGAAGTTCCTGCCCTTCCAGCAGTTCGGCCGG
GACATCGCCGACACCACCGACGCCGTGCGGGACCCCAGACCCTGGAGATCCTGGACATC
ACCCCCTGCAGCTTCGGCGGCGTGAGCGTGATCACCCCCGGCACCAACACCAGCAACCAGG
TGGCCGTGCTGTACCAGGGCGTGAACTGCACCGAGGTGCCCGTGGCCATCCACGCCGACCA
GCTGACCCCCACCTGGCGGGTGTACAGCACCGGCAGCAACGTGTTCCAGACCCGGGCCGGC
TGCCTGATCGGCGCCGAGTACGTGAACAACAGCTACGAGTGCGACATCCCCATCGGCGCCG
GCATCTGCGCCAGCTACCAGACCCAGACCAAGAGCCACCGGCGGGCCCGGAGCGTGGCCA
GCCAGAGCATCATCGCCTACACCATGAGCCTGGGCGCCGAGAACAGCGTGGCCTACAGCA
ACAACAGCATCGCCATCCCCACCAACTTCACCATCTCCGTGACCACCGAGATCCTGCCCGT
GAGCATGACCAAGACCAGCGTGGACTGCACCATGTACATCTGCGGCGACAGCACCGAGTG
CAGCAACCTGCTGCTGCAGTACGGCAGCTTCTGCACCCAGCTGAAGCGGGCCCTGACCGGC
ATCGCCGTGGAGCAGGACAAGAACACCCAGGAGGTGTTCGCCCAGGTGAAGCAGATCTAC
AAGACCCCCCCCATCAAGTACTTCGGCGGCTTCAACTTCAGCCAGATCCTGCCCGACCCCA
GCAAGCCCAGCAAGCGGAGCTTCATCGAGGACCTGCTGTTCAACAAGGTGACCCTGGCCG
ACGCCGGCTTCATCAAGCAGTACGGCGACTGCCTGGGCGACATCGCCGCCCGGGACCTGAT

CTGCGCCCAGAAGTTCAACGGCCTGACCGTGCTGCCCCCCCTGCTGACCGACGAGATGATC
GCCCAGTACACCAGCGCCCTGCTGGCCGGCACCATCACCAGCGGCTGGACCTTCGGCGCCG
GCGCCGCCCTGCAGATCCCCTTCGCCATGCAGATGGCCTACCGGTTCAACGGCATCGGCGT
GACCCAGAACGTGCTGTACGAGAACCAGAAGCTGATCGCCAACCAGTTCAACAGCGCCAT
CGGCAAGATCCAGGACAGCCTGAGCAGCACCGCCAGCGCCCTGGGCAAGCTGCAGGACGT
GGTGAACCACAACGCCCAGGCCCTGAACACCCTGGTGAAGCAGCTGAGCAGCAAGTTCGG
CGCCATCAGCAGCGTGCTGAACGACATCCTGAGCCGGCTGGACCCCCCCGAGGCCGAGGT
GCAGATCGACCGCCTGATCACCGGCCGGCTGCAGAGCCTGCAGACCTACGTGACCCAGCA
GCTGATCCGGGCCGCCGAGATCCGGGCCAGCGCCAACCTGGCCGCCACCAAGATGAGCGA
GTGCGTGCTGGGCCAGAGCAAGCGGGTGGACTTCTGCGGCAAGGGCTACCACCTGATGAG
CTTCCCCCAGAGCGCCCCCCACGGCGTGGTGTTCCTGCACGTGACCTACGTGCCCGCCCAG
GAGAAGAACTTCACCACCGCCCCCGCCATCTGCCACGACGGCAAGGCCCACTTCCCCCGGG
AGGGCGTGTTCGTGAGCAACGGCACCCACTGGTTCGTGACCCAGCGGAACTTCTACGAGCC
CCAGATCATCACCACCGACAACACCTTCGTGAGCGGCAACTGCGACGTGGTGATCGGCATC
GTGAACAACACCGTGTACGACCCCCTGCAGCCCGAGCTGGACAGCTTCAAGGAGGAGCTG
GACAAGTACTTCAAGAACCACACCAGCCCCGACGTGGACCTGGGCGACATCAGCGGCATC
AACGCCAGCGTGGTGAACATCCAGAAGGAGATCGACCGGCTGAACGAGGTGGCCAAGAAC
CTGAACGAGAGCCTGATCGACCTGCAGGAGCTGGGCAAGTACGAGCAGTACATCAAGTGG
CCCTGGTACATCTGGCTGGGCTTCATCGCCGGCCTGATCGCCATCGTGATGGTGACCATCAT
GCTGTGCTGCATGACCAGCTGCTGCAGCTGCCTGAAGGGCTGCTGCAGCTGCGGCAGCTGC
TGCAAGTTCGACGAGGACGACAGCGAGCCCGTGCTGAAGGGCGTGAAGCTGCACTACACC
TGATGACTCGAGCTGGTACTGCATGCACGCAATGCTAGCTGCCCCTTTCCCGTCCTGGGTAC
CCCGAGTCTCCCCCGACCTCGGGTCCCAGGTATGCTCCCACCTCCACCTGCCCCACTCACCA
CCTCTGCTAGTTCCAGACACCTCCCAAGCACGCAGCAATGCAGCTCAAAACGCTTAGCCTA
GCCACACCCCCACGGGAAACAGCAGTGATTAACCTTTAGCAATAAACGAAAGTTTAACTAA
GCTATACTAACCCCAGGGTTGGTCAATTTCGTGCCAGCCACACCCTGGAGCTAGCAAAAAA
AAAAAAAAAAAAAAAAAAAAAAAAAGCATATGACTAAAAAAAAAAAAAAAAAAAAAAAAAA
AAAAAAAAAAAAAAAAAAAAAAAAAAAAAAAAAAAAAAAAAAA

SEQ ID NO.24 (SF-17-BA.5 codon optimization sequence)

GGGAGACCCAAGCTGGCTAGCGTTTAAACTTAAGCTTGGTACCGAGCTCGGATCCAC
TAGTCCAGTGTGGTGGAATTCAGAATAAACTAGTATTCTTCTGGTCCCCACAGACTCAGAG
AGAACCCGCCACCATGTTCGTTTTTTTAGTTTTATTACCTTTAGTGAGCTCCCAGTGCGTGA
ACCTGATCACCCGGACCCAGAGCTACACCAACAGCTTCACCCGGGGCGTGTACTACCCCGA
CAAGGTGTTCCGGAGCAGCGTGCTGCACAGCACCCAGGACCTGTTCCTGCCCTTCTTCAGC
AACGTGACCTGGTTCCACGCCATCAGCGGCACCAACGGCACCAAGCGGTTCGACAACCCCG
TGCTGCCCTTCAACGACGGCGTGTACTTCGCCAGCACCGAGAAGAGCAACATCATCCGGGG
CTGGATCTTCGGCACCACCCTGGACAGCAAGACCCAGAGCCTGCTGATCGTGAACAACGCC
ACCAACGTGGTGATCAAGGTGTGCGAGTTCCAGTTCTGCAACGACCCCTTCCTGGACGTGT
ACTACCACAAGAACAACAAGAGCTGGATGGAGAGCGAGTTCCGGGTGTACAGCAGCGCCA
ACAACTGCACCTTCGAGTACGTGAGCCAGCCCTTCCTGATGGACCTGGAGGGCAAGCAGGG
CAACTTCAAGAACCTGCGGGAGTTCGTGTTCAAGAACATCGACGGCTACTTCAAGATCTAC
AGCAAGCACACCCCCATCAACCTGGGCCGGGACCTGCCCCAGGGCTTCAGCGCCCTGGAGC
CCCTGGTGGACCTGCCCATCGGCATCAACATCACCCGGTTCCAGACCCTGCTGGCCCTGCA
CCGGAGCTACCTGACCCCCGGCGACAGCAGCAGCGGCTGGACCGCCGGCGCCGCCGCCTA
CTACGTGGGCTACCTGCAGCCCCGGACCTTCCTGCTGAAGTACAACGAGAACGGCACCATC
ACCGACGCCGTGGACTGCGCCCTGGACCCCCTGAGCGAGACCAAGTGCACCCTGAAGAGC
TTCACCGTGGAGAAGGGCATCTACCAGACCAGCAACTTCCGGGTGCAGCCCACCGAGAGC
ATCGTGCGGTTCCCCAACATCACCAACCTGTGCCCCTTCGACGAGGTGTTCAACGCCACCC
GGTTCGCCAGCGTGTACGCCTGGAACCGGAAGCGGATCAGCAACTGCGTGGCCGACTACA
GCGTGCTGTACAACTTCGCCCCCTTCTTCGCCTTCAAGTGCTACGGCGTGAGCCCCACCAAG
CTGAACGACCTGTGCTTCACCAACGTGTACGCCGACAGCTTCGTGATCCGGGGCAACGAGG
TGAGCCAGATCGCCCCCGGCCAGACCGGCAACATCGCCGACTACAACTACAAGCTGCCCG
ACGACTTCACCGGCTGCGTGATCGCCTGGAACAGCAACAAGCTGGACAGCAAGGTGGGCG
GCAACTACAACTACCGGTACCGGCTGTTCCGGAAGAGCAACCTGAAGCCCTTCGAGCGGG
ACATCAGCACCGAGATCTACCAGGCCGGCAACAAGCCCTGCAACGGCGTGGCCGGCGTGA
ACTGCTACTTCCCCCTGCAGAGCTACGGCTTCCGGCCCACCTACGGCGTGGGCCACCAGCC
CTACCGGGTGGTGGTGCTGAGCTTCGAGCTGCTGCACGCCCCCGCCACCGTGTGCGGCCCC
AAGAAGAGCACCAACCTGGTGAAGAACAAGTGCGTGAACTTCAACTTCAACGGCCTGACC
GGCACCGGCGTGCTGACCGAGAGCAACAAGAAGTTCCTGCCCTTCCAGCAGTTCGGCCGGG
ACATCGCCGACACCACCGACGCCGTGCGGGACCCCCAGACCCTGGAGATCCTGGACATCAC
CCCCTGCAGCTTCGGCGGCGTGAGCGTGATCACCCCCGGCACCAACACCAGCAACCAGGTG
GCCGTGCTGTACCAGGGCGTGAACTGCACCGAGGTGCCCGTGGCCATCCACGCCGACCAGC
TGACCCCCACCTGGCGGGTGTACAGCACCGGCAGCAACGTGTTCCAGACCCGGGCCGGCTG
CCTGATCGGCGCCGAGTACGTGAACAACAGCTACGAGTGCGACATCCCCATCGGCGCCGGC
ATCTGCGCCTCCTACCAGACCCAGACCAAGAGCCACCGGCGGGCCCGGAGCGTGGCCAGC
CAGAGCATCATCGCCTACACCATGAGCCTGGGCGCCGAGAACAGCGTGGCCTACAGCAAC
AACAGCATCGCCATCCCCACCAACTTCACCATCAGCGTGACCACCGAGATCCTGCCCGTGA
GCATGACCAAGACCAGCGTGGACTGCACCATGTACATCTGCGGCGACAGCACCGAGTGCA
GCAACCTGCTGCTGCAGTACGGCAGCTTCTGCACCCAGCTGAAGCGGGCCCCTGACCGGCAT

CGCCGTGGAGCAGGACAAGAACACCCAGGAGGTGTTCGCCCAGGTGAAGCAGATCTACAA
GACCCCCCCCATCAAGTACTTCGGCGGCTTCAACTTCAGCCAGATCCTGCCCGACCCCAGC
AAGCCCAGCAAGCGGAGCTTCATCGAGGACCTGCTGTTCAACAAGGTGACCCTGGCCGAC
GCCGGCTTCATCAAGCAGTACGGCGACTGCCTGGGCGACATCGCCGCCCGGGACCTGATCT
GCGCCCAGAAGTTCAACGGCCTGACCGTGCTGCCCCCCCTGCTGACCGACGAGATGATCGC
CCAGTACACCAGCGCCCTGCTGGCCGGCACCATCACCAGCGGCTGGACCTTCGGCGCCGGC
GCCGCCCTGCAGATCCCCTTCGCCATGCAGATGGCCTACCGGTTCAACGGCATCGGCGTGA
CCCAGAACGTGCTGTACGAGAACCAGAAGCTGATCGCCAACCAGTTCAACAGCGCCATCG
GCAAGATCCAGGACAGCCTGAGCAGCACCGCCAGCGCCCTGGGCAAGCTGCAGGACGTGG
TGAACCACAACGCCCAGGCCCTGAACACCCTGGTGAAGCAGCTGAGCAGCAAGTTCGGCG
CCATCAGCAGCGTGCTGAACGACATCCTGAGCCGGCTGGACCCCCCCGAGGCCGAGGTGC
AGATCGACCGGCTGATCACCGGCCGGCTGCAGAGCCTGCAGACCTACGTGACCCAGCAGCT
GATCCGGGCCGCCGAGATCCGGGCCAGCGCCAACCTGGCCGCCACCAAGATGAGCGAGTG
CGTGCTGGGCCAGAGCAAGCGGGTGGACTTCTGCGGCAAGGGCTACCACCTGATGAGCTTC
CCCCAGAGCGCCCCCCACGGCGTGGTGTTCCTGCACGTGACCTACGTGCCCGCCCAGGAGA
AGAACTTCACCACCGCCCCCGCCATCTGCCACGACGGCAAGGCCCACTTCCCCCGGGAGGG
CGTGTTCGTGAGCAACGGCACCCACTGGTTCGTGACCCAGCGGAACTTCTACGAGCCCCAG
ATCATCACCACCGACAACACCTTCGTGAGCGGCAACTGCGACGTGGTGATCGGCATCGTGA
ACAACACCGTGTACGACCCCCTGCAGCCCGAGCTGGACAGCTTCAAGGAGGAGCTGGACA
AGTACTTCAAGAACCACACCAGCCCCGACGTGGACCTGGGCGACATCAGCGGCATCAACG
CCAGCGTGGTGAACATCCAGAAGGAGATCGACCGGCTGAACGAGGTGGCCAAGAACCTGA
ACGAGAGCCTGATCGACCTGCAGGAGCTGGGCAAGTACGAGCAGTACATCAAGTGGCCCT
GGTACATCTGGCTGGGCTTCATCGCCGGCCTGATCGCCATCGTGATGGTGACCATCATGCT
GTGCTGCATGACCAGCTGCTGCAGCTGCCTGAAGGGCTGCTGCAGCTGCGGCAGCTGCTGC
AAGTTCGACGAGGACGACAGCGAGCCCGTGCTGAAGGGCGTGAAGCTGCACTACACCTGA
TGAGCTCGCTTTCTTGCTGTCCAATTTCTATTAAAGGTTCCTTTGTTCCCTAAGTCCAACTAC
TAAACTGGGGGATATTATGAAGGGCCTTGAGCATCTGGATTCTGCCTAATAAAAAACATTT
ATTTTCATTGCAATTGCCATGTGTATGTGGGTTCGCCCACATACTCTGATGATCCCCAATCG
TGGCGTGTCGGCCTGCTTCGGCAGGCACTGGCGCCGGGATCATTCATGGCAAGCTGGAGCC
TCGGTGGCCATGCTTCTTGCCCCTTGGGCCTCCCCCCAGCCCCTCCTCCCCTTCCTGCACCC
GTACCCCCGTGGTCTTTGAATAAAGTCTGAGTGGGCGGCACTCGAGCTGGTACTGCATGCA
CGCAATGCTAGCTGCCCCTTTCCCGTCCTGGGTACCCCGAGTCTCCCCCGACCTCGGGTCCC
AGGTATGCTCCCACCTCCACCTGCCCCACTCACCACCTCTGCTAGTTCCAGACACCTCCAAA
AAAAAAAAAAAAAAAAAAAAAAAAAGCATATGACTAAAAAAAAAAAAAAAAAAAAAAA
AAAAAAAAAAAAAAAAAAAAAAAAAAAAAAAAAAAAAAAAAAAAAAAAA

SEQ ID NO.25 (SF-18-BA.5 codon optimization sequence)

GGGAGACCCAAGCTGGCTAGCGTTTAAACTTAAGCTTGGTACCGAGCTCGGATCCAC
TAGTCCAGTGTGGTGGAATTCAGAATAAACTAGTATTCTTCTGGTCCCCACAGACTCAGAG
AGAACCCGCCACCATGTTCGTTTTTTTTAGTTTTATTACCTTTAGTGAGCTCCCAGTGCGTGA
ACCTGATCACCCGGACCCAGAGCTACACCAACAGCTTCACCCGGGGCGTGTACTACCCCGA
CAAGGTGTTCCGGAGCAGCGTGCTGCACAGCACCCAGGACCTGTTCCTGCCCTTCTTCAGC
AACGTGACCTGGTTCCACGCCATCAGCGGCACCAACGGCACCAAGCGGTTCGACAACCCCG
TGCTGCCCTTCAACGACGGCGTGTACTTCGCCAGCACCGAGAAGAGCAACATCATCCGGGG
CTGGATCTTCGGCACCACCCTGGACAGCAAGACCCAGAGCCTGCTGATCGTGAACAACGCC
ACCAACGTGGTGATCAAGGTGTGCGAGTTCCAGTTCTGCAACGACCCCTTCCTGGACGTGT
ACTACCACAAGAACAACAAGAGCTGGATGGAGAGCGAGTTCCGGGTGTACAGCAGCGCCA
ACAACTGCACCTTCGAGTACGTGAGCCAGCCCTTCCTGATGGACCTGGAGGGCAAGCAGGG
CAACTTCAAGAACCTGCGGGAGTTCGTGTTCAAGAACATCGACGGCTACTTCAAGATCTAC
AGCAAGCACACCCCCATCAACCTGGGCCGGGACCTGCCCCAGGGCTTCAGCGCCCTGGAGC
CCCTGGTGGACCTGCCCATCGGCATCAACATCACCCGGTTCCAGACCCTGCTGGCCCTGCA
CCGGAGCTACCTGACCCCCGGCGACAGCAGCAGCGGCTGGACCGCCGGCGCCGCCGCCTA
CTACGTGGGCTACCTGCAGCCCCGGACCTTCCTGCTGAAGTACAACGAGAACGGCACCATC
ACCGACGCCGTGGACTGCGCCCTGGACCCCCTGAGCGAGACCAAGTGCACCCTGAAGAGC
TTCACCGTGGAGAAGGGCATCTACCAGACCAGCAACTTCCGGGTGCAGCCCACCGAGAGC
ATCGTGCGGTTCCCCAACATCACCAACCTGTGCCCCTTCGACGAGGTGTTCAACGCCACCC
GGTTCGCCAGCGTGTACGCCTGGAACCGGAAGCGGATCAGCAACTGCGTGGCCGACTACA
GCGTGCTGTACAACTTCGCCCCCTTCTTCGCCTTCAAGTGCTACGGCGTGAGCCCCACCAAG
CTGAACGACCTGTGCTTCACCAACGTGTACGCCGACAGCTTCGTGATCCGGGGCAACGAGG
TGAGCCAGATCGCCCCCGGCCAGACCGGCAACATCGCCGACTACAACTACAAGCTGCCCG
ACGACTTCACCGGCTGCGTGATCGCCTGGAACAGCAACAAGCTGGACAGCAAGGTGGGCG
GCAACTACAACTACCGGTACCGGCTGTTCCGGAAGAGCAACCTGAAGCCCTTCGAGCGGG
ACATCAGCACCGAGATCTACCAGGCCGGCAACAAGCCCTGCAACGGCGTGGCCGGCGTGA
ACTGCTACTTCCCCCTGCAGAGCTACGGCTTCCGGCCCACCTACGGCGTGGGCCACCAGCC
CTACCGGGTGGTGGTGCTGAGCTTCGAGCTGCTGCACGCCCCCGCCACCGTGTGCGGCCCC
AAGAAGAGCACCAACCTGGTGAAGAACAAGTGCGTGAACTTCAACTTCAACGGCCTGACC
GGCACCGGCGTGCTGACCGAGAGCAACAAGAAGTTCCTGCCCTTCCAGCAGTTCGGCCGGG
ACATCGCCGACACCACCGACGCCGTGCGGGACCCCCAGACCCTGGAGATCCTGGACATCAC
CCCCTGCAGCTTCGGCGGCGTGAGCGTGATCACCCCCGGCACCAACACCAGCAACCAGGTG
GCCGTGCTGTACCAGGGCGTGAACTGCACCGAGGTGCCCGTGGCCATCCACGCCGACCAGC
TGACCCCCACCTGGCGGGTGTACAGCACCGGCAGCAACGTGTTCCAGACCCGGGCCGGCTG

CCTGATCGGCGCCGAGTACGTGAACAACAGCTACGAGTGCGACATCCCCATCGGCGCCGGC
ATCTGCGCCTCCTACCAGACCCAGACCAAGAGCCACCGGCGGGCCCGGAGCGTGGCCAGC
CAGAGCATCATCGCCTACACCATGAGCCTGGGCGCCGAGAACAGCGTGGCCTACAGCAAC
AACAGCATCGCCATCCCCACCAACTTCACCATCAGCGTGACCACCGAGATCCTGCCCGTGA
GCATGACCAAGACCAGCGTGGACTGCACCATGTACATCTGCGGCGACAGCACCGAGTGCA
GCAACCTGCTGCTGCAGTACGGCAGCTTCTGCACCCAGCTGAAGCGGGCCCTGACCGGCAT
CGCCGTGGAGCAGGACAAGAACACCCAGGAGGTGTTCGCCCAGGTGAAGCAGATCTACAA
GACCCCCCCCATCAAGTACTTCGGCGGCTTCAACTTCAGCCAGATCCTGCCCGACCCCAGC
AAGCCCAGCAAGCGGAGCTTCATCGAGGACCTGCTGTTCAACAAGGTGACCCTGGCCGAC
GCCGGCTTCATCAAGCAGTACGGCGACTGCCTGGGCGACATCGCCGCCCGGGACCTGATCT
GCGCCCAGAAGTTCAACGGCCTGACCGTGCTGCCCCCCCTGCTGACCGACGAGATGATCGC
CCAGTACACCAGCGCCCTGCTGGCCGGCACCATCACCAGCGGCTGGACCTTCGGCGCCGGC
GCCGCCCTGCAGATCCCCTTCGCCATGCAGATGGCCTACCGGTTCAACGGCATCGGCGTGA
CCCAGAACGTGCTGTACGAGAACCAGAAGCTGATCGCCAACCAGTTCAACAGCGCCATCG
GCAAGATCCAGGACAGCCTGAGCAGCACCGCCAGCGCCCTGGGCAAGCTGCAGGACGTGG
TGAACCACAACGCCCAGGCCCTGAACACCCTGGTGAAGCAGCTGAGCAGCAAGTTCGGCG
CCATCAGCAGCGTGCTGAACGACATCCTGAGCCGGCTGGACCCCCCCGAGGCCGAGGTGC
AGATCGACCGGCTGATCACCGGCCGGCTGCAGAGCCTGCAGACCTACGTGACCCAGCAGCT
GATCCGGGCCGCCGAGATCCGGGCCAGCGCCAACCTGGCCGCCACCAAGATGAGCGAGTG
CGTGCTGGGCCAGAGCAAGCGGGTGGACTTCTGCGGCAAGGGCTACCACCTGATGAGCTTC
CCCCAGAGCGCCCCCCACGGCGTGGTGTTCCTGCACGTGACCTACGTGCCCGCCCAGGAGA
AGAACTTCACCACCGCCCCCGCCATCTGCCACGACGGCAAGGCCCACTTCCCCCGGGAGGG
CGTGTTCGTGAGCAACGGCACCCACTGGTTCGTGACCCAGCGGAACTTCTACGAGCCCCAG
ATCATCACCACCGACAACACCTTCGTGAGCGGCAACTGCGACGTGGTGATCGGCATCGTGA
ACAACACCGTGTACGACCCCCTGCAGCCCGAGCTGGACAGCTTCAAGGAGGAGCTGGACA
AGTACTTCAAGAACCACACCAGCCCCGACGTGGACCTGGGCGACATCAGCGGCATCAACG
CCAGCGTGGTGAACATCCAGAAGGAGATCGACCGGCTGAACGAGGTGGCCAAGAACCTGA
ACGAGAGCCTGATCGACCTGCAGGAGCTGGGCAAGTACGAGCAGTACATCAAGTGGCCCT
GGTACATCTGGCTGGGCTTCATCGCCGGCCTGATCGCCATCGTGATGGTGACCATCATGCT
GTGCTGCATGACCAGCTGCTGCAGCTGCCTGAAGGGCTGCTGCAGCTGCGGCAGCTGCTGC
AAGTTCGACGAGGACGACAGCGAGCCCGTGCTGAAGGGCGTGAAGCTGCACTACACCTGA
TGAGCTCGCTTTCTTGCTGTCCAATTTCTATTAAAGGTTCCTTTGTTCCCTAAGTCCAACTAC
TAAACTGGGGGATATTATGAAGGGCCTTGAGCATCTGGATTCTGCCTAATAAAAAACATTT
ATTTTCATTGCAATTGCCATGTGTATGTGGGTTCGCCCACATACTCTGATGATCCCCAATCG
TGGCGTGTCGGCCTGCTTCGGCAGGCACTGGCGCCGGGATCATTCATGGCAACTCGAGCTG
GTACTGCATGCACGCAATGCTAGCTGCCCCTTTCCCGTCCTGGGTACCCCGAGTCTCCCCCG
ACCTCGGGTCCCAGGTATGCTCCCACCTCCACCTGCCCCACTCACCACCTCTGCTAGTTCCA
GACACCTCCAAAAAAAAAAAAAAAAAAAAAAAAAAAAAAAAAGCATATGACTAAAAAAAAA
AAAAAAAAAAAAAAAAAAAAAAAAAAAAAAAAAAAAAAAAAAAAAAAAAAAAAAAAAAAA
AA

SEQ ID NO.26 (SF-19-BA.5 codon optimization sequence)

GGGAGACCCAAGCTGGCTAGCGTTTAAACTTAAGCTTGGTACCGAGCTCGGATCCAC
TAGTCCAGTGTGGTGGAATTCAGAATAAACTAGTATTCTTCTGGTCCCCACAGACTCAGAG
AGAACCCGCCACCATGTTCGTTTTTTTTAGTTTTATTACCTTTAGTGAGCTCCCAGTGCGTGA
ACCTGATCACCCGGACCCAGAGCTACACCAACAGCTTCACCCGGGGCGTGTACTACCCCGA
CAAGGTGTTCCGGAGCAGCGTGCTGCACAGCACCCAGGACCTGTTCCTGCCCTTCTTCAGC
AACGTGACCTGGTTCCACGCCATCAGCGGCACCAACGGCACCAAGCGGTTCGACAACCCCG
TGCTGCCCTTCAACGACGGCGTGTACTTCGCCAGCACCGAGAAGAGCAACATCATCCGGGG
CTGGATCTTCGGCACCACCCTGGACAGCAAGACCCAGAGCCTGCTGATCGTGAACAACGCC
ACCAACGTGGTGATCAAGGTGTGCGAGTTCCAGTTCTGCAACGACCCCTTCCTGGACGTGT
ACTACCACAAGAACAACAAGAGCTGGATGGAGAGCGAGTTCCGGGTGTACAGCAGCGCCA
ACAACTGCACCTTCGAGTACGTGAGCCAGCCCTTCCTGATGGACCTGGAGGGCAAGCAGGG
CAACTTCAAGAACCTGCGGGAGTTCGTGTTCAAGAACATCGACGGCTACTTCAAGATCTAC
AGCAAGCACACCCCCATCAACCTGGGCCGGGACCTGCCCCAGGGCTTCAGCGCCCTGGAGC
CCCTGGTGGACCTGCCCATCGGCATCAACATCACCCGGTTCCAGACCCTGCTGGCCCTGCA
CCGGAGCTACCTGACCCCCGGCGACAGCAGCAGCGGCTGGACCGCCGGCGCCGCCGCCTA
CTACGTGGGCTACCTGCAGCCCCGGACCTTCCTGCTGAAGTACAACGAGAACGGCACCATC
ACCGACGCCGTGGACTGCGCCCTGGACCCCCTGAGCGAGACCAAGTGCACCCTGAAGAGC
TTCACCGTGGAGAAGGGCATCTACCAGACCAGCAACTTCCGGGTGCAGCCCACCGAGAGC
ATCGTGCGGTTCCCCAACATCACCAACCTGTGCCCCTTCGACGAGGTGTTCAACGCCACCC
GGTTCGCCAGCGTGTACGCCTGGAACCGGAAGCGGATCAGCAACTGCGTGGCCGACTACA
GCGTGCTGTACAACTTCGCCCCCTTCTTCGCCTTCAAGTGCTACGGCGTGAGCCCCACCAAG
CTGAACGACCTGTGCTTCACCAACGTGTACGCCGACAGCTTCGTGATCCGGGGCAACGAGG
TGAGCCAGATCGCCCCCGGCCAGACCGGCAACATCGCCGACTACAACTACAAGCTGCCCG
ACGACTTCACCGGCTGCGTGATCGCCTGGAACAGCAACAAGCTGGACAGCAAGGTGGGCG
GCAACTACAACTACCGGTACCGGCTGTTCCGGAAGAGCAACCTGAAGCCCTTCGAGCGGG
ACATCAGCACCGAGATCTACCAGGCCGGCAACAAGCCCTGCAACGGCGTGGCCGGCGTGA
ACTGCTACTTCCCCCTGCAGAGCTACGGCTTCCGGCCCACCTACGGCGTGGGCCACCAGCC
CTACCGGGTGGTGGTGCTGAGCTTCGAGCTGCTGCACGCCCCCGCCACCGTGTGCGGCCCC
AAGAAGAGCACCAACCTGGTGAAGAACAAGTGCGTGAACTTCAACTTCAACGGCCTGACC

GGCACCGGCGTGCTGACCGAGAGCAACAAGAAGTTCCTGCCCTTCCAGCAGTTCGGCCGGG
ACATCGCCGACACCACCGACGCCGTGCGGGACCCCCAGACCCTGGAGATCCTGGACATCAC
CCCCTGCAGCTTCGGCGGCGTGAGCGTGATCACCCCCGGCACCAACACCAGCAACCAGGTG
GCCGTGCTGTACCAGGGCGTGAACTGCACCGAGGTGCCCGTGGCCATCCACGCCGACCAGC
TGACCCCCACCTGGCGGGTGTACAGCACCGGCAGCAACGTGTTCCAGACCCGGGCCGGCTG
CCTGATCGGCGCCGAGTACGTGAACAACAGCTACGAGTGCGACATCCCCATCGGCGCCGGC
ATCTGCGCCTCCTACCAGACCCAGACCAAGAGCCACCGGCGGGCCCGGAGCGTGGCCAGC
CAGAGCATCATCGCCTACACCATGAGCCTGGGCGCCGAGAACAGCGTGGCCTACAGCAAC
AACAGCATCGCCATCCCCACCAACTTCACCATCAGCGTGACCACCGAGATCCTGCCCGTGA
GCATGACCAAGACCAGCGTGGACTGCACCATGTACATCTGCGGCGACAGCACCGAGTGCA
GCAACCTGCTGCTGCAGTACGGCAGCTTCTGCACCCAGCTGAAGCGGGCCCTGACCGGCAT
CGCCGTGGAGCAGGACAAGAACACCCAGGAGGTGTTCGCCCAGGTGAAGCAGATCTACAA
GACCCCCCCCATCAAGTACTTCGGCGGCTTCAACTTCAGCCAGATCCTGCCCGACCCCAGC
AAGCCCAGCAAGCGGAGCTTCATCGAGGACCTGCTGTTCAACAAGGTGACCCTGGCCGAC
GCCGGCTTCATCAAGCAGTACGGCGACTGCCTGGGCGACATCGCCGCCCGGGACCTGATCT
GCGCCCAGAAGTTCAACGGCCTGACCGTGCTGCCCCCCCTGCTGACCGACGAGATGATCGC
CCAGTACACCAGCGCCCTGCTGGCCGGCACCATCACCAGCGGCTGGACCTTCGGCGCCGGC
GCCGCCCTGCAGATCCCCTTCGCCATGCAGATGGCCTACCGGTTCAACGGCATCGGCGTGA
CCCAGAACGTGCTGTACGAGAACCAGAAGCTGATCGCCAACCAGTTCAACAGCGCCATCG
GCAAGATCCAGGACAGCCTGAGCAGCACCGCCAGCGCCCTGGGCAAGCTGCAGGACGTGG
TGAACCACAACGCCCAGGCCCTGAACACCCTGGTGAAGCAGCTGAGCAGCAAGTTCGGCG
CCATCAGCAGCGTGCTGAACGACATCCTGAGCCGGCTGGACCCCCCCGAGGCCGAGGTGC
AGATCGACCGGCTGATCACCGGCCGGCTGCAGAGCCTGCAGACCTACGTGACCCAGCAGCT
GATCCGGGCCGCCGAGATCCGGGCCAGCGCCAACCTGGCCGCCACCAAGATGAGCGAGTG
CGTGCTGGGCCAGAGCAAGCGGGTGGACTTCTGCGGCAAGGGCTACCACCTGATGAGCTTC
CCCCAGAGCGCCCCCCACGGCGTGGTGTTCCTGCACGTGACCTACGTGCCCGCCCAGGAGA
AGAACTTCACCACCGCCCCCGCCATCTGCCACGACGGCAAGGCCCACTTCCCCCGGGAGGG
CGTGTTCGTGAGCAACGGCACCCACTGGTTCGTGACCCAGCGGAACTTCTACGAGCCCCAG
ATCATCACCACCGACAACACCTTCGTGAGCGGCAACTGCGACGTGGTGATCGGCATCGTGA
ACAACACCGTGTACGACCCCCTGCAGCCCGAGCTGGACAGCTTCAAGGAGGAGCTGGACA
AGTACTTCAAGAACCACACCAGCCCCGACGTGGACCTGGGCGACATCAGCGGCATCAACG
CCAGCGTGGTGAACATCCAGAAGGAGATCGACCGGCTGAACGAGGTGGCCAAGAACCTGA
ACGAGAGCCTGATCGACCTGCAGGAGCTGGGCAAGTACGAGCAGTACATCAAGTGGCCCT
GGTACATCTGGCTGGGCTTCATCGCCGGCCTGATCGCCATCGTGATGGTGACCATCATGCT
GTGCTGCATGACCAGCTGCTGCAGCTGCCTGAAGGGCTGCTGCAGCTGCGGCAGCTGCTGC
AAGTTCGACGAGGACGACAGCGAGCCCGTGCTGAAGGGCGTGAAGCTGCACTACACCTGA
TGAGCTCGCTTTCTTGCTGTCCAATTTCTATTAAAGGTTCCTTTGTTCCCTAAGTCCAACTAC
TAAACTGGGGGATATTATGAAGGGCCTTGAGCATCTGGATTCTGCCTAATAAAAAACATTT
ATTTTCATTGCAATTGCCATGTGTATGTGGGTTCGCCCACATACTCTGATGATCCCCAATCG
TGGCGTGTCGGCCTGCTTCGGCAGGCACTGGCGCCGGGGATCATTCATGCGCAAGCTGGAGCC
TCGGTGGCCATGCTTCTTGCCCCTTGGGCCTCCCCCCAGCCCCTCCTCCCCTTCCTGCACCC
GTACCCCCGTGGTCTTTGAATAAAGTCTGAGTGGGCGGCAAAAAAAAAAAAAAAAAAAAA
AAAAAAAAAAGCATATGACTAAAAAAAAAAAAAAAAAAAAAAAAAAAAAAAAAAAAAAA
AAAAAAAAAAAAAAAAAAAAAAAAAAAAA

SEQ IND NO: 27 Luciferase RNA sequence

AUGGAAGACGCCAAAAACAÚAAAGAAAGGCCCGGCGCCAUUCUAUCCGCUGGAAG
AUGGAACCGCUGGAGAGCAACUGCAUAAGGCUAUGAAGAGAUACGCCCUGGUUCCUGGA
ACAAUUGCUUUUACAGAUGCACAUAUCGAGGUGGACAUCACUUACGCUGAGUACUUCGA
AAUGUCCGUUCGGUUGGCAGAAGCUAUGAAACGAUAUGGGCUGAAUACAAAUCACAGA
AUCGUCGUAUGCAGUGAAAACUCUCUUCAAUUCUUUAUGCCGGUGUUGGGCGCGUUAUU
UAUCGGAGUUGCAGUUGCGCCCGCGAACGACAUUUAUAAUGAACGUGAAUUGCUCAACA
GUAUGGGCAUUUCGCAGCCUACCGUGGUGUUCGUUUCCAAAAAGGGGUUGCAAAAAAUU
UUGAACGUGCAAAAAAAGCUCCCAAUCAUCCAAAAAAUUAUUAUCAUGGAUUCUAAAAC
GGAUUACCAGGGAUUUCAGUCGAUGUACACGUUCGUCACAUCUCAUCUACCUCCCGGUU
UUAAUGAAUACGAUUUUGUGCCAGAGUCCUUCGAUAGGGACAAGACAAUUGCACUGAUC
AUGAACUCCUCUGGAUCUACUGGUCUGCUAAAGGUGCGCUCUGCCUCAUAGAACUGC
CUGCGUGAGAUUCUCGCAUGCCAGAGAUCCUAUUUUUGGCAAUCAAAUCAUUCCGGAUA
CUGCGAUUUUAAGUGUUGUUCCAUUCCAUCACGGUUUUGGAAUGUUUACUACACUCGGA
UAUUUGAUAUGUGGAUUUCGAGUCGUCUUAAUGUAUAGAUUUGAAGAAGAGCUGUUUC
UGAGGAGCCUUCAGGAUUACAAGAUUCAAAGUGCGCUGCUGGUGCCAACCCUAUUCUCC
UUCUUCGCCAAAAGCACUCUGAUUGACAAAUACGAUUUAUCUAAUUUACACGAAAUUGC
UUCUGGUGGCGCUCCCCUCUCUAAGGAAGUCGGGGAAGCGGUUGCCAAGAGGUUCCAUC
UGCCAGGUAUCAGGCAAGGAUAUGGGCUCACUGAGACUACAUCAGCUAUUCUGAUUACA
CCCGAGGGGGAUGAUAAACCGGGCGCGGUCGGUAAAGUUGUUCCAUUUUUUGAAGCGAA
GGUUGUGGAUCUGGAUACCGGGAAAACGCUGGGCGUUAAUCAAAGAGGCGAACUGUGU
GUGAGAGGUCCUAUGAUUAUGUCCGGUUAUGUAAACAAUCCGGAAGCGACCAACGCCUU
GAUUGACAAGGAUGGAUGGCUACAUUCUGGAGACAUAGCUUACUGGGACGAAGACGAA
CACUUCUUCAUCGUUGACCGCCUGAAGUCUCUGAUUAAGUACAAAGGCUAUCAGGUGGC
UCCCGCUGAAUUGGAAUCCAUCUUGCUCCAACACCCCAACAUCUUCGACGCAGGUGUCG

CAGGUCUUCCCGACGAUGACGCCGGUGAACUUCCCGCCGCCGUUGUUGUUUUGGAGCAC
GGAAAGACGAUGACGGAAAAAGAGAUCGUGGAUUACGUCGCCAGUCAAGUAACAACCGC
GAAAAAGUUGCGCGGAGGAGUUGUGUUUGUGGACGAAGUACCGAAAGGUCUUACCGGA
AAACUCGACGCAAGAAAAUCAGAGAGAUCCUCAUAAAGGCCAAGAAGGGCGGAAAGAU
CGCCGUGUAA

Note: T and U in the above sequence are interchangeable and can be replaced in whole or in part by modified bases, such as 1-methylpseudouridine or pseudouridine.

SEQ ID NO: 28
AUGGAAUmACUCUUGGUUmACdTdT

SEQ ID NO: 29
GUmAACmCmAAGAGUmAUmUmCmCmAUmdTdT

SEQ ID NO: 30
AGCGTGCTCTATAACTCGGC

SEQ ID NO: 31
TCGGACCTCATCGCCTCTAA

SEQ ID NO: 32
ACGGCGTGAGCCCCACAAAG

## Claims

1. A lipid nanoparticle composition for encapsulating a nucleic acid, wherein the no-load rate of the lipid nanoparticle is not more than 10%, preferably not more than 9%, and more preferably not more than 8.5%, wherein the no-load rate refers to the ratio of the number of empty lipid nanoparticles without nucleic acids to the total number of lipid nanoparticles in the composition.

2. The composition according to claim 1, wherein the average particle size of the lipid nanoparticles is 50 nm-150 nm, preferably 70 nm-120 nm, and more preferably 90 nm-110 nm.

3. The composition according to claim 1, wherein the encapsulation efficiency of the lipid nanoparticles is above 80%, preferably above 85%, and more preferably above 90%.

4. The composition according to any one of claims 1 to 3, wherein the lipid nanoparticle comprises:

   (1) a nucleic acid, and
   (2) a lipid component, which comprises an ionizable cationic lipid, a helper lipid, a structured lipid and a surfactant.

5. The composition according to claim 4, wherein the lipid component comprises 20-60 mol% of the ionizable cationic lipid, 25-55 mol% of the structured lipid, 2-25 mol% of the helper lipid and 0.5-15 mol% of the surfactant, based on the total molar content 100% of the lipid component.

6. The composition according to claim 4 or 5, wherein the cationic lipid is selected from the group consisting of SM-102, ALC-0315, Dlin-MC3-DMA, DODMA, C12-200 and DlinDMA, and/or the structured lipid is selected from cholesterol or cholesterol derivatives, and/or the helper lipid is selected from the group consisting of DSPC, DOPE, DOPC, DOPG and DOPS, and/or the surfactant is selected from the group consisting of mPEG-DMG-2K, ALC-0159, PEG-DSPE, DTDA-PEG2000 and TPGS.

7. The composition according to claim 4 or 5, wherein the lipid nanoparticles have an N:P ratio of from about 2:1 to about 30:1, preferably from about 2:1 to about 15:1, more preferably from about 2:1 to about 10:1, and even more preferably from about 3:1 to about 6:1.

8. The composition according to claim 4, wherein the weight ratio of the ionizable cationic lipid to the nucleic acid is from about 5:1 to about 100:1, preferably from about 5:1 to about 50:1, more preferably from about 5:1 to about 30:1, and even more preferably from about 10:1 to about 20:1.

9. The composition according to claim 4, wherein the lipid nanoparticle composition further comprises a final product buffer, which comprises a buffering agent and/or a cryoprotectant.

10. The composition according to claim 9, wherein the buffering agent is selected from the group consisting of acetates, formates, carbonates, phosphates, borates, succinates, gluconates, lactates, citric acid, glycine, leucine, barbituric acid, phthalic acid, tromethamine (Tris), triethylamine, ammonium salts and combinations thereof, and the pH value of the buffer solution is 7-8, and/or

the cryoprotectant is selected from the group consisting of sugars, polyols, polymers, surfactants, amino acids and salts, wherein the sugars are selected from the group consisting of lactose, sucrose, trehalose and galactose.

11. The composition according to claim 9, wherein the final product buffer comprises tromethamine, sodium acetate and sucrose, and has a pH value of 7-8.

12. The composition according to claim 1, wherein the nucleic acid is selected from an mRNA comprising a nucleotide sequence having at least 80%, at least 85%, at least 90%, at least 95%, at least 98%, at least 99% or 100% identity to any one of the nucleotide sequence of SEQ ID NO: 1 to SEQ ID NO: 6, or an mRNA encoding the coronavirus antigen which comprises an amino acid sequence having at least 80%, at least 85%, at least 90%, at least 95%, at least 98%, at least 99% or 100% identity to the amino acid sequence of SEQ ID NO: 7, or an mRNA comprising a nucleotide sequence having at least 80%, at least 85%, at least 90%, at least 95%, at least 98%, at least 99% or 100% identity to any one of the nucleotide sequence of SEQ ID NO: 8 to 26, preferably, the mRNA comprises the nucleotide sequence of SEQ ID NO: 1, more preferably, the mRNA is the nucleotide sequence of SEQ ID NO: 1.

13. The composition according to claim 1, comprising lipid nanoparticles encapsulating a nucleic acid, tromethamine, sodium acetate and sucrose, wherein

the pH of the composition is 7.0-8.0;
the nucleic acid is mRNA at a concentration of 100 μg/ml; and
the lipid component of the lipid nanoparticles comprises 50 mol% of SM-102, 10 mol% of DSPC, 38.5 mol% of cholesterol and 1.5 mol% of mPEG-DMG-2K.

14. A preparation method of the lipid nanoparticle composition according to any one of claims 1 to 13, comprising:

preparing precursor lipid nanoparticles to obtain a buffer system containing the precursor lipid nanoparticles, and replacing the buffer system containing the precursor lipid nanoparticles with a neutral buffer system to obtain a final product lipid nanoparticle composition,
wherein, by replacing the buffer system, the content of the organic solvent in the buffer system containing the precursor lipid nanoparticles is reduced to less than or equal to 5% (w/w), preferably less than 3% (w/w) when the pH value of the system is lower than the pKa of the lipid nanoparticles by more than 0.5 units.

15. The preparation method according to claim 14, wherein the replacement of the buffer system comprises a two-stage buffer system replacement process, preferably, the two-stage buffer system replacement process comprises replacement with an acidic buffer solution or a neutral buffer solution in the first stage and replacement with a neutral buffer solution in the second stage.

16. The preparation method according to claim 15, wherein the replacement process is selected from one or more of tangential flow filtration, membrane dialysis, column dialysis and dilution.

17. The preparation method according to claim 15, wherein:

at the end of the first stage, the pH value of the buffer system containing the precursor lipid nanoparticles is lower than the pKa of the lipid nanoparticles by more than 0.5 units, and the content of the organic solvent is reduced to less than or equal to 5% (w/w), preferably less than 3% (w/w); and/or
at the end of the second stage, the pH value of the buffer system containing the final product lipid nanoparticles is higher than the pKa of the lipid nanoparticles by more than 0.5 units, and/or the content of the organic solvent in the buffer system containing the final product lipid nanoparticles is reduced to less than 1% (w/w), preferably less than 0.1% (w/w).

18. The preparation method according to claim 15, wherein the pH value of the acidic buffer solution is lower than the pKa of the lipid nanoparticles, and the buffer solution comprises a buffering agent selected from the the group consisting of acetates, formates, carbonates, phosphates, borates, succinates, gluconates, lactates, citric acid, glycine, leucine, barbituric acid, phthalic acid, tromethamine (Tris), triethylamine, ammonium salts and combinations thereof, and optionally an osmotic pressure regulator selected from the group consisting of sodium chloride and potassium chloride, preferably, the buffer solution comprises citric acid and sodium chloride.

19. The preparation method according to claim 15, wherein the pH value of the neutral buffer solution is higher than the pKa of the lipid nanoparticles, and the buffer solution comprises a buffering agent selected from the group consisting of acetates, formates, carbonates, phosphates, borates, succinates, gluconates, lactates, citric acid, glycine, leucine, barbituric acid, phthalic acid, tromethamine (Tris), triethylamine, ammonium salts and combinations thereof, and optionally a cryoprotectant selected from the group consisting of sugars, polyols, polymers, surfactants, amino acids and salts, preferably, the buffer solution comprises tromethamine, sodium acetate and sucrose.

20. The preparation method according to any one of claims 14 to 19, wherein the pKa of the lipid nanoparticles is 6.0-7.0.

21. The preparation method according to claim 14, wherein the precursor lipid nanoparticles are prepared by:

dissolving a lipid component comprising an ionizable cationic lipid, a helper lipid, a structured lipid and a surfactant in an organic solvent to form a lipid phase; dissolving a nucleic acid in an acidic buffer solution to form an aqueous phase; and mixing the lipid phase and the aqueous phase to allow the lipid to encapsulate the nucleic acid to form the precursor lipid nanoparticles, or
dissolving a lipid component comprising an ionizable cationic lipid, a helper lipid, a structured lipid and a surfactant in an organic solvent to form a lipid phase, mixing the lipid phase and an acidic buffer solution to form empty lipid nanoparticles, dissolving a nucleic acid in an acidic buffer solution to form an aqueous phase, and mixing the empty lipid nanoparticles with the aqueous phase containing the nucleic acid to allow the lipid to encapsulate the nucleic acid to form the precursor lipid nanoparticles.

22. The preparation method of the lipid nanoparticle composition encapsulating a nucleic acid according to any one of claims 1 to 13, comprising the following steps:

(1) dissolving the lipid component in an organic solvent to form an organic phase;
(2) dissolving the nucleic acid in an acidic buffer solution to form an aqueous phase;
(3) mixing the organic phase and the aqueous phase to allow the lipid to encapsulate the nucleic acid to form the precursor lipid nanoparticles; and
(4) replacing the organic solvent-acidic buffer system containing the precursor lipid nanoparticles obtained in step (3) with a neutral buffer system in two stages to obtain a final product lipid nanoparticle composition, wherein an acidic buffer solution or a neutral buffer solution is used for the replacement in the first stage, and a neutral buffer solution is used for the replacement in the second stage.

23. The preparation method according to claim 22, wherein in step (4), at the end of the first stage, the pH value of the buffer system containing the precursor lipid nanoparticles is lower than the pKa of the lipid nanoparticles by more than 0.5 units, and/or the content of the organic solvent in the buffer system containing the precursor lipid nanoparticles is reduced to less than or equal to 5% (w/w), preferably less than 3% (w/w).

24. The preparation method according to claim 22, wherein in step (4), at the end of the second stage, the pH value of the buffer system containing the final product lipid nanoparticles is higher than the pKa of the lipid nanoparticles by more than 0.5 units, and/or the content of the organic solvent in the buffer system containing the final product lipid nanoparticles is reduced to less than 1% (w/w), preferably less than 0.1% (w/w).

25. The preparation method according to claim 22, wherein the method optionally comprises step (5) concentration, dilution, sterile filtration, aseptic filling or a combination thereof.

26. The preparation method according to claim 22, wherein the organic solvent in step (1) is selected from C1-C4 lower alcohols, preferably ethanol.

27. The preparation method according to claim 22, wherein the total concentration of the lipid component in the organic phase in step (1) is 10-15 mg/ml.

28. The preparation method according to claim 22, wherein the concentration of the nucleic acid in the aqueous phase in step (2) is 0.01-1 mg/ml, preferably 0.05-0.5 mg/ml, and more preferably 0.1-0.2 mg/ml.

29. The preparation method according to claim 22, wherein the acidic buffer solution in step (2) or step (4) comprises a buffering agent selected from the group consisting of acetates, formates, carbonates, phosphates, borates, succinates, gluconates, lactates, citric acid, glycine, leucine, barbituric acid, phthalic acid, tromethamine (Tris), triethylamine, ammonium salts and combinations thereof, and the pH value of the acidic buffer solution is lower than the pKa of the lipid nanoparticles.

30. The preparation method according to claim 29, wherein the pKa of the lipid nanoparticles is 6.0-7.0, and the pH value of the acidic buffer solution in step (2) or step (4) is 3.0-5.0, preferably 4.0-5.0.

31. The preparation method according to claim 22, wherein the acidic buffer solution in step (2) or step (4) further comprises an osmotic pressure regulator, which is selected from the group consisting of sodium chloride and potassium chloride.

32. The preparation method according to claim 31, wherein the acidic buffer solution in step (2) or step (4) comprises citric acid and sodium chloride, preferably, comprises 10-20 mM of citric acid and 120-140 mM of sodium chloride.

33. The preparation method according to claim 22, wherein the volume ratio of the organic phase and the aqueous phase mixed in step (3) is 1:2-1:9, preferably 1:2-1:5, and more preferably 1:3.

34. The preparation method according to claim 22, wherein the replacement process in step (4) is selected from one or more of tangential flow filtration, membrane dialysis, column dialysis, and dilution.

35. The preparation method according to claim 22, wherein the two-stage replacement process in step (4) can be repeated multiple times.

36. The preparation method according to claim 22, wherein the neutral buffer solution in step (4) comprises a buffering agent selected from the group consisting of acetates, formates, carbonates, phosphates, borates, succinates, gluconates, lactates, citric acid, glycine, leucine, barbituric acid, phthalic acid, tromethamine (Tris), triethylamine, ammonium salts and combinations thereof, and the pH value of the neutral buffer solution is higher than the pKa of the lipid nanoparticles.

37. The preparation method according to claim 36, wherein the pKa of the lipid nanoparticles is 6.0-7.0, and the pH value of the neutral buffer solution in step (4) is 7.0-8.0.

38. The preparation method according to claim 36, wherein the neutral buffer solution in step (4) further comprises a cryoprotectant selected from the group consisting of sugars, polyols, polymers, surfactants, amino acids and salts, wherein the sugars are selected from the group consisting of lactose, sucrose, trehalose and galactose.

Figure 1

Figure 2

Figure 3

Figure 4

Figure 5

Figure 6

Figure 7

Figure 8

Figure 9

Figure 10

Figure 11

Figure 12

Figure 13

## INTERNATIONAL SEARCH REPORT

| International application No. |
|---|
| **PCT/CN2024/096347** |

| A. | CLASSIFICATION OF SUBJECT MATTER |
|---|---|

A61K9/127(2006.01)i; A61K9/19(2006.01)i; A61K9/51(2006.01)i; A61K31/7088(2006.01)i; A61K39/12(2006.01)i;
A61K47/26(2006.01)i; A61K47/28(2006.01)i; A61K47/44(2017.01)i; A61K47/69(2017.01)i; C12N15/87(2006.01)i;
A61P31/16(2006.01)i

According to International Patent Classification (IPC) or to both national classification and IPC

| B. | FIELDS SEARCHED |
|---|---|

Minimum documentation searched (classification system followed by classification symbols)

IPC: A61K; C12N; A61P

Documentation searched other than minimum documentation to the extent that such documents are included in the fields searched

Electronic data base consulted during the international search (name of data base and, where practicable, search terms used)

CNTXT, WPABS, WPABSC, ENTXT, ENTXTC, DWPI, VCN, VEN, CJFD, CNKI, ISI-WEB OF SCIENCE, PUBMED, BING, STNext, 百度, BAIDU, 百度学术, BAIDU SCHOLAR, 读秀, DUXIU: 脂质纳米粒, 脂质纳米, 核酸, 可电离阳离子脂质, 可离子化脂质, 1, 2-二硬脂酰基-sn-甘油基-3-磷酸胆碱, 辅助脂质, 中性脂质, 阴离子脂质, 结构脂质, 胆固醇, 表面活性剂, 置换, 粒子融合, 乙醇, 缓冲, 酸性缓冲液, 中性缓冲液, 醋酸钠, 醋酸, 柠檬酸, 枸橼酸, 氯化钠, 冷冻保护, 氨丁三醇, 蔗糖, 新冠, 新型冠状, 冠状病毒, 切向流, 过滤, 透析, 稀释, 空载, 空白颗粒, 空白, 空白纳米粒, 装载, 负载, 荷载, 载荷, 包载, 空壳, 空包, 粒度, 粒径, 包封率, LNP, LNPs, lipid nanoparticle, mRNA, siRNA, RNA, DNA, mRNA-LNPs, SM-102, ALC-0315, Dlin-MC3-DMA, DODMA, C12-200, DlinDMA, DSPC, DOPE, DOPC, DOPG, DOPS, mPEG-DMG-2K, ALC-0159, PEG-DSPE, DTDA-PEG2000, TPGS, lyoprotection, ethanol, tangential flow filtration, TFF, dialysis, dilution, filtration, NaOAc, NaOK, NaCl, tris, sucrose, pH, pKa, blank, load, empty, empty LNPs, rate, proportion

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | GENG, Cong et al. "A preparation method for mRNA-LNPs with improved properties" *Journal of Controlled Release*, Vol. 364, 16 November 2023 (2023-11-16), pages 632-643 | 1-13, 21-38 |
| PY | GENG, Cong et al. "A preparation method for mRNA-LNPs with improved properties" *Journal of Controlled Release*, Vol. 364, 16 November 2023 (2023-11-16), pages 632-643 | 14-21 |

| ✓ | Further documents are listed in the continuation of Box C. | ✓ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **12 September 2024** | **20 September 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
|---|
| **PCT/CN2024/096347** |

**C.    DOCUMENTS CONSIDERED TO BE RELEVANT**

| Category* | Citation of document, with indication, where appropriate, of the relevant passages | Relevant to claim No. |
|---|---|---|
| PX | 上海市生物医药新媒体门户 (Non-official translation: Shanghai Biomedicine New Media Portal). "诺奖花落mRNA疗法，NanoFCM赋能核酸药物研究 (Non-official translation: Nobel Prize Goes to mRNA Therapy, NanoFCM Empowers Nucleic Acid Drug Research)" 生物谷 *(Bioon)*, 23 October 2023 (2023-10-23), https://h5.ifeng.com/c/vivo/v002bSEKOIbOoDT3ry89-_ttdk4Qya0heKWLjLmfvcf1RRI U__?isNews=1 | 1, 2 |
| X | CN 115487168 A (ZHEJIANG UNIVERSITY) 20 December 2022 (2022-12-20) claims 1-10, and description, paragraphs [0056]-[0101] | 1-38 |
| X | US 2022362372 A1 (PFIZER INC.) 17 November 2022 (2022-11-17) claim 18, description, paragraphs [0214]-[0217], [0231], and [0239]-[0250], and tables 1 and 9 | 1-38 |
| X | CN 110974954 A (ZHUHAI LIFANDA BIOTECHNOLOGY CO., LTD.) 10 April 2020 (2020-04-10) claims 1-10, description, paragraphs [0039]-[0061] and [0079]-[0095], and tables 2-4 | 1-38 |
| X | KULKARNI, J. A. et al. "Spontaneous, solvent-free entrapment of siRNA within lipid nanoparticles" *Nanoscale*, 31 December 2020 (2020-12-31), pages 23959-23966 | 1-38 |
| X | MIHAILA, R. et al. "Lipid nanoparticle purification by Spin Centrifugation–Dialysis (SCD): A facile and high-throughput approach for small scale preparation of siRNA–lipid complexes" *International Journal of Pharmaceutics*, Vol. 420, 27 August 2011 (2011-08-27), pages 118-121 | 1-38 |
| X | LEUNG, A. K. K. et al. "Lipid Nanoparticles for Short Interfering RNA Delivery" *Advances in Genetics*, Vol. 88, 31 December 2014 (2014-12-31), pages 71-110 | 1-13, 21-38 |
| Y | LEUNG, A. K. K. et al. "Lipid Nanoparticles for Short Interfering RNA Delivery" *Advances in Genetics*, Vol. 88, 31 December 2014 (2014-12-31), pages 71-110 | 14-21 |
| A | LI, Sixuan et al. "Payload distribution and capacity of mRNA lipid nanoparticles" *Nature Communications*, Vol. 13, No. 5561, 23 September 2022 (2022-09-23), https://doi.org/10.1038/s41467-022-33157-4 | 1-38 |

Form PCT/ISA/210 (second sheet) (July 2022)

**INTERNATIONAL SEARCH REPORT**

International application No.

**PCT/CN2024/096347**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

International application No.

**PCT/CN2024/096347**

| Patent document cited in search report | | | Publication date (day/month/year) | Patent family member(s) | | | Publication date (day/month/year) |
|---|---|---|---|---|---|---|---|
| CN | 115487168 | A | 20 December 2022 | None | | | |
| US | 2022362372 | A1 | 17 November 2022 | CA | 3218913 | A1 | 10 November 2022 |
| | | | | WO | 2022234417 | A1 | 10 November 2022 |
| | | | | AU | 2022268706 | A1 | 30 November 2023 |
| | | | | AU | 2022268706 | A9 | 07 December 2023 |
| | | | | KR | 20240004862 | A | 11 January 2024 |
| | | | | BR | 112023022928 | A2 | 23 January 2024 |
| | | | | CN | 117597144 | A | 23 February 2024 |
| | | | | EP | 4333880 | A1 | 13 March 2024 |
| | | | | JP | 2024517229 | W | 19 April 2024 |
| | | | | HK | 40103442 | A0 | 28 June 2024 |
| CN | 110974954 | A | 10 April 2020 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310626158
- CN 202311497985
- CN 202111445859 X

**Non-patent literature cited in the description**

- **KULKARNI JA et al.** *ACS Nano.*, 22 May 2018, vol. 12 (5), 4787-4795
- **MAMONTOV E et al.** *Medicina (Kaunas).*, 09 December 2021, vol. 57 (12), 1343
- **PATONE M et al.** Risk of Myocarditis After Sequential Doses of COVID-19 Vaccine and SARS-CoV-2 Infection by Age and Sex.. *Circulation.*, 06 September 2022, vol. 146 (10), 743-754
- **NDEUPEN S et al.** The mRNA-LNP platform's lipid nanoparticle component used in preclinical vaccine studies is highly inflammatory.. *iScience.*, 17 December 2021, vol. 24 (12), 103479
- **LI S et al.** Payload distribution and capacity of mRNA lipidnanoparticles.. *Nat Commun.*, 23 September 2022, vol. 13 (1), 5561
- *CHEMICAL ABSTRACTS*, 2089251-47-6
- *CHEMICAL ABSTRACTS*, 2036272-55-4
- *CHEMICAL ABSTRACTS*, 1224606-06-7
- *CHEMICAL ABSTRACTS*, 104162-47-2
- *CHEMICAL ABSTRACTS*, 1220890-25-4
- *CHEMICAL ABSTRACTS*, 871258-12-7
- *CHEMICAL ABSTRACTS*, 160743-62-4
- *CHEMICAL ABSTRACTS*, 1849616-42-7
- **BATZER et al.** *Nucleic Acid Res.*, 1991, vol. 19, 5081
- **OHTSUKA et al.** *J. Biol. Chem.*, 1985, vol. 260, 2605-2608
- **ROSSOLINI et al.** *Mol. Cell. Probes*, 1994, vol. 8, 91-98